(19) Europäisches Patentamt
European Patent Office
Office européen des brevets

(11) EP 2 064 185 B1

(12) **EUROPEAN PATENT SPECIFICATION**

(45) Date of publication and mention of the grant of the patent:
**08.06.2011 Bulletin 2011/23**

(21) Application number: **07788348.6**

(22) Date of filing: **09.08.2007**

(51) Int Cl.:
*C07D 215/06* (2006.01) *C07D 215/18* (2006.01)
*C07D 401/06* (2006.01) *C07D 401/14* (2006.01)
*C07D 409/06* (2006.01) *C07D 413/06* (2006.01)
*C07D 405/06* (2006.01) *C07D 417/06* (2006.01)
*A61K 31/47* (2006.01) *A61P 25/20* (2006.01)
*A61P 25/22* (2006.01)

(86) International application number:
**PCT/EP2007/058288**

(87) International publication number:
**WO 2008/017710 (14.02.2008 Gazette 2008/07)**

(54) **1H-QUINOLIN-4-ONE COMPOUNDS, WITH AFFINITY FOR THE GABA RECEPTOR, PROCESSES, USES AND COMPOSITIONS**

1H-CHINOLIN-4-ON-VERBINDUNGEN MIT AFFINITÄT ZUM GABA-REZEPTOR, VERFAHREN, ANWENDUNGEN UND ZUSAMMENSETZUNGEN

COMPOSES DE 1H-QUINOLIN-4-ONE, PROCEDES, UTILISATIONS ET COMPOSITIONS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **10.08.2006 EP 06118720**
**10.08.2006 US 836666 P**

(43) Date of publication of application:
**03.06.2009 Bulletin 2009/23**

(73) Proprietor: **Ferrer Internacional, S.A.**
**08028 Barcelona (ES)**

(72) Inventors:
- **FALCÓ, José Luis**
  **E-08004 Barcelona (ES)**
- **PALOMER, Albert**
  **E-08014 Barcelona (ES)**
- **GUGLIETTA, Antonio**
  **E-08750 Molins De Rei (ES)**

(74) Representative: **Reitstötter - Kinzebach**
**Patentanwälte**
**Sternwartstrasse 4**
**81679 München (DE)**

(56) References cited:
**EP-A1- 0 719 764** **WO-A-98/17662**
**WO-A2-02/22074** **WO-A2-03/097564**

- **TRAXLER, PETER ET AL: "Use of a Pharmacophore Model for the Design of EGFR Tyrosine Kinase Inhibitors: Isoflavones and 3-Phenyl-4(1H)-quinolones" JOURNAL OF MEDICINAL CHEMISTRY , 42(6), 1018-1026 CODEN: JMCMAR; ISSN: 0022-2623, 1999, XP002416417**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

TECHNICAL FIELD

This invention is directed to a new class of agents with affinity for $GABA_A$ receptor. The invention concerns specifically new 1H-quinolin-4-one compounds which are useful for treating or preventing anxiety, epilepsy and sleep disorders including insomnia, and for inducing sedation-hypnosis, anaesthesia, sleep and muscle relaxation.

BACKGROUND OF THE INVENTION

$GABA_A$ receptor ($\gamma$-aminobutyric $acid_A$) is a pentameric protein which forms a membrane ion channel. $GABA_A$ receptor is implicated in the regulation of sedation, anxiety, muscle tone, epileptogenic activity and memory functions. These actions are due to defined subunits of $GABA_A$ receptor, particularly the $\alpha_1$- and $\alpha_2$-subunits.

Sedation is modulated by the $\alpha_1$-subunit. Zolpidem is characterized by a high affinity for the $\alpha_1$-receptors and its sedative and hypnotic action is mediated by these receptors *in vivo.* Similarly, the hypnotic action of zaleplon is also mediated by the $\alpha_1$-receptors.

The anxiolytic action of diazepam is mediated by the enhancement of GABAergic transmission in a population of neurons expressing the $\alpha_2$-receptors. This indicates that the $\alpha_2$-receptors are highly specific targets for the treatment of anxiety.

Muscle relaxation in diazepam is mainly mediated by $\alpha_2$-receptors, since these receptors exhibit a highly specific expression in spinal cord.

The anticonvulsant effect of diazepam is partly due to $\alpha_1$-receptors. In diazepam, a memory-impairing compound, anterograde amnesia is mediated by $\alpha_1$-receptors.

$GABA_A$ receptor and its $\alpha_1$- and $\alpha_2$-subunits have been widely reviewed by H. Möhler et al. (J. Pharmacol. Exp. Ther., 300, 2-8, 2002); H. Möhler et al. (Curr. Opin. Pharmacol., 1, 22-25, 2001); U. Rudolph et al. (Nature, 401, 796-800, 1999); and D.J. Nutt et al. (Br. J. Psychiatry, 179, 390-396, 2001).

Diazepam and other classical benzodiazepines are extensively used as anxiolytic agents, hypnotic agents, anticonvulsants and muscle relaxants. Their side effects include anterograde amnesia, decrease in motor activity and potentiation of ethanol effects.

In this context, the compounds of this invention are ligands of $\alpha_1$- and $\alpha_2$-$GABA_A$ receptor for their clinical application in sleep disorders, preferably insomnia, anxiety and epilepsy.

Insomnia is a highly prevalent disease. Its chronicity affects 10% of the population and 30% when transitory insomnia is computed as well. Insomnia describes the trouble in getting to sleep or staying asleep and is associated with next-day hangover effects such as weariness, lack of energy, low concentration and irritability. The social and health impact of this complaint is important and results in evident socioeconomic repercussions.

Pharmacological therapy in the management of insomnia firstly included barbiturates and chloral hydrate, but these drugs elicit numerous known adverse effects, for example, overdose toxicity, metabolic induction, and enhanced dependence and tolerance. In addition, they affect the architecture of sleep by decreasing above all the duration and the number of REM sleep stages. Later, benzodiazepines meant an important therapeutic advance because of their lower toxicity, but they still showed serious problems of dependence, muscle relaxation, amnesia and rebound insomnia following discontinuation of medication.

The latest known therapeutic approach has been the introduction of non-benzodiazepine hypnotics, such as pyrrolo[3,4-b]pyrazines (zopiclone), imidazo[1,2-a] pyridines (zolpidem) and, finally, pyrazolo[1,5-a] pyrimidines (zaleplon). Later, two new pyrazolo[1,5-a] pyrimidines, indiplon and ocinaplon, have entered into development, the latter with rather anxiolytic action. All these compounds show a rapid sleep induction and have less next-day hangover effects, lower potential for abuse and lower risk of rebound insomnia than benzodiazepines. The mechanism of action of these compounds is the alosteric activation of $GABA_A$ receptor through its binding to benzodiazepine binding site (C. F. P. George, The Lancet, 358, 1623-1626, 2001). While benzodiazepines are unspecific ligands at $GABA_A$ receptor binding site, zolpidem and zaleplon show a greater selectivity for $\alpha_1$-subunit. Notwithstanding that, these drugs still affect the architecture of sleep and may induce dependence in long-term treatments.

Research for new active compounds in the management of insomnia answers an underlying health need, because even recently introduced hypnotics still affect the architecture of sleep and may induce dependence in long-term treatments.

It is therefore desirable to focus on the development of new hypnotic agents with a lower risk of side effects.

The present invention is directed to a new class of 1H-quinolin-4-one compounds, specifically a new chemotype consisting of phenyl-substituted N-acetyl-1H-quinolin-4-one, which is active versus $GABA_A$ receptor and, particularly, versus its $\alpha_1$- and $\alpha_2$-subunits. No related compounds belonging to this chemotype have been disclosed to present with ligand $GABA_A$ receptor or any other central nervous system activity.

Compounds containing the 1H-quinolin-4-one core have been disclosed for other purposes. Thus, EP 856255 discloses the use of some 1H-quinolin-4-ones as marine antifouling agents, WO 2001012607 discloses some other 1H-quinolin-4-ones which increase the activity of cytotoxic agents, and finally WO 2002022074 discloses the preparation of some 1H-3-phenyl-quinolin-4-ones as intimal neoproliferation inhibitors. Traxler et al., J. Med. Chem. 1999, 42, 1018-1026 and WO 98/17662 disclose isoflavones and 3-phenyl-4(1H)-quinolones which have inhibitory activity against the EGFR kinase. They have potential in the treatment of malignant and non-malignant epithelial diseases. EP 719 764 discloses 3-phenyl-isochinolin-1(2H-ones as ligands of GABA receptors. WO 03/097564 relates to 6-aminosubstituted quinolones that act as enhancers of GABA-facilitated $Cl^-$flux-mediated through the $GABA_a$ receptor complex (GRC).

The compounds of the present invention have a high affinity for $\alpha_1$- and $\alpha_2$-$GABA_A$ receptors. The *in vitro* activities shown by these compounds are consistent with those *in vivo* results obtained in sedation-hypnosis tests.

Consequently, the compounds of this invention are useful in the treatment and prevention of all those diseases mediated by $GABA_A$ receptor $\alpha_1$- and $\alpha_2$-subunits. Non-limitative examples of such diseases are sleep disorders, preferably insomnia, anxiety and epilepsy. Non-limitative examples of the relevant indications of the compounds of this invention are all those diseases or conditions, such as insomnia or anesthesia, in which an induction of sleep, an induction of sedation or an induction of muscle relaxation are needed.

SUMMARY OF THE INVENTION

The present invention relates to a compound of formula (I):

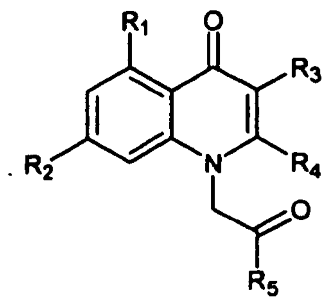

and pharmaceutically acceptable salts and hydrates thereof, which are ligands of $GABA_A$ receptor.

It is another object of this invention to provide synthetic procedures for preparing the compounds of formula (I). Novel methods of treating or preventing diseases associated with $GABA_A$ receptor modulation such as anxiety, epilepsy and sleep disorders including insomnia, and for inducing sedation-hypnosis, anesthesia, sleep and muscle relaxation by administering a therapeutically effective amount of said compounds are also within the scope of the invention.

DETAILED DESCRIPTION OF THE INVENTION

The present invention relates to a compound of formula (I):

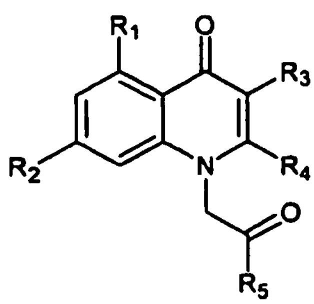

**(I)**

wherein $R_1$ and $R_2$ are independently selected from the group consisting of hydrogen, alkyl($C_1$-$C_6$), Oalkyl($C_1$-$C_6$), halogen and phenyl; $R_3$ and $R_4$ are independently selected from the group consisting of hydrogen and phenyl optionally substituted by alkyl($C_1$-$C_6$), halogen and Oalkyl($C_1$-$C_6$); $R_5$ is selected from the group consisting of $NR_6R_7$, $N(R_8)NH_2$, and a heteroaryl ring selected from the group consisting of pyridyl, furyl, thienyl, oxazolyl, isoxazolyl, imidazolyl, pyrrolyl, pyrazolyl, pyrimidinyl and pyrazinyl, wherein each heteroaryl ring contains one or two optional alkyl($C_1$-$C_6$) substituents; $R_6$ and $R_7$ are independently selected from the group consisting of hydrogen; alkyl($C_1$-$C_6$); alkenyl($C_2$-$C_6$); cycloalkyl ($C_3$-$C_6$)alkyl($C_1$-$C_6$); hydroxyalkyl($C_1$-$C_6$); heteroaryl selected from the group consisting of pyridyl, pyrimidinyl, pyrazinyl, thiazolyl, benzothiazolyl, oxazolyl, benzoxazolyl, isoxazolyl, benzisoxazolyl, pyrazolyl, furyl, benzofuryl, thienyl, benzothienyl, thiadiazolyl, pyrrolyl, imidazolyl, benzimidazolyl, indolyl, quinolyl and isoquinolyl, wherein each heteroaryl contains one or two optional substituents independently selected from the group consisting of alkyl($C_1$-$C_6$), Oalkyl($C_1$-$C_6$), haloalkyl($C_1$-$C_6$), cycloalkyl($C_3$-$C_6$), $NO_2$ and COalkyl($C_1$-$C_6$); thienylalkyl($C_1$-$C_6$), furylalkyl($C_1$-$C_6$), pyridylalkyl($C_1$-$C_6$); 3-methyl-2-thienylmethyl; and aryl selected from the group consisting of phenyl and indanyl, wherein each aryl contains one or two optional substituents selected from the group consisting of alkyl($C_1$-$C_6$), haloalkyl($C_1$-$C_6$), halogen, Ndialkyl ($C_1$-$C_6$), NHalkyl($C_1$-$C_6$), Oalkyl($C_1$-$C_6$), $NO_2$, CN, OH, $NH_2$, COOH, Coalkyl($C_1$-$C_6$), Cooalkyl($C_1$-$C_6$), CONHalkyl ($C_1$-$C_6$), CONdialkyl($C_1$-$C_6$) and COphenyl; or $R_6$ and $R_7$ can form, together with the nitrogen atom to which they are attached, a heterocycle selected from pyrrolidine, 3-pyrroline, 1,2,3,6-tetrahydropyridine, piperidine, morpholine, thiomorpholine and piperazine, wherein each heterocycle contains one, two, three or four optional substituents selected from the group consisting of OH, alkyl($C_1$-$C_6$) and COalkyl($C_1$-$C_6$); $R_8$ is selected from the group consisting of hydrogen and alkyl($C_1$-$C_6$); with the proviso that $R_3$ and $R_4$ cannot be hydrogen simultaneously; and pharmaceutically acceptable salts and hydrates thereof.

In a preferred embodiment $R^1$ is selected from the group consisting of hydrogen, alkyl($C_1$-$C_6$), Oalkyl($C_1$-$C_6$), halogen and phenyl; $R^2$ is selected from the group consisting of hydrogen, alkyl($C_1$-$C_6$), halogen and phenyl; and $R_3$, $R_4$, $R_5$, $R_6$, $R_7$ and $R_8$ are as defined above.

In another preferred embodiment, $R_1$ and $R_2$ are independently selected from hydrogen, alkyl($C_1$-$C_6$), halogen, in particular bromine, and phenyl.

In another preferred embodiment, $R_1$ and $R_2$ are independently selected from the group consisting of hydrogen, methyl, bromine and phenyl.

In another preferred embodiment, $R_3$ and $R_4$ are independently selected from the group consisting of hydrogen, phenyl, 4-fluorophenyl, 4-chlorophenyl, *p*-tolyl and 4-methoxyphenyl.

In another preferred embodiment, $R_5$ is selected from the group consisting of $NHNH_2$, N(methyl)$NH_2$, 2-pyridyl, 3-pyridil, 4-pyridyl, 2-pyrazinyl, 5-methyl-2-furyl, 2-thienyl and 2,4,dimethyl-5-oxazolyl.

In another preferred embodiment, $R_5$ is $NR_6R_7$ and $R_6$ and $R_7$ are independently selected from hydrogen, alkyl ($C_1$-$C_6$); alkenyl($C_2$-$C_6$); cycloalkyl($C_3$-$C_6$)alkyl($C_1$-$C_6$); hydroxyalkyl($C_1$-$C_6$); heteroaryl selected from the group consisting of pyridyl; pyrimidinyl, optionally substituted with one or two substituents independently selected from Oalkyl($C_1$-$C_6$) and OH; pyrazinyl; thiazolyl, optionally substituted with one or two substituents independently selected from alkyl($C_1$-$C_6$) and $NO_2$; benzothiazolyl; oxazolyl, optionally substituted with one or two alkyl($C_1$-$C_6$); benzoxazolyl; isoxazolyl, optionally substituted with one or two alkyl($C_1$-$C_6$); benzisoxazolyl; pyrazolyl, optionally substituted with one or two substituents; independently selected from alkyl($C_1$-$C_6$) and cycloalkyl($C_1$-$C_6$); furyl, optionally substituted with one or two alkyl($C_1$-$C_6$); benzofuryl; thienyl, optionally substituted with one or two COalkyl($C_1$-$C_6$); benzothienyl; thiadiazolyl, optionally substituted with one or two haloalkyl($C_1$-$C_6$); pyrrolyl, optionally substituted with one or two alkyl($C_1$-$C_6$); imidazolyl; benzimidazolyl; indolyl, optionally substituted with one or two alkyl($C_1$-$C_6$); quinolyl; and isoquinolyl; thienylalkyl($C_1$-$C_6$), furylalkyl($C_1$-$C_6$), pyridylalkyl($C_1$-$C_6$); phenyl, optionally substituted with one or two substituents independently selected from alkyl($C_1$-$C_6$), haloalkyl($C_1$-$C_6$), Oalkyl($C_1$-$C_6$), halogen, NHalkyl($C_1$-$C_6$), and Ndialkyl($C_1$-$C_6$); and indanyl; or $R_6$ and $R_7$ can form, together with the nitrogen atom to which they are attached, a heterocycle selected from pyrrolidine, 3-pyrroline, optionally substituted with one or two alkyl($C_1$-$C_6$), 1,2,3,6-tetrahydropyridine, piperidine, optionally substituted with one or two substituents independently selected from alkyl($C_1$-$C_6$) and OH, morpholine, optionally substituted with one or two alkyl ($C_1$-$C_6$), thiomorpholine and piperazine, optionally substituted with one or two substituents independently selected from alkyl($C_1$-$C_6$) and COalkyl($C_1$-$C_6$).

In another preferred embodiment, $R_5$ is $NR_6R_7$ and $R_6$ and $R_7$ are independently selected from the group consisting of hydrogen, ethyl, propyl, iso-propyl, butyl, hexyl, allyl, hydroxyethyl, cyclopropylmethyl, 1,3,4-thiadiazol-2-yl, 1,3-dimethyl-5-pyrazolyl, 1-methyl-3-pyrazolyl, 2,5-dimethyl-3-pyrrolinyl, 1,2,3,6-tetrahydropiperidinyl, 2,5-dimethyl-3-pyrazolyl, 2-acetyl-3-thienyl, 2-indanyl, 2-methyl-3-pyrazolyl, 2-methyl-5-indolyl, 2-pirazinyl, 2-pyrimidinyl, 2-quinolyl, 2-thiazolyl, 3,5-isoxazol-4-yl, 3-isoxazolyl, 3-methyl-2-thienylmethyl, 3-methyl-5-isoxazolyl, 3-methyl-6-pyridyl, 3-methylisoxazol-5-yl, 3-oxazolyl, 3-pyrazolyl, 1,3,4-thiadiazol-2-yl, 4-methyl-2-thiazolyl, 5-trifluoromethyl-1,3,4-thiadiazol-2-yl, 5-cyclopropyl-3-pyrazolyl, 5-methyl-2-pyridyl, 5-methyl-3-pyrazolyl, 5-methyl-isoxazol-3-yl, 5-nitro-2-thiazol-2-yl, 6-methoxy-4-pyrimidinyl, 2-furylmethyl, 2-pyridylmethyl, 2-thienylethyl, phenyl, *p*-tolyl, 4-trifluoromethylphenyl, 4-dimethylaminophenyl, 4-fluorophenyl and 4-methoxyphenyl; or $R_6$ and $R_7$ form, together with the nitrogen atom to which they are attached, pyrrole, 2,5-dimethyl-3-pyrroline, piperidine, 1,2,3,6-tetrahydropyridine, 4-hydroxypiperidine, 3,5-dimethylpiperidine, morpholine, 2,6-dimethylmorpholine, 4-methylpiperazine and 4-acetylpiperazine.

The term "pharmaceutically acceptable salts" means salts which are acceptable for administration to a patient, such as a mammal (e.g., salts having acceptable mammalian safety for a given dosage regime). Such salts can be derived from pharmaceutically acceptable inorganic or organic bases and from pharmaceutically acceptable inorganic or organic acids. Salts derived from pharmaceutically acceptable inorganic bases include ammonium, calcium, copper, ferric, ferrous, lithium, magnesium, manganic, manganous, potassium, sodium, zinc and the like. Particularly preferred are ammonium, calcium, magnesium, potassium and sodium salts. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable acids include acetic, ascorbic, benzenesulfonic, benzoic, camphosulfonic, citric, ethanesulfonic, edisylic, fumaric, gentisic, gluconic, glucoronic, glutamic, hippuric, hydrobromic, hydrochloric, isethionic, lactic, lactobionic, maleic, malic, mandelic, methanesulfonic, mucic, naphthalenesulfonic, naphthalene-1,5-disulfonic, naphthalene-2,6-disulfonic, nicotinic, nitric, orotic, pamoic, pantothenic, phosphoric, succinic, sulfuric, tartaric, *p*-toluenesulfonic, xinafoic and the like. Particularly preferred are citric, hydrobromic, hydrochloric, isethionic, maleic, naphthalene-1,5-disulfonic, phosphoric, sulfuric and tartaric acids. Halogen or halo means F, Cl, Br, and I.

Alkyl($C_1$-$C_6$) means a straight or branched alkyl chain with 1 to 6 carbon atoms such as methyl, ethyl, n-propyl, I-propyl, n-butyl, sec-butyl, 1-butyl, t-butyl, n-pentyl and n-hexyl.

Alkenyl($C_2$-$C_6$) means a straight or branched alkenyl chain with 2 to 6 carbon atoms such as allyl.

Specific compounds of formula (I) are selected from the group consisting of:

5-Methyl-1-(2-morpholin-4-yl-2-oxo-ethyl)-2-phenyl-1H-quinolin-4-one;
N,N-Diethyl-2-(7-methyl-4-oxo-2-phenyl-4H-quinolin-1-yl)-acetamide;
7-Methyl-1-(2-morpholin-4-yl-2-oxo-ethyl)-2-phenyl-1H-quinolin-4-one;
(7-Methyl-4-oxo-2-phenyl-4H-quinolin-1-yl)-acetic acid ethyl ester;
N,N-Dibutyl-2-(7-methyl-4-oxo-2-phenyl-4H-quinolin-1-yl)-acetamide;
N,N-Diisopropyl-2-(5-methyl-4-oxo-2-phenyl-4H-quinolin-1-yl)-acetamide;
5-Methyl-1-(2-oxo-2-pyrrolidin-1-yl-ethyl)-2-phenyl-1H-quinolin-4-one;
2-(7-Methyl-4-oxo-2-phenyl-4H-quinolin-1-yl)-N,N-dipropyl-acetamide;
N,N-Dihexyl-2-(5-methyl-4-oxo-2-phenyl-4H-quinolin-1-yl)-acetamide;
5-Methyl-1-(2-oxo-2-piperidin-1-yl-ethyl)-2-phenyl-1H-quinolin-4-one;
5-Methyl-1-(2-oxo-2-pyrrolidin-1-yl-ethyl)-2-phenyl-1H-quinolin-4-one;
7-Methyl-1-(2-oxo-2-pyrrolidin-1-yl-ethyl)-2-phenyl-1H-quinolin-4-one;
N,N-Dibutyl-2-(7-methyl-4-oxo-3-phenyl-4H-quinolin-1-yl)-acetamide;
2-(7-Methyl-4-oxo-3-phenyl-4H-quinolin-1-yl)-N,N-dipropyl-acetamide;
7-Methyl-1-(2-oxo-2-piperidin-1-yl-ethyl)-3-phenyl-1H-quinolin-4-one;
N,N-Dihexyl-2-(7-methyl-4-oxo-3-phenyl-4H-quinolin-1-yl)-acetamide;
N,N-Diethyl-2-(7-methyl-4-oxo-3-phenyl-4H-quinolin-1-yl)-acetamide;
7-Methyl-1-(2-oxo-2-pyrrolidin-1-yl-ethyl)-3-phenyl-1H-quinolin-4-one;
7-Methyl-1-(2-morpholin-4-yl-2-oxo-ethyl)-3-phenyl-1H-quinolin-4-one;
N,N-Diisopropyl-2-(7-methyl-4-oxo-3-phenyl-4H-quinolin-1-yl)-acetamide;
N,N-Dihexyl-2-(5-methyl-4-oxo-3-phenyl-4H-quinolin-1-yl)-acetamide;
5-Methyl-1-(2-oxo-2-piperidin-1-yl-ethyl)-3-phenyl-1H-quinolin-4-one;
5-Methyl-1-(2-morpholin-4-yl-2-oxo-ethyl)-3-phenyl-1H-quinolin-4-one;
N,N-Diethyl-2-(5-methyl-4-oxo-3-phenyl-4H-quinolin-1-yl)-acetamide;
N,N-Dibutyl-2-(5-methyl-4-oxo-3-phenyl-4H-quinolin-1-yl)-acetamide;
7-Methyl-1-(2-oxo-2-pyridin-3-yl-ethyl)-3-phenyl-1H-quinolin-4-one;
7-Methyl-1-(2-oxo-2-thiophen-2-yl-ethyl)-3-phenyl-1H-quinolin-4-one;
7-Methyl-1-(2-oxo-2-pyridin-2-yl-ethyl)-3-phenyl-1H-quinolin-4-one;
7-Methyl-1-(2-oxo-2-pyridin-4-yl-ethyl)-3-phenyl-1H-quinolin-4-one;
1-[2-(4-Acetyl-piperazin-1-yl)-2-oxo-ethyl]-7-methyl-3-phenyl-1H-quinolin-4-one;
7-Methyl-1-[2-(5-methyl-furan-2-yl)-2-oxo-ethyl]-3-phenyl-1H-quinolin-4-one;
1-[2-(2,6-Dimethyl-morpholin-4-yl)-2-oxo-ethyl]-7-methyl-3-phenyl-1H-quinolin-4-one;
1-[2-(2,4-Dimethyl-oxazol-5-yl)-2-oxo-ethyl]-7-methyl-3-phenyl-1H-quinolin-4-one;
7-Methyl-1-(2-oxo-2-pyrazin-2-yl-ethyl)-3-phenyl-1H-quinolin-4-one;

7-Methyl-1-[2-(4-methyl-piperazin-1-yl)-2-oxo-ethyl]-3-phenyl-1H-quinolin-4-one;
2-[3-(4-Methoxy-phenyl)-7-methyl-4-oxo-4H-quinolin-1-yl]-N,N-dipropyl-acetamide;
2-[3-(4-Chloro-phenyl)-7-methyl-4-oxo-4H-quinolin-1-yl]-N,N-dipropyl-acetamide;
2-(7-Methyl-4-oxo-3-*p*-tolyl-4H-quinolin-1-yl)-N,N-dipropyl-acetamide;
N-Cyclopropylmethyl-2-(7-methyl-4-oxo-3-phenyl-4H-quinolin-1-yl)-N-propyl-acetamide;
1-[2-(3,5-Dimethyl-piperidin-1-yl)-2-oxo-ethyl]-7-methyl-3-phenyl-1H-quinolin-4-one;
1-[2-(3,6-Dihydro-2H-pyridin-1-yl)-2-oxo-ethyl]-7-methyl-3-phenyl-1H-quinolin-4-one;
1-[2-(2,5-Dimethyl-2,5-d ihydro-pyrrol-1-yl)-2-oxo-ethyl]-7-methyl-3-phenyl-1H-quinolin-4-one;
N,N-Diallyl-2-(7-methyl-4-oxo-3-phenyl-4H-quinolin-1-yl)-acetamide;
2-(7-Bromo-4-oxo-3-phenyl-4H-quinolin-1-yl)-N,N-dipropyl-acetamide;
2-(5-Bromo-4-oxo-3-phenyl-4H-quinolin-1-yl)-N,N-dipropyl-acetamide;
2-(4-Oxo-3,7-diphenyl-4H-quinolin-1-yl)-N,N-dipropyl-acetamide;
2-(4-Oxo-3-phenyl-4H-quinolin-1-yl)-N,N-dipropyl-acetamide;
2-(4-Oxo-3,5-diphenyl-4H-quinolin-1-yl)-N,N-dipropyl-acetamide;
(7-Methyl-4-oxo-3-*p*-tolyl-4H-quinolin-1-yl)-acetic acid methyl ester;
(7-Methyl-4-oxo-3-*p*-tolyl-4H-quinolin-1-yl)-acetic acid;
[3-(4-Chloro-phenyl)-7-methyl-4-oxo-4H-quinolin-1-yl]-acetic acid methyl ester;
[3-(4-Fluoro-phenyl)-7-methyl-4-oxo-4H-quinolin-1-yl]-acetic acid methyl ester;
[3-(4-Fluoro-phenyl)-7-methyl-4-oxo-4H-quinolin-1-yl]-acetic acid;
2-[3-(4-Chloro-phenyl)-7-methyl-4-oxo-4H-quinolin-1-yl]-N-phenyl-acetamide;
2-[3-(4-Chloro-phenyl)-7-methyl-4-oxo-4H-quinolin-1-yl]-N-pyrazin-2-yl-acetamide;
[3-(4-Chloro-phenyl)-7-methyl-4-oxo-4H-quinolin-1-yl]-acetic acid;
[3-(4-Chloro-phenyl)-7-methyl-4-oxo-4H-quinolin-1-yl]-acetic acid hydrazide;
2-(7-Methyl-4-oxo-3-*p*-tolyl-4H-quinolin-1-yl)-N-phenyl-acetamide;
2-[3-(4-Fluoro-phenyl)-7-methyl-4-oxo-4H-quinolin-1-yl]-N-phenyl-acetamide;
2-(7-Methyl-4-oxo-3-*p*-tolyl-4H-quinolin-1-yl)-N-thiazol-2-yl-acetamide;
N-(5-Methyl-isoxazol-3-yl)-2-(7-methyl-4-oxo-3-*p*-tolyl-4H-quinolin-1-yl)-acetamide;
[3-(4-Fluoro-phenyl)-7-methyl-4-oxo-4H-quinolin-1-yl]-acetic acid hydrazide;
(7-Methyl-4-oxo-3-*p*-tolyl-4H-quinolin-1-yl)-acetic acid hydrazide;
2-[3-(4-Chloro-phenyl)-7-methyl-4-oxo-4H-quinolin-1-yl]-N-(5-methyl-isoxazol-3-yl)-acetamide;
2-[3-(4-Fluoro-phenyl)-7-methyl-4-oxo-4H-quinolin-1-yl]-N-(5-methyl-isoxazol-3-yl)-acetamide;
N-Ethyl-N-(2-hydroxy-ethyl)-2-(7-methyl-4-oxo-3-phenyl-4H-quinolin-1-yl)-acetamide;
N-(2-Hydroxy-ethyl)-2-(7-methyl-4-oxo-3-phenyl-4H-quinolin-1-yl)-N-propyl-acetamide;
N,N-Diallyl-2-(5-methyl-4-oxo-3-phenyl-4H-quinolin-1-yl)-acetamide;
N-(2-Hydroxy-ethyl)-2-(5-methyl-4-oxo-3-phenyl-4H-quinolin-1-yl)-N-propyl-acetamide;
(7-Methyl-4-oxo-3-phenyl-4H-quinolin-1-yl)-acetic acid;
(5-Methyl-4-oxo-3-phenyl-4H-quinolin-1-yl)-acetic acid;
[3-(4-Chloro-phenyl)-7-methyl-4-oxo-4H-quinolin-1-yl]-acetic acid N-methyl-hydrazide;
(7-Methyl-4-oxo-3-*p*-tolyl-4H-quinolin-1-yl)-acetic acid N-methyl-hydrazide;
[3-(4-F)uoro-phenyl)-7-methyl-4-oxo-4H-quinolin-1-yl]-acetic acid N-methyl-hydrazide;
2-[3-(4-Fluoro-phenyl)-7-methyl-4-oxo-4H-quinolin-1-yl]-N-thiazol-2-yl-acetamide;
2-[3-(4-Chloro-phenyl)-7-methyl-4-oxo-4H-quinolin-1-yl]-N-thiazol-2-yl-acetamide;
2-[3-(4-Methoxy-phenyl)-7-methyl-4-oxo-4H-quinolin-1-yl]-N-(4-trifluoromethyl-phenyl)-acetamide;
2-(7-Methyl-4-oxo-3-phenyl-4H-quinolin-1-yl)-N-(4-trifluoromethyl-phenyl)-acetamide;
N-(4-Methoxy-phenyl)-2-(7-methyl-4-oxo-3-phenyl-4H-quinolin-1-yl)-acetamide;
N-(4-Methoxy-phenyl)-2-[3-(4-methoxy-phenyl)-7-methyl-4-oxo-4H-quinolin-1-yl]-acetamide;
2-[3-(4-Methoxy-phenyl)-7-methyl-4-oxo-4H-quinolin-1-yl]-N-(5-methyl-2H-pyrazol-3-yl)-acetamide;
2-(7-Methyl-4-oxo-3-phenyl-4H-quinolin-1-yl)-N-thiazol-2-yl-acetamide;
2-[3-(4-Methoxy-phenyl)-7-methyl-4-oxo-4H-quinolin-1-yl]-N-thiazol-2-yl-acetamide;
2-(7-Methyl-4-oxo-3-phenyl-4 H-quinolin-1-yl)-N-(3-methyl-thiophen-2-ylmethyl)-acetamide;
2-[3-(4-Methoxy-phenyl)-7-methyl-4-oxo-4H-quinolin-1-yl]-N-(3-methyl-isoxazol-5-yl)-acetamide;
2-[3-(4-Methoxy-phenyl)-7-methyl-4-oxo-4H-quinolin-1-yl]-N-(5-methyl-pyridin-2-yl)-acetamide;
2-(7-Methyl-4-oxo-3-phenyl-4H-quinolin-1-yl)-N-pyrimidin-2-yl-acetamide;
N-Furan-2-ylmethyl-2-(7-methyl-4-oxo-3-phenyl-4H-quinolin-1-yl)-acetamide;
N-(2,5-Dimethyl-2H-pyrazol-3-yl)-2-[3-(4-methoxy-phenyl)-7-methyl-4-oxo-4H-quinolin-1-yl]-acetamide;
N,N-Diethyl-2-[3-(4-methoxy-phenyl)-7-methyl-4-oxo-4H-quinolin-1-yl]-acetamide;
3-(4-Methoxy-phenyl)-7-methyl-1-(2-oxo-2-pyrrolidin-1-yl-ethyl)-1H-quinolin-4-one;
3-(4-Methoxy-phenyl)-7-methyl-1-(2-oxo-2-piperidin-1-yl-ethyl)-1H-quinolin-4-one;

N-Ethyl-N-(2-hydroxy-ethyl)-2-[3-(4-methoxy-phenyl)-7-methyl-4-oxo-4H-quinolin-1-yl]-acetamide;
N-(2-Hydroxy-ethyl)-2-[3-(4-methoxy-phenyl)-7-methyl-4-oxo-4H-quinolin-1-yl]-N-propyl-acetamide;
N-Cyclopropylmethyl-2-[3-(4-methoxy-phenyl)-7-methyl-4-oxo-4H-quinolin-1-yl]-N-propyl-acetamide;
N,N-Bis-(2-hydroxy-ethyl)-2-[3-(4-methoxy-phenyl)-7-methyl-4-oxo-4H-quinolin-1-yl]-acetamide;
N,N-Diallyl-2-[3-(4-methoxy-phenyl)-7-methyl-4-oxo-4H-quinolin-1-yl]-acetamide;
N-(3,5-Dimethyl-isoxazol-4-yl)-2-(7-methyl-4-oxo-3-phenyl-4H-quinolin-1-yl)-acetamide;
2-(7-Methyl-4-oxo-3-phenyl-4H-quinolin-1-yl)-N-(1-methyl-1H-pyrazol-3-yl)-acetamide;
N-(2,5-Dimethyl-2H-pyrazol-3-yl)-2-(7-methyl-4-oxo-3-phenyl-4H-quinolin-1-yl)-acetamide;
2-(7-Methyl-4-oxo-3-phenyl-4H-quinolin-1-yl)-N-(5-methyl-pyridin-2-yl)-acetamide;
2-(7-Methyl-4-oxo-3-phenyl-4H-quinolin-1-yl)-N-[1,3,4]thiadiazol-2-yl-acetamide;
N-(4-Dimethylamino-phenyl)-2-(7-methyl-4-oxo-3-phenyl-4H-quinolin-1-yl)-acetamide;
N-(3-Methyl-isoxazol-5-yl)-2-(7-methyl-4-oxo-3-phenyl-4H-quinolin-1-yl)-acetamide;
2-(7-Methyl-4-oxo-3-phenyl-4H-quinolin-1-yl)-N-*p*-tolyl-acetamide;
2-[3-(4-Methoxy-phenyl)-7-methyl-4-oxo-4H-quinolin-1-yl]-N-(1-methyl-1H-pyrazol-3-yl)-acetamide;
1-[2-(2,5-Dimethyl-2,5-dihydro-pyrrol-1-yl)-2-oxo-ethyl]-3-(4-methoxy-phenyl)-7-methyl-1H-quinolin-4-one;
2-(7-Methyl-4-oxo-3-phenyl-4H-quinolin-1-yl)-N-(5-methyl-2H-pyrazol-3-yl)-acetamide;
2-(7-Methyl-4-oxo-3-phenyl-4H-quinolin-1-yl)-N-pyridin-2-ylmethyl-acetamide;
N-(2-Acetyl-thiophen-3-yl)-2-(7-methyl-4-oxo-3-phenyl-4H-quinolin-1-yl)-acetamide;
N-Ethyl-2-(7-methyl-4-oxo-3-phenyl-4H-quinolin-1-yl)-N-[1,3,4]thiadiazol-2-yl-acetamide;
N-(2-Methyl-1H-indol-5-yl)-2-(7-methyl-4-oxo-3-phenyl-4H-quinolin-1-yl)-acetamide;
1-[2-(3,6-Dihydro-2H-pyridin-1-yl)-2-oxo-ethyl]-7-methyl-3-phenyl-1H-quinolin-4-one;
2-(7-Methyl-4-oxo-3-phenyl-4H-quinolin-1-yl)-N-(1H-pyrazol-3-yl)-acetamide;
N-(4-Fluoro-phenyl)-2-(7-methyl-4-oxo-3-phenyl-4H-quinolin-1-yl)-acetamide;
2-(7-Methyl-4-oxo-3-phenyl-4H-quinolin-1-yl)-N-(5-trifluoromethyl-[1,3,4]thiadiazol-2-yl)-acetamide;
2-(7-Methyl-4-oxo-3-phenyl-4H-quinolin-1-yl)-N-quinolin-2-yl-acetamide;
2-(7-Methyl-4-oxo-3-phenyl-4H-quinolin-1-yl)-N-(2-methyl-2H-pyrazol-3-yl)-acetamide;
N-(5-Cyclopropyl-2H-pyrazol-3-yl)-2-(7-methyl-4-oxo-3-phenyl-4H-quinolin-1-yl)-acetamide;
N-Isoquinolin-3-yl-2-(7-methyl-4-oxo-3-phenyl-4H-quinolin-1-yl)-acetamide;
N-Indan-2-yl-2-(7-methyl-4-oxo-3-phenyl-4H-quinolin-1-yl)-acetamide;
2-(7-Methyl-4-oxo-3-phenyl-4H-quinolin-1-yl)-N-(2-thiophen-2-yl-ethyl)-acetamide;
7-Bromo-1-[2-(3,6-dihydro-2H-pyridin-1-yl)-2-oxo-ethyl]-3-phenyl-1H-quinolin-4-one;
(7-Bromo-4-oxo-3-phenyl-4H-quinolin-1-yl)-acetic acid hydrazide;
2-(7-Bromo-4-oxo-3-phenyl-4H-quinolin-1-yl)-N,N-dibutyl-acetamide:
7-Bromo-1-(2-oxo-2-pyrrolidin-1-yl-ethyl)-3-phenyl-1H-quinolin-4-one;
7-Bromo-1-(2-oxo-2-piperidin-1-yl-ethyl)-3-phenyl-1H-quinolin-4-one;
7-Bromo-1-[2-(2,6-dimethyl-morpholin-4-yl)-2-oxo-ethyl]-3-phenyl-1H-quinolin-4-one;
N,N-Diallyl-2-(7-bromo-4-oxo-3-phenyl-4H-quinolin-1-yl)-acetamide;
7-Bromo-1-[2-(2,5-dimethyl-2,5-dihydro-pyrrol-1-yl)-2-oxo-ethyl]-3-phenyl-1H-quinolin-4-one;
2-(7-Bromo-4-oxo-3-phenyl-4H-quinolin-1-yl)-N-cyclopropylmethyl-N-propyl-acetamide;
2-(7-Methyl-4-oxo-3-phenyl-4H-quinolin-1-yl)-N-(5-nitro-thiazol-2-yl)-acetamide;
N-Isoxazol-3-yl-2-(7-methyl-4-oxo-3-phenyl-4H-quinolin-1-yl)-acetamide;
2-(7-Methyl-4-oxo-3-phenyl-4H-quinolin-1-yl)-N-(4-methyl-thiazol-2-yl)-acetamide;
7-Bromo-1-(2-oxo-2-piperidin-1-yl-ethyl)-3-phenyl-1H-quinolin-4-one;
(7-Methyl-4-oxo-3-phenyl-4H-quinolin-1-yl)-acetic acid methyl ester;
(7-Methyl-4-oxo-3-phenyl-4H-quinolin-1-yl)-acetic acid;
[3-(4-Methoxy-phenyl)-7-methyl-4-oxo-4H-quinolin-1-yl]-acetic acid methyl ester;
[3-(4-Methoxy-phenyl)-7-methyl-4-oxo-4H-quinolin-1-yl]-acetic acid;
(7-Bromo-4-oxo-3-phenyl-4H-quinolin-1-yl)-acetic acid;
1-[2-(2,6-Dimethyl-morpholin-4-yl)-2-oxo-ethyl]-3-(4-methoxy-phenyl)-7-methyl-1H-quinolin-4-one;
1-[2-(3,5-Dimethyl-piperidin-1-yl)-2-oxo-ethyl]-3-(4-methoxy-phenyl)-7-methyl-1H-quinolin-4-one;
N-(6-Methoxy-pyrimidin-4-yl)-2-(7-methyl-4-oxo-3-phenyl-4H-quinolin-1-yl)-acetamide;
1-[2-(4-Hydroxy-piperidin-1-yl)-2-oxo-ethyl]-7-methyl-3-phenyl-1H-quinolin-4-one;
N-Isoxazol-3-yl-2-[3-(4-methoxy-phenyl)-7-methyl-4-oxo-4H-quinolin-1-yl]-acetamide; and
1-[2-(4-Hydroxy-piperidin-1-yl)-2-oxo-ethyl]-3-(4-methoxy-phenyl)-7-methyl-1H-quinolin-4-one.

Another embodiment of the present invention is to provide a process for preparing the compounds of formula (I).

The compounds of general structure (I) can be obtained following the synthetic strategy showed below (Scheme 1):

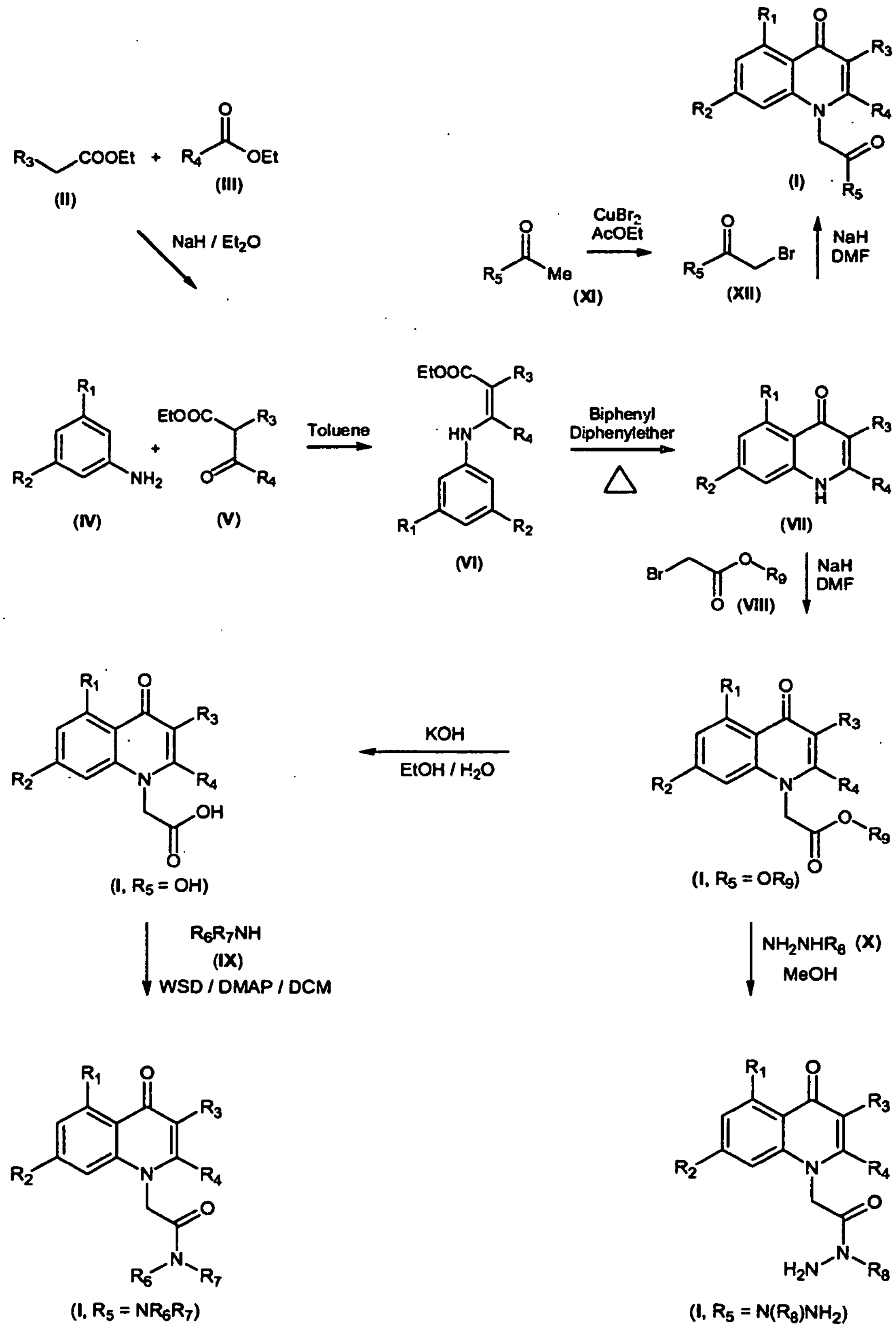
(I)
(II)
(III)
NaH / $Et_2O$
$CuBr_2$
AcOEt
(XI)
(XII)
NaH
DMF
(IV)
(V)
Toluene
(VI)
Biphenyl
Diphenylether
(VII)
NaH
DMF
(VIII)
KOH
EtOH / $H_2O$
(I, $R_5$ = OH)
(I, $R_5$ = $OR_9$)
$R_6R_7NH$
(IX)
WSD / DMAP / DCM
$NH_2NHR_8$ (X)
MeOH
(I, $R_5$ = $NR_6R_7$)
(I, $R_5$ = $N(R_8)NH_2$)

Scheme 1

Starting from a Claisen condensation between two esters (**II**) and (**III**), it is possible to obtain the 1,3-dicarbonylic system (**V**), which in reaction with the aniline (**IV**) in toluene yields the enamine (**VI**). The Friedel-Crafts intramolecular condensation of (**VI**) at high temperature affords the quinolone system (**VII**). This quinolone can be converted into compounds of general structure (**I**) by three different ways. The first one is the N-alkylation of (**VII**) by using a substituted 2-bromo ethanone (**XII**, commercially available or synthesized from (**XI**) by bromination). On the other hand, the latter quinolone (**VII**) can be N-substituted by using an alkyl bromoacetate (**VIII**) to yield (I, $R_5$ = $OR_9$). Methyl and ethyl bromoacetates are preferred. More preferred is methyl bromoacetate. The ester group present in (I, $R_5$ = $OR_9$) can be substituted by an hydrazine (**X**), giving the final hydrazide compound (I, $R_5$ = $N(R_8)NH_2$), Finally, esters (I, $R_5$ = $OR_9$) can be converted into acids (I, $R_5$ = OH) by conventional saponification, and these acids, by coupling with amines (**IX**), yield compounds (**I**) when the functional group to obtain is an amide.

The compounds of the present invention formula I, wherein $R_5$ may be in addition OH or ORg with Rg being alkyl ($C_1$-$C_6$) or their pharmaceutically acceptable salts or hydrates can be used for the preparation of a medicament for treating or preventing diseases associated with $GABA_A$ receptor modulation in a human or non-human mammal. More specifically, diseases associated with $GABA_A$ receptor modulation comprise diseases associated with $\alpha_1$-GABAA receptor modulation and/or $\alpha_2$-$GABA_A$ receptor modulation. A non-limitative list of such diseases comprises anxiety, epilepsy, sleep disorders, including insomnia, and the like.

Another embodiment of the present invention is to provide the use of a compound of the present invention or a pharmaceutically acceptable salt or hydrate thereof for the preparation of a medicament for treating or preventing anxiety in a human or non-human mammal.

Another embodiment of the present invention is to provide the use of a compound of the present invention or a pharmaceutically acceptable salt or hydrate thereof for the preparation of a medicament for treating or preventing epilepsy in a human or non-human mammal in need thereof.

Another embodiment of the present invention is to provide the use of a compound of the present invention or a pharmaceutically acceptable salt or hydrate thereof for the preparation of a medicament for treating or preventing sleep disorders in a human or non-human mammal in need thereof.

Another embodiment of the present invention is to provide the use of a compound of the present invention or a pharmaceutically acceptable salt or hydrate thereof for the preparation of a medicament for treating or preventing insomnia in a human or non-human mammal in need thereof.

Another embodiment of the present invention is to provide the use of a compound of the present invention or a pharmaceutically acceptable salt or hydrate thereof for the preparation of a medicament for inducing sedation-hypnosis in a human or non-human mammal in need thereof.

Another embodiment of the present invention is to provide the use of a compound of the present invention or a pharmaceutically acceptable salt or hydrate thereof for the preparation of a medicament for inducing anesthesia in a human or non-human mammal in need thereof.

Another embodiment of the present invention is to provide the use of a compound of the present invention or a pharmaceutically acceptable salt or hydrate thereof for the preparation of a medicament for modulating the necessary time to induce sleep and its duration in a human or non-human mammal in need thereof.

Another embodiment of the present invention is to provide the use of a compound of the present invention or a pharmaceutically acceptable salt or hydrate thereof for the preparation of a medicament for inducing muscle relaxation in a human or non-human mammal in need thereof.

Treatment or prevention of a human or non-human mammal suffering from diseases associated with $GABA_A$ receptor modulation in a human or non-human mammal comprises administering to said human or non-human mammal in need thereof a therapeutically effective amount of a compound of the present invention or pharmaceutically acceptable salts or hydrates thereof, together with pharmaceutically acceptable diluents or carriers. More specifically, diseases associated with $GABA_A$ receptor modulation comprise diseases associated with $\alpha_1$-$GABA_A$ receptor modulation and/or $\alpha_2$-$GABA_A$ receptor modulation. A non-limitative list of such diseases comprises anxiety, epilepsy, sleep disorders, including insomnia, and the like.

Further preferred embodiments of the invention relate to:

treating or preventing diseases associated with the $GABA_A$ receptor modulation in a human or non-human mamal in need thereof, which comprises administering to said mammal an effective amount of a compound of formula I as defined above, wherein, preferably, the $GABA_A$ receptor is the $\alpha_1$-$GABA_A$ receptor and/or a $\alpha_2$-$GABA_A$ receptor;

treating or preventing anxiety in a human or non-human mammal in need thereof, which comprises administering to said mammal an effective amount of a compound of formula I as defined above;

treating or preventing epilepsy in a human or non-human mammal in need thereof, which comprises administering to said mammal an effective amount of a compound of formula I as defined above;

treating or preventing sleep disorders in a human or non-human mammal in need thereof, which comprises administering to said mammal an effective amount of a compound of formula I as defined above;

treating or preventing insomnia in a human or non-human mammal in need thereof, which comprises administering to said mammal an effective amount of a compound of formula I as defined above;

inducing sedation hypnosis in a human or non-human mammal in need thereof, which comprises administering to said mammal an effective amount of a compound of formula I as defined above;

inducing anesthesia in a human or non-human mammal in need thereof, which comprises administering to said mammal an effective amount of a compound of formula I as defined above;

for modulating the necessary time to induce sleep and its duration in a human or non-human mammal in need thereof, which comprises administering to said mammal an effective amount of a compound of formula I as defined above; and

inducing muscle relaxation in a human or non-human mammal in need thereof, which comprises administering to said mammal an effective amount of a compound of formula I as defined above.

As used herein, the term "mammal" shall refer to the Mammalian class of higher vertebrates. The term "mammal" includes, but is not limited to, a human.

Another embodiment of the present invention is to provide a pharmaceutical composition containing a compound of the present invention or pharmaceutically acceptable salts and hydrates thereof, in association with therapeutically inert carriers.

The compositions include those suitable for oral, rectal and parenteral (including subcutaneous, intramuscular, and intravenous) administration, although the most suitable route will depend on the nature and severity of the condition being treated. The most preferred route of the present invention is the oral route. The compositions may be conveniently presented in unit dosage form, and prepared by any of the methods well known in the art of pharmacy.

The active compound can be combined with a pharmaceutical carrier according to conventional pharmaceutical compounding techniques. The carrier may take a wide variety of forms depending on the form of the preparation desired for administration, e.g. oral or parenteral (including intravenous injections or infusions). In preparing the compositions for oral dosage form any of the usual pharmaceutical media may be employed. Usual pharmaceutical media include, for example, water, glycols, oils, alcohols, flavoring agents, preservatives, coloring agents, and the like, in the case of oral liquid preparations (such as for example, suspensions, solutions, emulsions and elixirs); aerosols; or carriers such as starches, sugars, microcrystalline cellulose, diluents, granulating agents, lubricants, binders, disintegrating agents and the like, in the case of oral solid preparations (such as for example, powders, capsules, and tablets) with the oral solid preparations being preferred over the oral liquid preparations.

Because of their ease of administration, tablets and capsules represent the most advantageous oral dosage unit form, in which case solid pharmaceutical carriers are employed. If desired, tablets may be coated by standard aqueous or non-aqueous techniques.

A suitable dosage range for use is from about 0.01 mg to about 100.00 mg total daily dose, given as a once daily administration or in divided doses if required.

In accordance with the results obtained, certain compounds of the present invention have evidenced pharmacological activity both *in vitro* and *in vivo,* which has been similar to or higher than that of prior-art compound zolpidem. All these results support!their use in diseases or conditions modulated by $\alpha_1$- and $\alpha_2$-$GABA_A$ receptors, such as insomnia or anesthesia, in which an induction of sleep, an induction of sedation or an induction of muscle relaxation are needed.

Thus, surprisingly, some compounds of the present invention have shown similar or higher affinity for $\alpha_1$-subunit of $GABA_A$ receptor, which is involved in the sedative activity. Moreover, this higher affinity correlates well with the *in vivo* activity in a predictive model of sedative-hypnotic activity, particularly for compounds of examples 1, 11, 14, 39, 44 and 129 (Table 3 below).

Furthermore, some compounds, in particularly compounds of examples 15, 18 and 32 of the present invention, have displayed higher $\alpha_1$- / $\alpha_2$-selectivity than the most selective therapeutically used compound zolpidem.

In addition, some compounds of the present invention have exhibited a higher affinity than prior art compound zolpidem for the $\alpha_2$-subunit of $GABA_A$ receptor, which is involved in anxiety. Compounds of examples 27 and 43 are particularly effective in anxiety related syndromes.

The pharmacological activity of the compounds of the present invention has been determined as described below.

a) Ligand-binding assays. Determination of the affinity of test compounds for $\alpha_1$- and $\alpha_2$-$GABA_A$ receptor

Male Sprague-Dawley rats weighing 200-250 g at the time of experiment were used. After decapitation of the animal, the cerebellum (tissue that mostly contains $\alpha_1$-$GABA_A$ receptor) and spinal cord (tissue that mostly contains $\alpha_2$-$GABA_A$ receptor) were removed. The membranes were prepared according to the method by J. Lameh et al. (Prog. Neuro-Psychopharmacol. Biol. Psychiatry, 24, 979-991, 2000) and H. Noguchi et al. (Eur J Pharm, 434, 21-28, 2002) with slight modifications. Once the tissues weighed, they were suspended in 50 mM Tris·HCl (pH 7.4), 1:40 (v/v), or sucrose 0.32 M in the case of spinal cord, homogenized and then centrifuged at 20,000 g for 10 min at 7°C. The resulting pellet was resuspended under the same conditions and centrifuged again. The pellet was finally resuspended on a minimum volume and kept at -80°C overnight. On the next day, the process was repeated until the final pellet was resuspended at a ratio of 1:10 (v/v) in the case of cerebellum and at a ratio of 1:5 (v/v) in the case of spinal cord.

Affinity was determined by competitive tests using radiolabeled flumazenil as ligand. The tests were performed according to the methods described by S. Arbilla et al. (Eur.. J. Pharmacol., 130, 257-263, 1986); and Y. Wu et al. (Eur. J. Pharmacol., 278, 125-132, 1995) using 96-well microtiter plates. The membranes containing the study receptors, flumazenil (radiolabeling at a final concentration of 1 nM) and ascending concentrations of test compounds (in a total volume of 230 μL in 50 mM [pH 7.4] Tris·HCl buffer) were incubated. Simultaneously, the membranes were only incubated with the radiolabeled flumazenil (total binding, 100%) and in the presence of an elevated concentration of unradiolabeled flumazenil (non-specific binding, % estimation of radiolabeled ligand). The reactions started on adding the radiolabeled ligand followed by incubation for 60 minutes at 4°C. At the end of the incubation period, 200 μL of reaction were transferred to a multiscreen plate (Millipore) and filtered using a vacuum manifold and then washed three times with cold test buffer. The multiscreen plates were equipped with a GF/B filter that retained the membranes containing the receptors and the radiolabeled ligand which had been bound to the receptors. After washing, the plates were left till dry. Once dried, scintillation liquid was added and left under stirring overnight. The next day the plates were counted using a Perkin-Elmer Microbeta scintillation counter.

For analysis of the results the percentage of specific binding for every concentration of test compound was calculated as follows:

$$\text{\% specific binding} = (X\text{-}N/T\text{-}N) \times 100$$

where,

X: amount of bound ligand for every concentration of compound.

T: total binding, maximum amount bound to the radiolabeled ligand.

N: non-specific binding, amount of radiolabeled ligand bound in a non-specific way irrespective of the receptor used.

Every concentrations of compound were tested in triplicate and their mean values were used to determine the experimental values of % specific binding versus the concentration of compound. Affinity data are expressed as % inhibition at $10^{-5}$M and $10^{-7}$M concentrations. The results of these tests are given in Tables 1 and 2.

Table 1. Affinity for the $\alpha_1$-subunit of the $GABA_A$ receptor

| 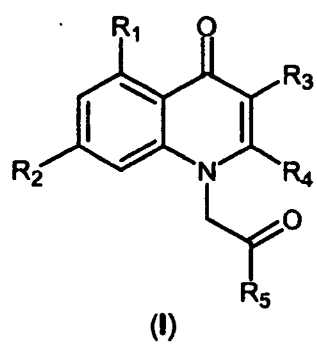 (I) | | | | | | % inhibition | |
|---|---|---|---|---|---|---|---|
| **Example** | **$R_1$** | **$R_2$** | **$R_3$** | **$R_4$** | **$R_5$** | **$10^{-5}$M** | **$11^{-7}$M** |
| 1 | Me | H | H | Ph | N-Morpholinyl | 99.5 | 57.9 |
| 11 | Me | H | H | Ph | N-Pyrrolidinyl | 99.9 | 52.6 |

(continued)

| Example | $R_1$ | $R_2$ | $R_3$ | $R_4$ | $R_5$ | $10^{-5}$M | $11^{-7}$M |
|---|---|---|---|---|---|---|---|
| 14 | H | Me | Ph | H | N(n-Pr)$_2$ | 98.4 | 36.7 |
| 15 | H | Me | Ph | H | N-Piperidinyl | 87.3 | 0.0 |
| 17 | H | Me | Ph | H | NEt$_2$ | 91.4 | 0.7 |
| 18 | H | Me | Ph | H | N-Pyrrolidinyl | 86.2 | 7.5 |
| 27 | H | Me | Ph | H | 2-Thienyl | 97.7 | 14.0 |
| 28 | H | Me | Ph | H | 2-Pyridyl | 97.9 | 22.5 |
| 32 | H | Me | Ph | H | 2,6-Dimethyl-N-Morpholinyl | 86.2 | 18.4 |
| 33 | H | Me | Ph | H | 2,4-Dimethyl-5-Oxazolyl | 97.0 | 33.9 |
| 36 | H | Me | 4-MeOPh | H | N(n-Pr)$_2$ | 95.9 | 20.3 |
| 39 | H | Me | Ph | H | N(Cyclopropylmethyl)(Pr) | 98.8 | 56.3 |
| 42 | H | Me | Ph | H | 2,5-Dimethyl-3-Pyrrolinyl | 97.0 | 25.6 |
| 43 | H | Me | Ph | H | N(Allyl)$_2$ | 99.0 | 49.9 |
| 44 | H | Br | Ph | H | N(n-Pr)$_2$ | 99.8 | 61.9 |
| 47 | H | H | Ph | H | N(n-Pr)$_2$ | 88.1 | 2.3 |
| 80 | H | Me | 4-MeOPh | H | NH(4-MeOPh) | 78.5 | 28.5 |
| 97 | H | Me | 4-MeOPh | H | N(Allyl)$_2$ | 90.7 | 4.9 |
| 129 | H | Br | Ph | H | N(Allyl)$_2$ | 99.7 | 85.2 |
| zolpidem | | | | | | 99.8 | 51.9 |

Table 2. Affinity for the $\alpha_2$-subunit of the $GABA_A$ receptor

| 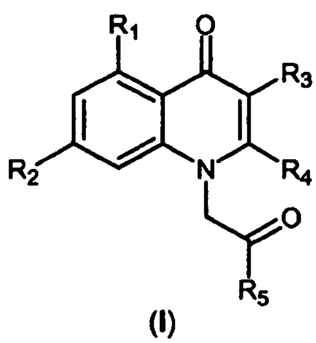 (I) | | | | | | % inhibition | |
|---|---|---|---|---|---|---|---|
| Example | $R_1$ | $R_2$ | $R_3$ | $R_4$ | $R_5$ | $10^{-5}$M | $10^{-7}$M |
| 14 | H | Me | Ph | H | N(n-Pr)$_2$ | 77.3 | 40.6 |
| 15 | H | Me | Ph | H | N-Piperidinyl | 13.3 | 0.0 |
| 17 | H | Me | Ph | H | NEt$_2$ | 70.1 | 0.0 |
| 18 | H | Me | Ph | H | N-Pyrrolidinyl | 66.3 | 0.0 |
| 27 | H | Me | Ph | H | 2-Thienyl | 89.9 | 8.4 |
| 28 | H | Me | Ph | H | 2-Pyridyl | 88.0 | 0.0 |
| 32 | H | Me | Ph | H | 2,6-Dimethyl-N-Morpholinyl | 55.4 | 0.0 |

(continued)

| Example | $R_1$ | $R_2$ | $R_3$ | $R_4$ | $R_5$ | $10^{-5}$M | $10^{-7}$M |
|---|---|---|---|---|---|---|---|
| 39 | H | Me | Ph | H | N(Cyclopropylmethyl)(Pr) | 93.4 | 28.5 |
| 42 | H | Me | Ph | H | 2,5-Dimethyl-3-Pyrrolinyl | 74.1 | 0.0 |
| 43 | H | Me | Ph | H | $N(Allyl)_2$ | 88.2 | 12.5 |
| 47 | H | H | Ph | H | $N(n-Pr)_2$ | 68.9 | 0.0 |
| 80 | H | Me | 4-MeOPh | H | NH(4-MeOPh) | 65.1 | 15.7 |
| 97 | H | Me | 4-MeOPh | H | $N(Allyl)_2$ | 62.0 | 0.0 |
| zolpidem | | | | | | 74.1 | 19.9 |

b) *In vivo* determination of predictive sedative-hypnotic action

The *in vivo* effects of these compounds were assessed by a predictive sedation-hypnosis test in mice (D. J. Sanger et al., Eur. J. Pharmacol., 313, 35-42, 1996; and G. Griebel et al., Psychopharmacology, 146, 205-213, 1999).

Groups of 5-8 male CD1 mice, weighing 22-26 g at the time of test, were used. The test compounds were administered in single equimolecular intraperitoneal doses, suspended in 0.25% agar with one drop of Tween in a volume of 10 mL/kg. Control animals received the vehicle alone. Using a Smart System (Panlab, S.L., Spain) the traveled distance in cm was recorded for each mouse at 5 min intervals during a period of 30 minutes after dosing. The inhibition percentage traveled distance of treated animals versus control animals (the first 5 min were discarded) was calculated. The results of this test are given in **Table 3.**

Table 3. Determination of *in vivo* sedative-hypnotic activity in mice.

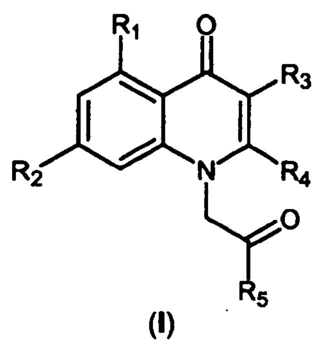

**(I)**

| Example | $R_1$ | $R_2$ | $R_3$ | $R_4$ | $R_5$ | %(*) |
|---|---|---|---|---|---|---|
| 14 | H | Me | Ph | H | $N(n-Pr)_2$ | 96.22 |
| 15 | H | Me | Ph | H | N-Piperidinyl | 84.65 |
| 17 | H | Me | Ph | H | $NEt_2$ | 81.47 |
| 18 | H | Me | Ph | H | N-Pyrrolidinyl | 84.55 |
| 27 | H | Me | Ph | H | 2-Thienyl | 70.0 |
| 28 | H | Me | Ph | H | 2-Pyridyl | 84.41 |
| 32 | H | Me | Ph | H | 2,6-Dimethyl-N-Morpholinyl | 89.96 |
| 33 | H | Me | Ph | H | 2,4-Dimethyl-5-Oxazolyl | 91.67 |
| 36 | H | Me | 4-MeOPh | H | $N(n-Pr)_2$ | 92.06 |
| 39 | H | Me | Ph | H | N(Cyclopropylmethyl)(Pr) | 92.46 |
| 42 | H | Me | Ph | H | 2,5-Dimethyl-3-Pyrrolinyl | 92.19 |

(continued)

| Example | $R_1$ | $R_2$ | $R_3$ | $R_4$ | $R_5$ | %(*) |
|---|---|---|---|---|---|---|
| 43 | H | Me | Ph | H | $N(Allyl)_2$ | 90.99 |
| 44 | H | Br | Ph | H | $N(n\text{-}Pr)_2$ | 95.25 |
| 47 | H | H | Ph | H | $N(n\text{-}Pr)_2$ | 74.72 |
| 80 | H | Me | 4-MeOPh | H | NH(4-MeOPh) | 71.91 |
| 97 | H | Me | 4-MeOPh | H | $N(Allyl)_2$ | 76.76 |
| 129 | H | Br | Ph | H | $N(Allyl)_2$ | 93.52 |
| zolpidem | | | | | | 91.70 |
| (*) % of inhibition spontaneous motor activity (98 μmol/kg) | | | | | | |

**Preparative examples**

Step 1: General method for the Claisen condensation

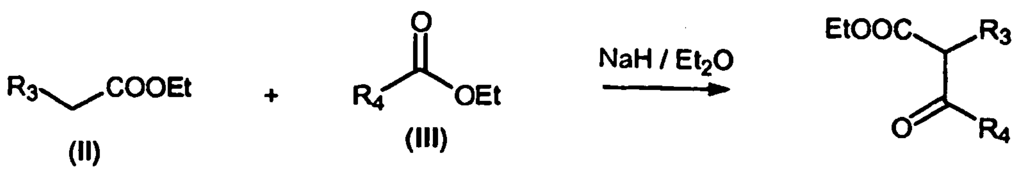


(II) (III) (V)

To a solution of acetate (**II**) (1eq) and ester (**III**) (1eq) in ethyl ether 95% sodium hydride (4eq) was slowly added over a 2 h interval under nitrogen atmosphere. After addition was complete the solution was stirred for 2.5 h. The mixture was treated with 4.5% HCl and the organic phase was extracted with diethyl ether and dried ($MgSO_4$). Evaporation of the solvent gave the title compound (**V**) in the enolic form, which was used for the next step without further purification.

*Example given for $R_1$=H, $R_2$=Me, $R_3$=Ph, $R_4$=H*

$^1H$ NMR ($CDCl_3$) δ 12.13 (d, J=12.6 Hz, 1H, OH), 7.31-7.25 (m, 6 H, 5 Har + C=CH), 4.29 (q, J=7.2 Hz, 2 H, $CH_2$-O), 1.29 (t, J=7.2 Hz, 3 H, $CH_3$).

Step 2: General method for the formation of the enamine

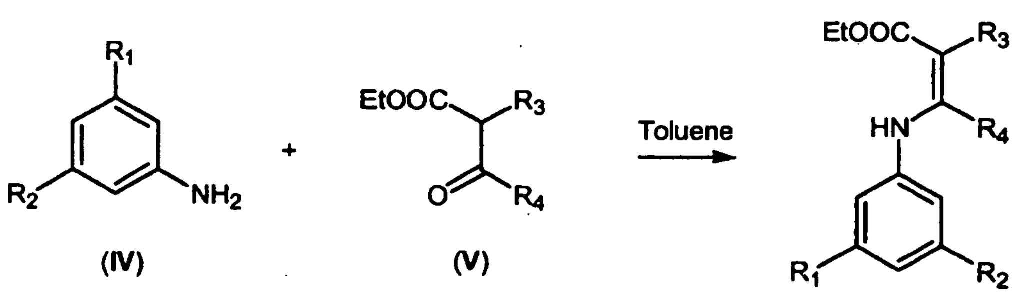


(IV) (V) (VI)

A solution of ketoester (**V**) (1.1eq) and aniline (**IV**) (1 eq) in toluene was refluxed under stirring for 19 hours in

a Dean-Stark apparatus. After cooling toluene (18 mL) was added and the mixture was treated with 10% HCl and water. The organic phase was dried with $MgSO_4$ and the residue obtained after evaporation was purified by silica gel column chromatography (hexane/ethyl acetate), to give the title compound (**VI**).

*Example given for $R_1$=H, $R_2$=Me, $R_3$=Ph, $R_4$=H*
$^1$H NMR ($CDCl_3$) δ 10.30 (d, J=12.6 Hz, 1H, NH), 7.43-6.83 (m, 10 H, 9 Har + C=CH), 4.26 (q, J=7.2 Hz, 2 H, $CH_2$-O), 2.33 (s, 3H, $CH_3$-Ph), 1.30 (t, J=7.2 Hz, 3 H, $CH_3$).

Step 3: General method for the Friedel-Crafts cyclisation

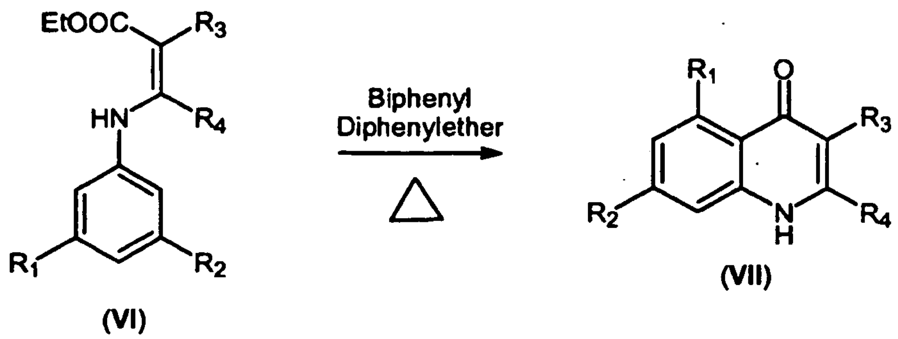


(VI) (VII)

To a solution of biphenyl and diphenyl ether (1:7 molar ratio) heated at 280 °C , enamine (**VI**) was slowly added and the reaction mixture was stirred at 280 °C for 20 min. The mixture was cooled with an ice bath and the precipitate was filtered and washed with hexane to give a mixture of the two possible isomers. These were separated by column chromatography (hexane/ethyl acetate) to give the desired product (**VII**).

*Example given for $R_1$=H, $R_2$=Me, $R_3$=Ph, $R_4$=H*
$^1$H NMR (DMSO-$d_6$) δ 11.9 (br, 1H, NH), 8.09-7.14 (m, 8 H, Har), 2.44 (s, 3 H, $CH_3$).

Step 4: General method for the N-alkylation (by using (VIII) or (XII))

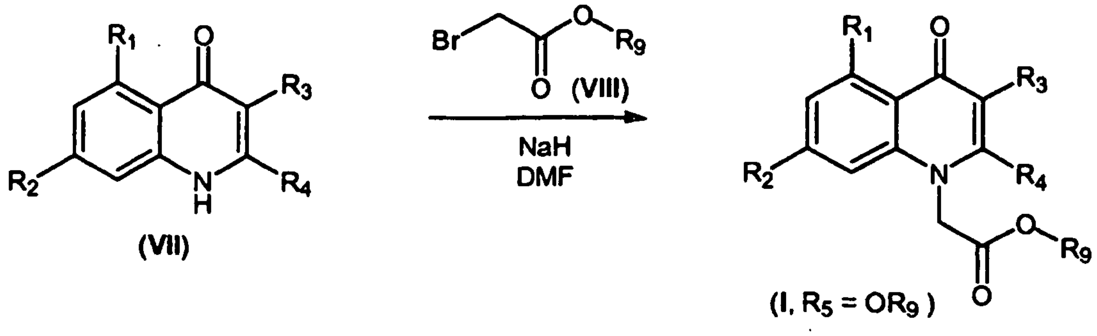


(VII) (VIII) (I, $R_5$ = $OR_9$ )

To a suspension of quinolone (**VII**) (1eq) in dry dimethylformamide (DMF) under nitrogen atmosphere, 95% sodium hydride (1.6eq) was slowly added. After addition was complete, a solution of methyl bromoacetate (**VIII**, $R_9$ = Me) (2eq) in dry DMF was added and the mixture was heated to 95 °C for 3.5 hours. After cooling, the mixture was treated with brine and extracted with ethyl acetate. The organic phase was dried ($MgSO_4$) and the solvent was evaporated. The residue obtained was purified by silica gel column chromatography (hexane/ethyl acetate) to give the title compound (I, $R_5$ = OMe).

*Example given for $R_1$=H, $R_2$=Me, $R_3$=Ph, $R_4$=H*
$^1$H NMR ($CDCl_3$) δ 8.38 (d, J=8.1 Hz, 1H, Har), 7.63-7.60 (m, 2 H, Har), 7.47 (s, 1H, CH=C), 7.36-7.12 (m, 5 H, Har), 6.75 (s, 1H, Har), 4.77 (s, 2H, $CH_2$-C=O), 4.10. (s, 3H, OMe), 2.41 (s, 3 H, $CH_3$-Ph).

To substitute the ester group by a hydrazine residue, to a solution of 1eq of quinolone-ester in methanol a solution of 5eq of the corresponding hydrazine (**X**) in methanol was added and the mixture was heated at reflux for 24h. The reaction was allowed to cool and the solvent was removed. Then, acetone was added to the mixture and the solid obtained was filtered off, washed with acetone and dried, to give the title compound (I, $R_5$=N($R_8$)$NH_2$) with an average yield of 90%.

Step 5: General method to proceed with the saponification

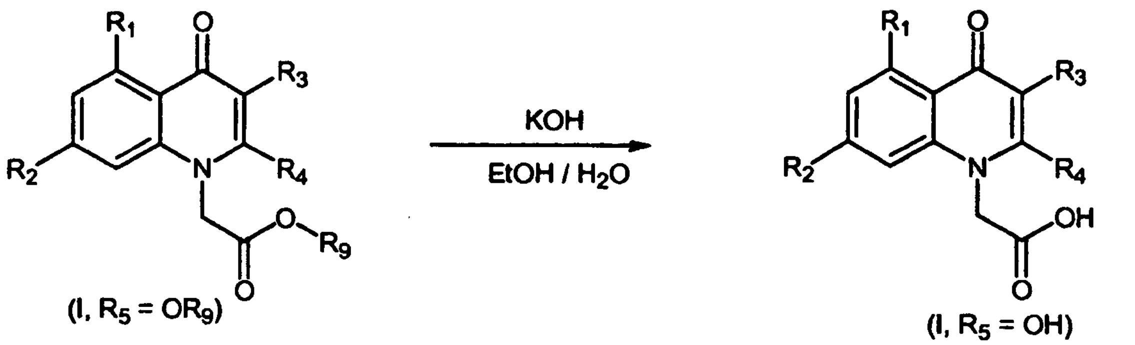


(I, $R_5$ = $OR_9$) (I, $R_5$ = OH)

To a solution of quinolone-ester (**I**, $R_5$ = $OR_9$) (1eq) in ethanol an aqueous solution of KOH (3eq) was added. The mixture was heated at reflux for 6h. After cooling, a solution of HCl 6N was added until pH=1. The solid thus obtained was filtered off, washed with cold water and dried, to give the title compound (**I**, $R_5$ = OH).

Step 6: General method for amide formation

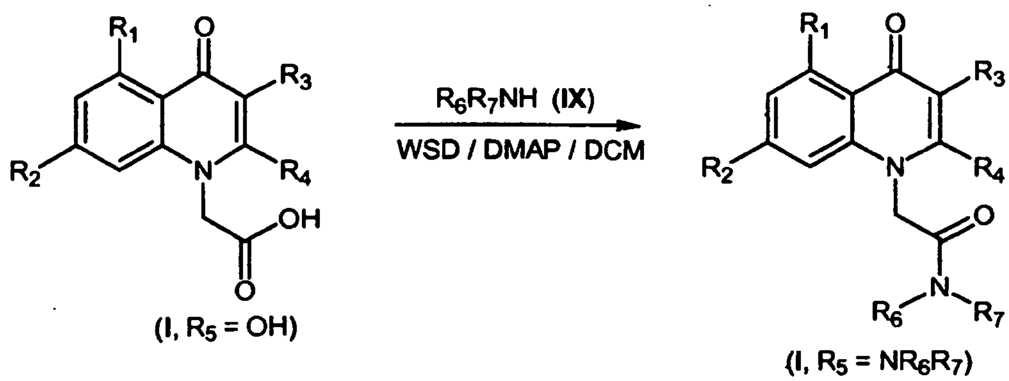


(I, $R_5$ = OH) (I, $R_5$ = $NR_6R_7$)

To a solution of 1eq of the quinolone-acid in dichloromethane (DCM) a solution of 1.5eq of water-soluble carbodiimide (WSC) in DCM was added. The mixture was stirred for 30 min at room temperature. Then, a solution of 1.5eq of the corresponding amine and 0.5eq of 4-(N,N-dimethylamino)-pyridine (DMAP) in DCM was added and the resulting mixture was stirred for 24h at room temperature. The crude was extracted with HCl 1N, and the organic layer was dried and the solvent was removed. The residue was purified by LCMS. The average yield of the process was 60%.

*Example given for $R_1$=H, $R_2$=Me, $R_3$=Ph, $R_4$=H, $R_6$=$R_7$=propyl*

$^{1}$H NMR ($CDCl_3$) δ 8.38 (d, J=8.1 Hz, 1H, Har), 7.63-7.60 (m, 2 H, Har), 7.47 (s, 1H, CH=C), 7.36-7.12 (m, 4 H, Har), 6.75 (s, 1H, Har), 4.77 (s, 2H, $CH_2$-C=O), 3.34-3.29 (m, 4 H, 2 $CH_2$-N), 2.41 (s, 3 H, $CH_3$-Ph), 1.78-1.51 (m, 4 H, 2 $CH_2$-$CH_3$), 1.03 (t, J=7.2 Hz, 3 H, $CH_3$), 0.87 (t, J=7.2 Hz, 3 H, $CH_3$);

$^{13}$C NMR δ 175.7 (C=O), 164.9 (N-C=O), 142.6, 142.4, 139.8, 135.3, 128.5, 128.0, 127.3, 126.7, 125.1, 125.0, 121.7,

114.4 (Car + 2 C=C), 53.7 ($CH_2$-C=O), 48.9, 48.1 (2 $CH_2$-N), 22.3, 22.2 (2 $CH_2$-$CH_3$), 20.8 ($CH_3$-Ph), 11.4, 11.4 (2 $CH_3$).

Compounds 1-146, Table 4, were prepared according to the above reported preparative examples.

Table 4

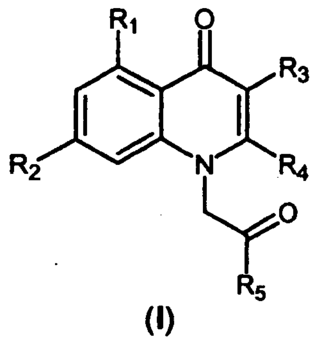

(I)

| Number | $R_1$ | $R_2$ | $R_3$ | $R_4$ | $R_5$ | Purity (%) | LCMS (M+1) |
|---|---|---|---|---|---|---|---|
| 1 | Me | H | H | Ph | N-Morpholinyl | 98 | 363.4 |
| 2 | H | Me | H | Ph | $NEt_2$ | 95 | 349.4 |
| 3 | H | Me | H | Ph | N-Morpholinyl | 97 | 363.4 |
| 4 | H | Me | H | Ph | OEt | 97 | 322.4 |
| 5 | H | Me | H | Ph | $N(n\text{-}Bu)_2$ | 97 | 405.6 |
| 6 | Me | H | H | Ph | $N(iPr)_2$ | 99 | 377.5 |
| 7 | Me | H | H | Ph | N-Pyrrolidinyl | 97 | 347.4 |
| 8 | H | Me | H | Ph | $N(n\text{-}Pr)_2$ | 96 | 377.5 |
| 9 | Me | H | H | Ph | $N(n\text{-}Hex)_2$ | 98 | 461.7 |
| 10 | Me | H | H | Ph | N-Piperidinyl | 97 | 361.5 |
| 11 | Me | H | H | Ph | N-Pyrrolidinyl | 98 | 347.4 |
| 12 | H | Me | H | Ph | N-Pyrrolidinyl | 96 | 347.4 |
| 13 | H | Me | Ph | H | $N(n\text{-}Bu)_2$ | 95 | 405.6 |
| 14 | H | Me | Ph | H | $N(n\text{-}Pr)_2$ | 99 | 377.5 |
| 15 | H | Me | Ph | H | N-Piperidinyl | 99 | 361.5 |
| 16 | H | Me | Ph | H | $N(n\text{-}Hex)_2$ | 98 | 461.7 |
| 17 | H | Me | Ph | H | $NEt_2$ | 97 | 349.4 |
| 18 | H | Me | Ph | H | N-Pyrrolidinyl | 96 | 347.4 |
| 19 | H | Me | Ph | H | N-Morpholinyl | 100 | 363.4 |
| 20 | H | Me | Ph | H | $N(iPr)_2$ | 97 | 377.5 |
| 21 | Me | H | Ph | H | $N(n\text{-}Hex)_2$ | 98 | 461.7 |
| 22 | Me | H | Ph | H | N-Piperidinyl | 97 | 361.5 |
| 23 | Me | H | Ph | H | N-Morpholinyl | 98 | 363.4 |
| 24 | Me | H | Ph | H | $NEt_2$ | 97 | 349.4 |
| 25 | Me | H | Ph | H | $N(n\text{-}Bu)_2$ | 96 | 405.6 |
| 26 | H | Me | Ph | H | 3-Pyridyl | 100 | 355.4 |
| 27 | H | Me | Ph | H | 2-Thienyl | 98 | 360.4 |

(continued)

| Number | $R_1$ | $R_2$ | $R_3$ | $R_4$ | $R_5$ | Purity (%) | LCMS (M+1) |
|---|---|---|---|---|---|---|---|
| 28 | H | Me | Ph | H | 2-Pyridyl | 100 | 355.4 |
| 29 | H | Me | Ph | H | 4-Pyridyl | 97 | 355.4 |
| 30 | H | Me | Ph | H | N'-Acetyl-N-Piperazinyl | 99 | 404.5 |
| 31 | H | Me | Ph | H | 5-Methyl-2-Furyl | 97 | 358.4 |
| 32 | H | Me | Ph | H | 2,6-Dimethyl-N-Morpholinyl | 97 | 391.5 |
| 33 | H | Me | Ph | H | 2,4-Dimethyl-5-Oxazolyl | 95 | 373.4 |
| 34 | H | Me | Ph | H | 2-Pyrazinyl | 99 | 356.4 |
| 35 | H | Me | Ph | H | N'-Methyl-N-Piperazinyl | 96 | 376.5 |
| 36 | H | Me | 4-MeOPh | H | $N(n-Pr)_2$ | 97 | 407.5 |
| 37 | H | Me | 4-ClPh | H | $N(n-Pr)_2$ | 97 | 411.9 |
| 38 | H | Me | *p*-Tolyl | H | $N(n-Pr)_2$ | 96 | 391.5 |
| 39 | H | Me | Ph | H | N(Cyclopropylmethyl)(n-Pr) | 99 | 389.5 |
| 40 | H | Me | Ph | H | 3,5-Dimethyl-N-Piperidinyl | 98 | 389.5 |
| 41 | H | Me | Ph | H | 1,2,3,6-Tetrahydropyridin-1-yl | 98 | 359.4 |
| 42 | H | Me | Ph | H | 2,5-Dimethyl-3-Pyrrolin-1-yl | 97 | 373.5 |
| 43 | H | Me | Ph | H | $N(Allyl)_2$ | 96 | 373.5 |
| 44 | H | Br | Ph | H | $N(n-Pr)_2$ | 95 | 442.4 |
| 45 | Br | H | Ph | H | $N(n-Pr)_2$ | 96 | 442.4 |
| 46 | H | Ph | Ph | H | $N(n-Pr)_2$ | 97 | 439.6 |
| 47 | H | H | Ph | H | $N(n-Pr)_2$ | 95 | 363.5 |
| 48 | Ph | H | Ph | H | $N(n-Pr)_2$ | 97 | 439.6 |
| 49 | H | Me | *p*-Tolyl | H | OMe | 100 | 322.4 |
| 50 | H | Me | *p*-Tolyl | H | OH | 97 | 308.3 |
| 51 | H | Me | 4-ClPh | H | OMe | 98 | 342.8 |
| 52 | H | Me | 4-FPh | H | OMe | 97 | 326.3 |
| 53 | H | Me | 4-FPh | H | OH | 99 | 312.3 |
| 54 | H | Me | 4-ClPh | H | NHPh | 97 | 403.9 |
| 55 | H | Me | 4-ClPh | H | NH(2-Pyrazinyl) | 100 | 405.9 |
| 56 | H | Me | 4-ClPh | H | OH | 99 | 328.8 |
| 57 | H | Me | 4-ClPh | H | $NHNH_2$ | 96 | 342.8 |
| 58 | H | Me | *p*-Tolyl | H | NHPh | 99 | 383.5 |
| 59 | H | Me | 4-FPh | H | NHPh | 98 | 387.4 |
| 60 | H | Me | *p*-Tolyl | H | NH(2-Thiazolyl) | 99 | 390.5 |
| 61 | H | Me | ρ-Tolyl | H | NH(5-Methyl-Isoxazol-3-yl) | 96 | 388.4 |
| 62 | H | Me | 4-FPh | H | $NHNH_2$ | 96 | 326.3 |
| 63 | H | Me | *p*-Tolyl | H | $NHNH_2$ | 98 | 322.4 |
| 64 | H | Me | 4-ClPh | H | NH(5-Methyl-Isoxazol-3-yl) | 96 | 408.9 |
| 65 | H | Me | 4-FPh | H | NH(5-Methyl-Isoxazol-3-yl) | 96 | 392.4 |

(continued)

| Number | $R_1$ | $R_2$ | $R_3$ | $R_4$ | $R_5$ | Purity (%) | LCMS (M+1) |
|---|---|---|---|---|---|---|---|
| 66 | H | Me | Ph | H | N(Hydroxyethyl)(Et) | 100 | 365.4 |
| 67 | H | Me | Ph | H | N(Hydroxyethyl)(n-Pr) | 98 | 379.5 |
| 68 | Me | H | Ph | H | $N(Allyl)_2$ | 100 | 373.5 |
| 69 | Me | H | Ph | H | N(Hydroxyethyl)(n-Pr) | 98 | 379.5 |
| 70 | H | Me | Ph | H | OH | 97 | 294.3 |
| 71 | Me | H | Ph | H | OH | 96 | 294.3 |
| 72 | H | Me | 4-ClPh | H | $N(Me)NH_2$ | 97 | 356.8 |
| 73 | H | Me | *p*-Tolyl | H | $N(Me)NH_2$ | 97 | 336.4 |
| 74 | H | Me | 4-FPh | H | $N(Me)NH_2$ | 97 | 340.4 |
| 75 | H | Me | 4-FPh | H | NH(2-Thiazolyl) | 97 | 394.4 |
| 76 | H | Me | 4-ClPh | H | NH(2-Thiazolyl) | 98 | 410.9 |
| 77 | H | Me | 4-MeOPh | H | $NH(4-CF_3Ph)$ | 98 | 467.5 |
| 78 | H | Me | Ph | H | $NH(4-CF_3Ph)$ | 96 | 437.4 |
| 79 | H | Me | Ph | H | NH(4-MeOPh) | 97 | 399.5 |
| 80 | H | Me | 4-MeOPh | H | NH(4-MeOPh) | 96 | 429.5 |
| 81 | H | Me | 4-MeOPh | H | NH(5-Methyl-3-Pyrazolyl) | 96 | 403.5 |
| 82 | H | Me | Ph | H | NH(2-Thiazolyl) | 96 | 376.4 |
| 83 | H | Me | 4-MeOPh | H | NH(2-Thiazolyl) | 97 | 406.5 |
| 84 | H | Me | Ph | H | NH(3-Methyl-2-Thienylmethyl) | 97 | 403.5 |
| 85 | H | Me | 4-MeOPh | H | NH(3-Methyl-5-Isoxazolyl) | 96 | 404.4 |
| 86 | H | Me | 4-MeOPh | H | NH(3-Methyl-6-Pyridyl) | 98 | 414.5 |
| 87 | H | Me | Ph | H | NH(2-Pyrimidyl) | 97 | 371.4 |
| 88 | H | Me | Ph | H | NH(2-Furylmethyl) | 97 | 373.4 |
| 89 | H | Me | 4-MeOPh | H | NH(1,3-Dimethyl-5-Pyrazolyl) | 99 | 417.5 |
| 90 | H | Me | 4-MeOPh | H | $NEt_2$ | 99 | 379.5 |
| 91 | H | Me | 4-MeOPh | H | N-Pyrrolidinyl | 96 | 377.5 |
| 92 | H | Me | 4-MeOPh | H | N-Piperidinyl | 98 | 391.5 |
| 93 | H | Me | 4-MeOPh | H | N(Hydroxyethyl)(Et) | 96 | 395.5 |
| 94 | H | Me | 4-MeOPh | H | N(Hydroxyethyl)(n-Pr) | 96 | 409.5 |
| 95 | H | Me | 4-MeOPh | H | N(Cyclopropylmethyl)(n-Pr) | 99 | 419.5 |
| 96 | H | Me | 4-MeOPh | H | $N(Hydroxyethyl)_2$ | 96 | 411.5 |
| 97 | H | Me | 4-MeOPh | H | $N(Allyl)_2$ | 98 | 403.5 |
| 98 | H | Me | Ph | H | NH(3,5-Isoxazol-4-yl) | 99 | 388.4 |
| 99 | H | Me | Ph | H | NH(1-Methyl-3-Pyrazolyl) | 100 | 373.4 |
| 100 | H | Me | Ph | H | NH(2,5-Dimethyl-3-Pyrazolyl) | 95 | 387.5 |
| 101 | H | Me | Ph | H | NH(5-Methyl-2-Pyridinyl) | 95 | 384.5 |
| 102 | H | Me | Ph | H | NH(1,3,4-Thiadiazol-2-yl) | 98 | 377.4 |
| 103 | H | Me | Ph | H | NH(4-Dimethylamino-Ph) | 95 | 412.5 |

(continued)

| Number | $R_1$ | $R_2$ | $R_3$ | $R_4$ | $R_5$ | Purity (%) | LCMS (M+1) |
|---|---|---|---|---|---|---|---|
| 104 | H | Me | Ph | H | NH(3-Methylisoxazol-5-yl) | 98 | 374.4 |
| 105 | H | Me | Ph | H | NH(*p*-Tolyl) | 96 | 383.5 |
| 106 | H | Me | 4-MeOPh | H | NH(1-Methyl-3-Pyrazolyl) | 99 | 403.5 |
| 107 | H | Me | 4-MeOPh | H | NH(2,5-Dimethyl-3-Pyrrolinyl) | 96 | 403.5 |
| 108 | H | Me | Ph | H | NH(5-Methyl-3-Pyrazolyl) | 95 | 373.4 |
| 109 | H | Me | Ph | H | NH(2-Pyridylmethyl) | 98 | 384.5 |
| 110 | H | Me | Ph | H | NH(2-Acetyl-3-Thienyl) | 95 | 417.5 |
| 111 | H | Me | Ph | H | N(Et)(1,3,4-Thiadiazol-2-yl) | 99 | 405.5 |
| 112 | H | Me | Ph | H | NH(2-Methyl-5-Indolyl) | 98 | 422.5 |
| 113 | H | Me | Ph | H | 1,2,3,6-Tetrahydro-N-pyridyl | 96 | 359.4 |
| 114 | H | Me | Ph | H | NH(3-Pyrazolyl) | 98 | 359.4 |
| 115 | H | Me | Ph | H | NH(4-FPh) | 97 | 387.4 |
| 116 | H | Me | Ph | H | NH[5-(CF3)-1,3,4-Thiadiazol-2-yl] | 98 | 445.4 |
| 117 | H | Me | Ph | H | NH(2-Quinolyl) | 99 | 420.5 |
| 118 | H | Me | Ph | H | NH(2-Methyl-3-Pyrazolyl) | 98 | 373.4 |
| 119 | H | Me | Ph | H | NH(5-Cyclopropyl-3-Pyrazolyl) | 98 | 399.5 |
| 120 | H | Me | Ph | H | NH(2-Quinolyl) | 97 | 420.5 |
| 121 | H | Me | Ph | H | NH(2-Indanyl) | 96 | 409.5 |
| 122 | H | Me | Ph | H | NH(2-Thienylethyl) | 99 | 403.5 |
| 123 | H | Br | Ph | H | 1,2,3,6-Tetrahydropyridyl | 97 | 424.3 |
| 124 | H | Br | Ph | H | $NHNH_2$ | 98 | 373.2 |
| 125 | H | Br | Ph | H | $N(n\text{-}Bu)_2$ | 97 | 470.4 |
| 126 | H | Br | Ph | H | N-Pyrrolidinyl | 99 | 412.3 |
| 127 | H | Br | Ph | H | N-Piperidinyl | 96 | 426.3 |
| 128 | H | Br | Ph | H | 2,6-Dimethyl-N-Morpholinyl | 97 | 456.4 |
| 129 | H | Br | Ph | H | $N(Allyl)_2$ | 99 | 438.3 |
| 130 | H | Br | Ph | H | 2,5-Dimethyl-3-Pyrrolinyl | 97 | 438.3 |
| 131 | H | Br | Ph | H | N(Cyclopropylmethyl)(n-Pr) | 96 | 454.4 |
| 132 | H | Me | Ph | H | NH(5-Nitro-2-Thiazol-2-yl) | 99 | 421.4 |
| 133 | H | Me | Ph | H | NH(3-Oxazolyl) | 98 | 360.4 |
| 134 | H | Me | Ph | H | NH(4-Methyl-2-Thiazolyl) | 99 | 390.5 |
| 135 | H | Br | Ph | H | N-Piperidinyl | 96 | 426.3 |
| 136 | H | Me | Ph | H | OMe | 97 | 308.3 |
| 137 | H | Me | Ph | H | OH | 98 | 294.3 |
| 138 | H | Me | 4-MeOPh | H | OMe | 96 | 338.4 |
| 139 | H | Me | 4-MeOPh | H | OH | 98 | 324.3 |
| 140 | H | Br | Ph | H | OH | 99 | 359.2 |
| 141 | H | Me | 4-MeOPh | H | 2,6-Dimethyl-N-Morpholinyl | 97 | 421.5 |

(continued)

| Number | $R_1$ | $R_2$ | $R_3$ | $R_4$ | $R_5$ | Purity (%) | LCMS (M+1) |
|---|---|---|---|---|---|---|---|
| 142 | H | Me | 4-MeOPh | H | 3,5-Dimethyl-N-Piperidinyl | 100 | 419.5 |
| 143 | H | Me | Ph | H | NH(6-Methoxy-4-Pyrimidinyl) | 96 | 401.4 |
| 144 | H | Me | Ph | H | 4-Hydroxy-N-Piperidinyl | 98 | 377.5 |
| 145 | H | Me | 4-MeOPh | H | NH(3-Isoxazolyl) | 96 | 390.4 |
| 146 | H | Me | 4-MeOPh | H | 4-Hydroxy-N-Piperidinyl | 99 | 407.5 |

**Composition example 1**: 5 mg tablets

| Active ingredient | 5.0 mg |
|---|---|
| Colloidal silicon dioxide | 0.6 mg |
| Croscarmellose sodium | 12.0 mg |
| Talc | 4.0 mg |
| Magnesium stearate | 1.5 mg |
| Polysorbate 80 | 1.0 mg |
| Lactose | 75.0 mg |
| Hydroxypropyl methylcellulose | 3.0 mg |
| Polyethylene glycol 4000 | 0.5 mg |
| Titanium dioxide E171 | 1.5 mg |
| Microcrystalline cellulose q.s. to | 125.0 mg |

**Composition example 2**: 10 mg capsules

| Active ingredient | 10.0 mg |
|---|---|
| Colloidal silicon dioxide | 0.6 mg |
| Crospovidone | 12.0 mg |
| Talc | 4.0 mg |
| Magnesium stearate | 1.5 mg |
| Lauryl sulfate sodium | 1.5 mg |
| Lactose | 77.0 mg |
| Gelatin | 28.5 mg |
| Titanium dioxide E171 | 1.5 mg |
| Indigotin E132 | 0.02 mg |
| Microcrystalline cellulose q.s. to | 155.0 mg |

**Composition example 3:** oral drops

| Active ingredient | 0.5 g |
|---|---|
| Propylene glycol | 10.0 g |
| Glycerin | 5.0 g |
| Saccharin sodium | 0.1 g |
| Polysorbate 80 | 1.0 g |

(continued)

| Active ingredient | 0.5 g |
| --- | --- |
| Lemon flavor | 0.2 g |
| Ethanol | 25.0 mL |
| Purified water q.s. to | 100.0 mL |

**Composition example 4:** 2.5 mg tablets

| Active ingredient | 2.5 mg |
| --- | --- |
| Colloidal silicon dioxide | 0.6 mg |
| Croscaramellose sodium | 12.0 mg |
| Talc | 4.0 mg |
| Magnesium stearate | 1.5 mg |
| Polysorbate 80 | 1.0 mg |
| Lactose | 75.0 mg |
| Hydroxypropyl methylcellulose | 3.0 mg |
| Polyethylene glycol 4000 | 0.5 mg |
| Titanium dioxide E171 | 1.5 mg |
| Microcrystalline cellulose q.s. to | 125.0 mg |

**Composition example 5:** 5 mg capsules

| Active ingredient | 5.0 mg |
| --- | --- |
| Colloidal silicon dioxide | 0.6 mg |
| Crospovidone | 12.0 mg |
| Talc | 4.0 mg |
| Magnesium stearate | 1.5 mg |
| Lauryl sulfate sodium | 1.5 mg |
| Lactose | 77.0 mg |
| Gelatin | 28.5 mg |
| Titanium dioxide E171 | 1.5 mg |
| Indigotin E132 | 0.02 mg |
| Microcrystalline q.s.to | 155.0 mg |

**Composition example 6**: Oral drops

| Active ingredient | 0.25 g |
| --- | --- |
| Propylene glycol | 10.0 g |
| Glycerin | 5.0 g |
| Saccharin sodium | 0.1 g |
| Polysorbate 80 | 1.0 g |
| Lemon flavor | 0.2 g |
| Ethanol | 25.0 mL |
| Purified q.s. to | 100.0 mL |

Abbreviations used throughout the specification:
Me = methyl; Et = ethyl; nPr = n-propyl; iPr = I-propyl; n-Bu = n-butyl ; n-Hex = n-hexyl ; Ph = phenyl

## Claims

1. A 1H-quinolin-one compound of formula (**I**):

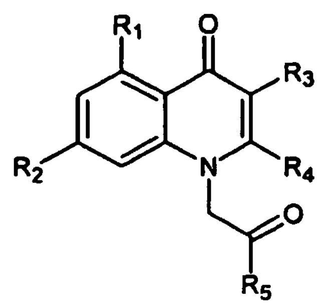

**(I)**

wherein
$R_1$ and $R_2$ are independently selected from the group consisting of hydrogen, alkyl($C_1$-$C_6$), Oalkyl($C_1$-$C_6$), halogen and phenyl;
$R_3$ and $R_4$ are independently selected from the group consisting of hydrogen and phenyl optionally substituted by alkyl($C_1$-$C_6$), halogen and Oalkyl($C_1$-$C_6$);
$R_5$ is selected from the group consisting of $NR_6R_7$, $N(R_8)NH_2$ and a heteroaryl ring selected from the group consisting of pyridyl, furyl, thienyl, oxazolyl, isoxazolyl, imidazolyl, pyrrolyl, pyrazolyl, pyrimidinyl and pyrazinyl, wherein each heteroaryl ring contains one or two optional alkyl($C_1$-$C_6$) substituents;
$R_6$ and $R_7$ are independently selected from the group consisting of hydrogen; alkyl($C_1$-$C_6$); alkenyl($C_2$-$C_6$); cycloalkyl ($C_3$-$C_6$)alkyl($C_1$-$C_6$); hydroxyalkyl($C_1$-$C_6$); heteroaryl selected from the group consisting of pyridyl, pyrimidinyl, pyrazinyl, thiazolyl, benzothiazolyl, oxazolyl, benzoxazolyl, isoxazolyl, benzisoxazolyl, pyrazolyl, furyl, benzofuryl, thienyl, benzothienyl, thiadiazolyl, pyrrolyl, imidazolyl, benzimidazolyl, indolyl, quinolyl and isoquinolyl, wherein each heteroaryl contains one or
two optional substituents independently selected from the group consisting of alkyl($C_1$-$C_6$), Oalkyl($C_1$-$C_6$), haloalkyl ($C_1$-$C_6$), cycloalkyl($C_3$-$C_6$), $NO_2$ and COalkyl($C_1$-$C_6$); thienylalkyl($C_1$-$C_6$), furylalkyl($C_1$-$C_6$), pyridylalkyl($C_1$-$C_6$), 3-methyl-2-thienylmethyl,; and aryl selected from the group consisting of phenyl and indanyl, wherein each aryl contains one or two optional substituents selected from the group consisting of alkyl($C_1$-$C_6$), haloalkyl($C_1$-$C_6$), halogen, Ndialkyl($C_1$-$C_6$), NHalkyl($C_1$-$C_6$), Oalkyl($C_1$-$C_6$), $NO_2$, CN, OH, $NH_2$, COOH, COalkyl($C_1$-$C_6$), COOalkyl($C_1$-$C_6$), CONHalkyl($C_1$-$C_6$), CONdialkyl($C_1$-$C_6$) and COphenyl; with the proviso that $NR_6R_7$ is not $NH_2$, NH($C_1$-$C_4$ alkyl) or N($C_1$-$C_4$ alkyl)$_2$;
or $R_6$ and $R_7$ can form, together with the nitrogen atom to which they are attached, a heterocycle selected from pyrrolidine, 3-pyrroline, 1,2,3,6-tetrahydropyridine, piperidine, morpholine, thiomorpholine and piperazine, wherein each heterocycle contains one, two, three or four optional substituents selected from the group consisting of OH, alkyl($C_1$-$C_6$) and COalkyl($C_1$-$C_6$);
$R_8$ is selected from the group consisting of hydrogen and alkyl($C_1$-$C_6$);
with the proviso that $R_3$ and $R_4$ cannot be hydrogen simultaneously;
and pharmaceutically acceptable salts and hydrates thereof.

2. The compound of claim 1 wherein $R^1$ is selected from the group consisting of hydrogen, alkyl($C_1$-$C_6$), Oalkyl($C_1$-$C_6$), halogen and phenyl; $R^2$ is selected from the group consisting of hydrogen, alkyl($C_1$-$C_6$), halogen and phenyl; and $R_3$, $R_4$, $R_5$, $R_6$, $R_7$, and $R_8$ are as defined in claim 1.

3. A compound according to any one of the preceding claims, wherein $R_1$ and $R_2$ are independently selected from the group consisting of hydrogen, methyl, bromine and phenyl.

**4.** A compound according to any one of the preceding claims, wherein $R_3$ and $R_4$ are independently selected from the group consisting of hydrogen, phenyl, 4-fluorophenyl, 4-chlorophenyl, p-tolyl and 4-methoxyphenyl.

**5.** A compound according to one of the preceding claims, wherein $R_5$ is selected from the group consisting of $NHNH_2$, N(methyl)$NH_2$, 2-pyridyl, 3-pyridyl, 4-pyridyl, 2-pyrazinyl, 5-methyl-2-furyl, 2-thienyl and 2,4-dimethyl-5-oxazolyl.

**6.** A compound according to one of the preceding claims, wherein $R_6$ and $R_7$ are independently selected from the group consisting of hexyl, allyl, hydroxyethyl, cyclopropylmethyl, 1,3,4-thiadiazol-2-yl, 1,3-dimethyl-5-pyrazolyl, 1-methyl-3-pyrazolyl, 2,5-dimethyl-3-pyrrolinyl, 1,2,3,6-tetrahydropiperidinyl, 2,5-dimethyl-3-pyrazolyl, 2-acetyl-3-thienyl, 2-indanyl, 2-methyl-3-pyrazolyl, 2-methyl-5-indolyl, 2-pyrazinyl, 2-pyrimidinyl, 2-quinolyl, 2-thiazolyl, 3,5-isoxazol-4-yl, 3-isoxazolyl, 3-methyl-2-thienylmethyl, 3-methyl-5-isoxazolyl, 3-methyl-6-pyridyl, 3-methylisoxazol-5-yl, 3-oxazolyl, 3-pyrazolyl, 1,3,4-thiadiazol-2-yl, 4-methyl-2-thiazolyl, 5-trifluoromethyl-1,3,4-thiadiazol-2-yl, 5-cyclopropyl-3-pyrazolyl, 5-methyl-2-pyridyl, 5-methyl-3-pyrazolyl, 5-methyl-isoxazol-3-yl, 5-nitro-2-thiazol-2-yl, 6-methoxy-4-pyrimidinyl, 2-furylmethyl, 2-pyridylmethyl, 2-thienylethyl, phenyl, p-tolyl, 4-trifluoromethylphenyl, 4-dimethylaminophenyl, 4-fluorophenyl and 4-methoxyphenyl; or $R_6$ and $R_7$ form, together with the nitrogen atom to which they are attached, pyrrole, 2,5-dimethyl-3-pyrroline, piperidine, 1,2,3,6-tetrahydropyridine, 4-hydroxypiperidine, 3,5-dimethylpiperidine, morpholine, 2,6-dimethylmorpholine, 4-methylpiperazine and 4-acetylpiperazine.

**7.** A compound according to claim 1 selected from the group consisting of
5-Methyl-1-(2-morpholin-4-yl-2-oxo-ethyl)-2-phenyl-1H-quinolin-4-one;
N,N-Diethyl-2-(7-methyl-4-oxo-2-phenyl-4H-quinolin-1-yl)-acetamide;
7-Methyl-1-(2-morpholin-4-yl-2-oxo-ethyl)-2-phenyl-1H-quinolin-4-one;
N,N-Dibutyl-2-(7-methyl-4-oxo-2-phenyl-4H-quinolin-1-yl)-acetamide;
N,N-Diisopropyl-2-(5-methyl-4-oxo-2-phenyl-4H-quinolin-1-yl)-acetamide;
5-Methyl-1-(2-oxo-2-pyrrolidin-1-yl-ethyl)-2-phenyl-1H-quinolin-4-one;
2-(7-Methyl-4-oxo-2-phenyl-4H-quinolin,1-yl)-N,N-dipropyl-acetamide;
N,N-Dihexyl-2-(5-methyl-4-oxo-2-phenyl-4H-quinolin-1-yl)-acetamide;
5-Methyl-1-(2-oxo-2-piperidin-1-yl-ethyl)-2-phenyl-1H-quinolin-4-one;
5-Methyl-1-(2-oxo-2-pyrrolidin-1-yl-ethyl)-2-phenyl-1H-quinolin-4-one;
7-Methyl-1-(2-oxo-2-pyrrolidin-1-yl-ethyl)-2-phenyl-1H-quinolin-4-one;
N,N-Dibutyl-2-(7-methyl-4-oxo-3-phenyl-4H-quinolin-1-yl)-acetamide;
2-(7-Methyl-4-oxo-3-phenyl-4H-quinolin-1-yl)-N,N-dipropyl-acetamide;
7-Methyl-1-(2-oxo-2-piperidin-1-yl-ethyl)-3-phenyl-1H-quinolin-4-one;
N,N-Dihexyl-2-(7-methyl-4-oxo-3-phenyl-4H-quinolin-1-yl)-acetamide;
N,N-Diethyl-2-(7-methyl-4-oxo-3-phenyl-4H-quinolin-1-yl)-acetamide;
7-Methyl-1-(2-oxo-2-pyrrolidin-1-yl-ethyl)-3-phenyl-1H-quinolin-4-one;
7-Methyl-1-(2-morpholin-4-yl-2-oxo-ethyl)-3-phenyl-1H-quinolin-4-one;
N,N-Diisopropyl-2-(7-methyl-4-oxo-3-phenyl-4H-quinolin-1-yl)-acetamide;
N,N-Dihexyl-2-(5-methyl-4-oxo-3-phenyl-4H-quinolin-1-yl)-acetamide;
5-Methyl-1-(2-oxo-2-piperidin-1-yl-ethyl)-3-phenyl-1H-quinolin-4-one;
5-Methyl-1-(2-morpholin-4-yl-2-oxo-ethyl)-3-phenyl-1H-quinolin-4-one;
N,N-Diethyl-2-(5-methyl-4-oxo-3-phenyl-4H-quinolin-1-yl)-acetamide;
N,N-Dibutyl-2-(5-methyl-4-oxo-3-phenyl-4H-quinolin-1-yl)-acetamide;
7-Methyl-1-(2-oxo-2-pyridin-3-yl-ethyl)-3-phenyl-1H-quinolin-4-one;
7-Methyl-1-(2-oxo-2-thiophen-2-yl-ethyl)-3-phenyl-1H-quinolin-4-one;
7-Methyl-1-(2-oxo-2-pyridin-2-yl-ethyl)-3-phenyl-1H-quinolin-4-one;
7-Methyl-1-(2-oxo-2-pyridin-4-yl-ethyl)-3-phenyl-1H-quinolin-4-one;
1-[2-(4-Acetyl-piperazin-1-yl)-2-oxo-ethyl]-7-methyl-3-phenyl-1H-quinolin-4-one;
7-Methyl-1-[2-(5-methyl-furan-2-yl)-2-oxo-ethyl]-3-phenyl-1H-quinolin-4-one;
1-[2-(2,6-Dimethyl-morpholin-4-yl)-2-oxo-ethyl]-7-methyl-3-phenyl-1H-quinolin-4-one;
1-[2-(2,4-Dimethyl-oxazol-5-yl)-2-oxo-ethyl]-7-methyl-3-phenyl-1H-quinolin-4-one;
7-Methyl-1-(2-oxo-2-pyrazin-2-yl-ethyl)-3-phenyl-1H-quinolin-4-one;
7-Methyl-1-[2-(4-methyl-piperazin-1-yl)-2-oxo-ethyl]-3-phenyl-1H-quinolin-4-one;
2-[3-(4-Methoxy-phenyl)-7-methyl-4-oxo-4H-quinolin-1-yl]-N,N-dipropyl-acetamide;
2-[3-(4-Chloro-phenyl)-7-methyl-4-oxo-4H-quinolin-1-yl]-N,N-dipropyl-acetamide;
2-(7-Methyl-4-oxo-3-p-tolyl-4H-quinolin-1-yl)-N,N-dipropyl-acetamide;
N-Cyclopropylmethyl-2-(7-methyl-4-oxo-3-phenyl-4H-quinolin-1-yl)-N-propyl-acetamide;
1-[2-(3,5-Dimethyl-piperidin-1-yl)-2-oxo-ethyl]-7-methyl-3-phenyl-1H-quinolin-4-one;

1-[2-(3,6-Dihydro-2H-pyridin-1-yl)-2-oxo-ethyl]-7-methyl-3-phenyl-1H-quinolin-4-one;
1-[2-(2,5-Dimethyl-2,5-dihydro-pyrrol-1-yl)-2-oxo-ethyl]-7-methyl-3-phenyl-1H-quinolin-4-one;
N,N-Diallyl-2-(7-methyl-4-oxo-3-phenyl-4H-quinolin-1-yl)-acetamide;
2-(7-Bromo-4-oxo-3-phenyl-4H-quinolin-1-yl)-N,N-dipropyl-acetamide;
2-(5-Bromo-4-oxo-3-phenyl-4H-quinolin-1-yl)-N,N-dipropyl-acetamide;
2-(4-Oxo-3,7-diphenyl-4H-quinolin-1-yl)-N,N-dipropyl-acetamide;
2-(4-Oxo-3-phenyl-4H-quinolin-1-yl)-N,N-dipropyl-acetamide;
2-(4-Oxo-3,5-diphenyl-4H-quinolin-1-yl)-N,N-dipropyl-acetamide;
2-[3-(4-Chloro-phenyl)-7-methyl-4-oxo-4H-quinolin-1-yl]-N-phenyl-acetamide;
2-[3-(4-Chloro-phenyl)-7-methyl-4-oxo-4H-quinolin-1-yl]-N-pyrazin-2-yl-acetamide;
[3-(4-Chloro-phenyl)-7-methyl-4-oxo-4H-quinolin-1-yl]-acetic acid hydrazide;
2-(7-Methyl-4-oxo-3-p-tolyl-4H-quinolin-1-yl)-N-phenyl-acetamide;
2-[3-(4-Fluoro-phenyl)-7-methyl-4-oxo-4H-quinolin-1-yl]-N-phenyl-acetamide;
2-(7-Methyl-4-oxo-3-p-tolyl-4H-quinolin-1-yl)-N-thiazol-2-yl-acetamide;
N-(5-Methyl-isoxazol-3-yl)-2-(7-methyl-4-oxo-3-p-tolyl-4H-quinolin-1-yl)-acetamide;
[3-(4-Fluoro-phenyl)-7-methyl-4-oxo-4H-quinolin-1-yl]-acetic acid hydrazide;
(7-Methyl-4-oxo-3-p-tolyl-4H-quinolin-1-yl)-acetic acid hydrazide;
2-[3-(4-Chloro-phenyl)-7-methyl-4-oxo-4H-quinolin-1-yl]-N-(5-methyl-isoxazol-3-yl)-acetamide;
2-[3-(4-Fluoro-phenyl)-7-methyl-4-oxo-4H-quinolin-1-yl]-N-(5-methyl-isoxazol-3-yl)-acetamide;
N-Ethyl-N-(2-hydroxy-ethyl)-2-(7-methyl-4-oxo-3-phenyl-4H-quinolin-1-yl)-acetamide;
N-(2-Hydroxy-ethyl)-2-(7-methyl-4-oxo-3-phenyl-4H-quinolin-1-yl)-N-propyl-acetamide;
N,N-Diallyl-2-(5-methyl-4-oxo-3-phenyl-4H-quinolin-1-yl)-acetamide;
N-(2-Hydroxy-ethyl)-2-(5-methyl-4-oxo-3-phenyl-4H-quinolin-1-yl)-N-propyl-acetamide;
[3-(4-Chloro-phenyl)-7-methyl-4-oxo-4H-quinolin-1-yl]-acetic acid N-methyl-hydrazide;
(7-Methyl-4-oxo-3-*p*-tolyl-4H-quinolin-1-yl)-acetic acid N-methyl-hydrazide;
[3-(4-Fluoro-phenyl)-7-methyl-4-oxo-4-H-quinolin-1-yl]-acetic acid N-methyl-hydrazide;
2-[3-(4-Fluoro-phenyl)-7-methyl-4-oxo-4H-quinolin-1-yl]-N-thiazol-2-yl-acetamide;
2-[3-(4-Chloro-phenyl)-7-methyl-4-oxo-4H-quinolin-1-yl]-N-thiazol-2-yl-acetamide;
2-[3-(4-Methoxy-phenyl)-7-methyl-4-oxo-4H-quinolin-1-yl]-N-(4-trifluoromethyl-phenyl)-acetamide;
2-(7-Methyl-4-oxo-3-phenyl-4H-quinolin-1-yl)-N-(4-trifluoromethyl-phenyl)-acetamide;
N-(4-Methoxy-phenyl)-2-(7-methyl-4-oxo-3-phenyl-4H-quinolin-1-yl)-acetamide;
N-(4-Methoxy-phenyl)-2-[3-(4-methoxy-phenyl)-7-methyl-4-oxo-4H-quinolin-1-yl]-acetamide;
2-[3-(4-Methoxy-phenyl)-7-methyl-4-oxo-4H-quinolin-1-yl]-N-(5-methyl-2H-pyrazol-3-yl)-acetamide;
2-(7-Methyl-4-oxo-3-phenyl-4H-quinolin-1-yl)-N-thiazol-2-yl-acetamide;
2-[3-(4-Methoxy-phenyl)-7-methyl-4-oxo-4H-quinolin-1-yl]-N-thiazol-2-yl-acetamide;
2-(7-Methyl-4-oxo-3-phenyl-4H-quinolin-1-yl)-N-(3-methyl-thiophen-2-ylmethyl)-acetamide;
2-[3-(4-Methoxy-phenyl)-7-methyl-4-oxo-4H-quinolin-1-yl]-N-(3-methyl-isoxazol-5-yl)-acetamide;
2-[3-(4-Methoxy-phenyl)-7-methyl-4-oxo-4H-quinolin-1-yl]-N-(5-methyl-pyridin-2-yl)-acetamide;
2-(7-Methyl-4-oxo-3-phenyl-4H-quinolin-1-yl)-N-pyrimidin-2-yl-acetamide;
N-Furan-2-ylmethyl-2-(7-methyl-4-oxo-3-phenyl-4H-quinolin-1-yl)-acetamide;
N-(2,5-Dimethyl-2H-pyrazol-3-yl)-2-[3-(4-methoxy-phenyl)-7-methyl-4-oxo-4H-quinolin-1-yl]-acetamide;
N,N-Diethyl-2-[3-(4-methoxy-phenyl)-7-methyl-4-oxo-4H-quinolin-1-yl]-acetamide;
3-(4-Methoxy-phenyl)-7-methyl-1-(2-oxo-2-pyrrolidin-1-yl-ethyl)-1H-quinolin-4-one;
3-(4-Methoxy-phenyl)-7-methyl-1-(2-oxo-2-piperidin-1-yl-ethyl)-1H-quinolin-4-one;
N-Ethyl-N-(2-hydroxy-ethyl)-2-[3-(4-methoxy-phenyl)-7-methyl-4-oxo-4H-quinolin-1-yl]-acetamide;
N-(2-Hydroxy-ethyl)-2-[3-(4-methoxy-phenyl)-7-methyl-4-oxo-4H-quinolin-1-yl]-N-propyl-acetamide;
N-Cyclopropylmethyl-2-[3-(4-methoxy-phenyl)-7-methyl-4-oxo-4H-quinolin-1-yl]-N-propyl-acetamide;
N,N-Bis-(2-hydroxy-ethyl)-2-[3-(4-methoxy-phenyl)-7-methyl-4-oxo-4H-quinolin-1-yl]-acetamide;
N,N-Diallyl-2-[3-(4-methoxy-phenyl)-7-methyl-4-oxo-4H-quinolin-1-yl]-acetamide;
N-(3,5-Dimethyl-isoxazol-4-yl)-2-(7-methyl-4-oxo-3-phenyl-4H-quinolin-I - yl)-acetamide;
2-(7-Methyl-4-oxo-3-phenyl-4H-quinolin-1-yl)-N-(1-methyl-1H-pyrazol-3-yl)-acetamide;
N-(2,5-Dimethyl-2H-pyrazol-3-yl)-2-(7-methyl-4-oxo-3-phenyl-4H-quinolin-1-yl)-acetamide;
2-(7-Methyl-4-oxo-3-phenyl-4H-quinolin-1-yl)-N-(5-methyl-pyridin-2-yl)-acetamide;
2-(7-Methyl-4-oxo-3-phenyl-4H-quinolin-1-yl)-N-[1,3,4]thiadiazol-2-yl-acetamide;
N-(4-Dimethylamino-phenyl)-2-(7-methyl-4-oxo-3-phenyl-4H-quinolin-1-yl)-acetamide;
N-(3-Methyl-isoxazol-5-yl)-2-(7-methyl-4-oxo-3-phenyl-4H-quinolin-1-yl)-acetamide;
2-(7-Methyl-4-oxo-3-phenyl-4H-quinolin-1-yl)-N-p-tolyl-acetamide;
2-[3-(4-Methoxy-phenyl)-7-methyl-4-oxo-4H-quinolin-1-yl]-N-(1-methyl-1H-pyrazol-3-yl)-acetamide;

1-[2-(2,5-Dimethyl-2,5-dihydro-pyrrol-1-yl)-2-oxo-ethyl]-3-(4-methoxy-phenyl)-7-methyl-1H-quinolin-4-one;
2-(7-Methyl-4-oxo-3-phenyl-4H-quinolin-1-yl)-N-(5-methyl-2H-pyrazol-3-yl)-acetamide;
2-(7-Methyl-4-oxo-3-phenyl-4H-quinolin-1-yl)-N-pyridin-2-ylmethyl-acetamide;
N-(2-Acetyl-thiophen-3-yl)-2-(7-methyl-4-oxo-3-phenyl-4H-quinolin-1-yl)-acetamide;
N-Ethyl-2-(7-methyl-4-oxo-3-phenyl-4H-quinolin-1-yl)-N-[1,3,4]thiadiazol-2-yl-acetamide;
N-(2-Methyl-1H-indol-5-yl)-2-(7-methyl-4-oxo-3-phenyl-4H-quinolin-1-yl)-acetamide;
1-[2-(3,6-Dihydro-2H-pyridin-1-yl)-2-oxo-ethyl]-7-methyl-3-phenyl-1H-quinolin-4-one;
2-(7-Methyl-4-oxo-3-phenyl-4H-quinolin-1-yl)-N-(1H-pyrazol-3-yl)-acetamide;
N-(4-Fluoro-phenyl)-2-(7-methyl-4-oxo-3-phenyl-4H-quinolin-1-yl)-acetamide;
2-(7-Methyl-4-oxo-3-phenyl-4H-quinolin-1-yl)-N-(5-trifluoromethyl-[1,3,4]thiadiazol-2-yl)-acetamide;
2-(7-Methyl-4-oxo-3-phenyl-4H-quinolin-1-yl)-N-quinolin-2-yl-acetamide;
2-(7-Methyl-4-oxo-3-phenyl-4H-quinolin-1-yl)-N-(2-methyl-2H-pyrazol-3-yl)-acetamide;
N-(5-Cyclopropyl-2H-pyrazol-3-yl)-2-(7-methyl-4-oxo-3-phenyl-4H-quinolin-1-yl)-acetamide;
N-Isoquinolin-3-yl-2-(7-methyl-4-oxo-3-phenyl-4H-quinolin-1-yl)-acetamide;
N-Indan-2-yl-2-(7-methyl-4-oxo-3-phenyl-4H-quinolin-1-yl)-acetamide;
2-(7-Methyl-4-oxo-3-phenyl-4H-quinolin-1-yl)-N-(2-thiophen-2-yl-ethyl)-acetamide;
7-Bromo-1-[2-(3,6-dihydro-2H-pyridin-1-yl)-2-oxo-ethyl]-3-phenyl-1H-quinolin-4-one;
(7-Bromo-4-oxo-3-phenyl-4H-quinolin-1-yl)-acetic acid hydrazide;
2-(7-Bromo-4-oxo-3-phenyl-4H-quinolin-1-yl)-N,N-dibutyl-acetamide;
7-Bromo-1-(2-oxo-2-pyrrolidin-1-yl-ethyl)-3-phenyl-1H-quinolin-4-one;
7-Bromo-1-(2-oxo-2-piperidin-1-yt-ethyl)-3-phenyl-1H-quinolin-4-one;
7-Bromo-1-[2-(2,6-dimethyl-morpholin-4-yl)-2-oxo-ethyl]-3-phenyl-1H-quinolin-4-one;
N,N-Diallyl-2-(7-bromo-4-oxo-3-phenyl-4H-quinolin-1-yl)-acetamide;
7-Bromo-1-[2-(2,5-dimethyl-2,5-dihydro-pyrrol-1-yl)-2-oxo-ethyl]-3-phenyl-1H-quinolin-4-one;
2-(7-Bromo-4-oxo-3-phenyl-4H-quinolin-1-yl)-N-cyclopropylmethyl-N-propyl-acetamide;
2-(7-Methyl-4-oxo-3-phenyl-4H-quinolin-1-yl)-N-(5-nitro-thiazol-2-yl)-acetamide;
N-Isoxazol-3-yl-2-(7-methyl-4-oxo-3-phenyl-4H-quinolin-1-yl)-acetamide; 2-(7-Methyl-4-oxo-3-phenyl-4H-quinolin-1-yl)-N-(4-methyl-thiazol-2-yl)-acetamide;
7-Bromo-1-(2-oxo-2-piperidin-1-yl-ethyl)-3-phenyl-1H-quinolin-4-one; 1-[2-(2,6-Dimethyl-morpholin-4-yl)-2-oxo-ethyl]-3-(4-methoxy-phenyl)-7-methyl-1H-quinolin-4-one;
1-[2-(3,5-Dimethyl-piperidin-1-yl)-2-oxo-ethyl]-3-(4-methoxy-phenyl)-7-methyl-1H-quinolin-4-one;
N-(6-Methoxy-pyrimidin-4-yl)-2-(7-methyl-4-oxo-3-phenyl-4H-quinolin-1-yl)-acetamide;
1-[2-(4-Hydroxy-piperidin-1-yl)-2-oxo-ethyl]-7-methyl-3-phenyl-1H-quinolin-4-one;
N-Isoxazol-3-yl-2-[3-(4-methoxy-phenyl)-7-methyl-4-oxo-4H-quinolin-1-yl]-acetamide; and
1-[2-(4-Hydroxy-piperidin-1-yl)-2-oxo-ethyl]-3-(4-methoxy-phenyl)-7-methyl-1H-quinolin-4-one; and
the pharmaceutically acceptable salts and hydrates thereof.

**8.** A compound selected from
(7-Methyl-4-oxo-2-phenyl-4H-quinolin-1-yl)-acetic acid ethyl ester;
(7-Methyl-4-oxo-3-*p*-tolyl-4H-quinolin-1-yl)-acetic acid methyl ester;
(7-Methyl-4-oxo-3-*p*-tolyl-4H-quinolin-1-yl)-acetic acid;
[3-(4-Chloro-phenyl)-7-methyl-4-oxo-4H-quinolin-1-yl]-acetic acid methyl ester;
[3-(4-Fluoro-phenyl)-7-methyl-4-oxo-4H-quinolin-1-yl]-acetic acid methyl ester;
[3-(4-Fluoro-phenyl)-7-methyl-4-oxo-4H-quinolin-1-yl]-acetic acid;
[3-(4-Chloro-phenyl)-7-methyl-4-oxo-4H-quinolin-1-yl]-acetic acid;
(7-Methyl-4-oxo-3-phenyl-4H-quinolin-1-yl)-acetic acid;
(5-Methyl-4-oxo-3-phenyl-4H-quinolin-1-yl)-acetic acid;
(7-Methyl-4-oxo-3-phenyl-4H-quinolin-1-yl)-acetic acid methyl ester;
(7-Methyl-4-oxo-3-phenyl-4H-quinolin-1-yl)-acetic acid;
[3-(4-Methoxy-phenyl)-7-methyl-4-oxo-4H-quinolin-1-yl]-acetic acid methyl ester;
[3-(4-Methoxy-phenyl)-7-methyl-4-oxo-4H-quinolin-1-yl]-acetic acid;
(7-Bromo-4-oxo-3-phenyl-4H-quinolin-1-yl)-acetic acid; and
the pharmaceutically acceptable salts and hydrates thereof.

**9.** A process for preparing a compound of formula **(I)** as defined in claim 1, comprising reacting an intermediate compound of formula **(VII):**

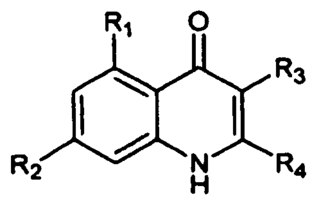

**(VII)**

wherein $R_1$, $R_2$, $R_3$ and $R_4$ are as defined for **(I)**, with the appropriate 2-bromo ethanone **(XII):**

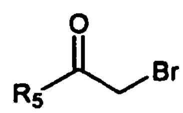

**(XII)**

wherein $R_5$ is as defined in claim 1.

**10.** A process according to claim 9 for preparing an amide compound of formula **(I)**, wherein $R_5$ is $NR_6R_7$, comprising reacting an acid compound of formula **(I)** as defined in claim 1, wherein $R_5$ is OH, with the appropriate amine $R_6R_7NH$ **(IX)**, wherein $R_6$ and $R_7$ are as defined in claim 1.

**11.** A process according to claim 9 for preparing an hydrazide compound of formula **(I)**, wherein $R_5$ is $N(R_8)NH_2$, comprising reacting an ester compound of formula **(I)**, wherein $R_5$ is $OR_9$, with the appropriate hydrazine of formula $NH_2NHR_8$ **(X)**, wherein $R_8$ is as defined in claim 1.

**12.** Use of a compound according to any one of claims 1 to 8 for the preparation of a medicament for treating or preventing diseases associated with the $GABA_A$ receptor modulation, selected from anxiety, epilepsy, sleep disorders or insomnia, for inducing sedation-hypnosis, anaesthesia or muscle relaxation or for modulating the necessary time to induce sleep and its duration in a human or non-human mammal in need thereof.

**13.** The use of claim 12 wherein the $GABA_A$ receptor is the $\alpha_1$-$GABA_A$ receptor or the $\alpha_2$-$GABA_A$ receptor.

**14.** A pharmaceutical composition comprising a therapeutically effective amount of a compound as defined in any one of claims 1 to 8, together with appropriate amounts of pharmaceutical excipients or carriers.

**15.** The use of a 1H-quinolin-one compound of formula **(I):**

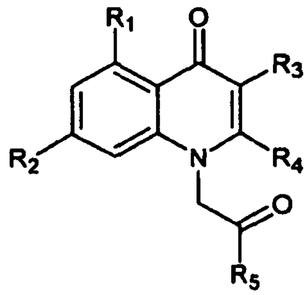

**(I)**

wherein
$R_5$ is selected from the group
consisting of $NR_6R_7$, $N(R_8)NH_2$, OH, $OR_9$ and a heteroaryl ring selected from the group consisting of pyridyl, furyl, thienyl, oxazolyl, isoxazolyl, imidazolyl, pyrrolyl, pyrazolyl, pyrimidinyl and pyrazinyl, wherein each heteroaryl ring contains one or two optional alkyl($C_1$-$C_6$) substituents;
$R_9$ is alkyl($C_1$-$C_6$); and
$R_1$, $R_2$, $R_3$, $R_4$, $R_6$, $R_7$, and $R_8$ are as defined in any one of claims 1 to 6;
or a pharmaceutically acceptable salt or hydrate thereof,
for the preparation of a medicament for treating or preventing diseases associated with the $GABA_A$ receptor modulation, selected from anxiety, epilepsy, sleep disorders or insomnia, for inducing sedation-hypnosis, anaesthesia or muscle relaxation or for modulating the necessary time to induce sleep and its duration in a human or non-human mammal in need thereof.

**16.** A 1H-quinoline-one compound as defined in any one of claims 1 to 8 or 15 for use in the treatment or prevention of diseases associated with the $GABA_A$ receptor modulation, selected from anxiety, epilepsy, sleep disorders or insomnia, for inducing sedation-hypnosis, anaesthesia or muscle relaxation or for modulating the necessary time to induce sleep and its duration in a human or non-human mammal in need thereof.

**17.** The 1H-quinoline-one compound of claim 16, wherein the $GABA_A$ receptor is the $\alpha_1$-$GABA_A$ receptor or the $\alpha_2$-$GABA_A$ receptor.

**Patentansprüche**

**1.** 1H-Chinolinon-Verbindung der Formel **(I):**

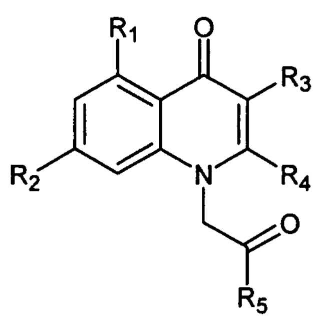

**(I)**

worin

$R_1$ und $R_2$ unabhängig voneinander ausgewählt sind aus der Gruppe, bestehend aus Wasserstoff, Alkyl($C_1$-$C_6$), O-Alkyl($C_1$-$C_6$), Halogen und Phenyl;

$R_3$ und $R_4$ unabhängig voneinander ausgewählt sind aus der Gruppe, bestehend aus Wasserstoff und Phenyl, das gegebenenfalls mit Alkyl($C_1$-$C_6$), Halogen und O-Alkyl($C_1$-$C_6$) substituiert ist;

$R_5$ ausgewählt ist aus der Gruppe, bestehend aus $NR_6R_7$, $N(R_8)NH_2$ und einem Heteroaryl-Ring, der ausgewählt ist aus der Gruppe, bestehend aus Pyridyl, Furyl, Thienyl, Oxazolyl, Isoxazolyl, Imidazolyl, Pyrrolyl, Pyrazolyl, Pyrimidinyl und Pyrazinyl, wobei jeder Heteroaryl-Ring gegebenenfalls einen oder zwei Alkyl($C_1$-$C_6$)-Substituenten enthält;

$R_6$ und $R_7$ unabhängig voneinander ausgewählt sind aus der Gruppe, bestehend aus Wasserstoff; Alkyl($C_1$-$C_6$); Alkenyl($C_2$-$C_6$); Cycloalkyl($C_3$-$C_6$)alkyl($C_1$-$C_6$); Hydroxyalkyl($C_1$-$C_6$); Heteroaryl, das ausgewählt ist aus der Gruppe, bestehend aus Pyridyl, Pyrimidinyl, Pyrazinyl, Thiazolyl, Benzothiazolyl, Oxazolyl, Benzoxazolyl, Isoxazolyl, Benzisoxazolyl, Pyrazolyl, Furyl, Benzofuryl, Thienyl, Benzothienyl, Thiadiazolyl, Pyrrolyl, Imidazolyl, Benzimidazolyl, Indolyl, Chinolyl und Isochinolyl, wobei jedes Heteroaryl gegebenenfalls einen oder zwei Substituenten enthält, die unabhängig voneinander ausgewählt sind aus der Gruppe, bestehend aus Alkyl($C_1$-$C_6$), O-Alkyl($C_1$-$C_6$), Halogenalkyl($C_1$-$C_6$), Cycloalkyl($C_3$-$C_6$), $NO_2$ und CO-Alkyl($C_1$-$C_6$); Thienylalkyl($C_1$-$C_6$), Furylalkyl($C_1$-$C_6$), Pyridylalkyl ($C_1$-$C_6$), 3-Methyl-2-thienylmethyl und Aryl, das ausgewählt ist aus der Gruppe, bestehend aus Phenyl und Indanyl, wobei jedes Aryl gegebenenfalls einen oder zwei Substituenten enthält, die ausgewählt sind aus der Gruppe, bestehend aus Alkyl($C_1$-$C_6$), Halogenalkyl($C_1$-$C_6$), Halogen, N-Dialkyl($C_1$-$C_6$), NH-Alkyl($C_1$-$C_6$), O-Alkyl($C_1$-$C_6$), $NO_2$, CN, OH, $NH_2$, COOH, CO-Alkyl($C_1$-$C_6$), COO-Alkyl($C_1$-$C_6$), CONH-Alkyl($C_1$-$C_6$), CON-Dialkyl($C_1$-$C_6$) und CO-Phenyl; unter der Bedingung, dass $NR_6R_7$ nicht $NH_2$, NH($C_1$-$C_4$ Alkyl) oder N($C_1$-$C_4$ Alkyl)$_2$ ist;

oder $R_6$ und $R_7$ zusammen mit dem Stickstoffatom, an das sie gebunden sind, einen Heterocyclus bilden können, der ausgewählt ist unter Pyrrolidin, 3-Pyrrolin, 1,2,3,6-Tetrahydropyridin, Piperidin, Morpholin, Thiomorpholin und Piperazin, wobei jeder Heterocyclus gegebenenfalls einen, zwei, drei oder vier Substituenten enthält, die ausgewählt sind aus der Gruppe, bestehend aus OH, Alkyl($C_1$-$C_6$) und CO-Alkyl($C_1$-$C_6$);

$R_8$ ausgewählt ist aus der Gruppe, bestehend aus Wasserstoff und Alkyl($C_1$-$C_6$);

unter der Bedingung, dass $R_3$ und $R_4$ nicht gleichzeitig Wasserstoff sein können;

und pharmazeutisch akzeptable Salze und Hydrate davon.

**2.** Verbindung nach Anspruch 1, worin $R^1$ ausgewählt ist aus der Gruppe, bestehend aus Wasserstoff, Alkyl($C_1$-$C_6$), O-Alkyl($C_1$-$C_6$), Halogen und Phenyl;
$R^2$ ausgewählt ist aus der Gruppe, bestehend aus Wasserstoff, Alkyl($C_1$-$C_6$), Halogen und Phenyl; und $R_3$, $R_4$, $R_5$, $R_6$ ,$R_7$, und $R_8$ wie in Anspruch 1 definiert sind.

**3.** Verbindung nach einem der vorhergehenden Ansprüche, worin $R_1$ und $R_2$ unabhängig voneinander ausgewählt sind aus der Gruppe, bestehend aus Wasserstoff, Methyl, Brom und Phenyl.

**4.** Verbindung nach einem der vorhergehenden Ansprüche, worin $R_3$ und $R_4$ unabhängig voneinander ausgewählt sind aus der Gruppe, bestehend aus Wasserstoff, Phenyl, 4-Fluorphenyl, 4-Chlorphenyl, p-Tolyl und 4-Methoxyphenyl.

**5.** Verbindung nach einem der vorhergehenden Ansprüche, worin $R_5$ ausgewählt ist aus der Gruppe, bestehend aus $NHNH_2$, N-(Methyl)$NH_2$, 2-Pyridyl, 3-Pyridyl, 4-Pyridyl, 2-Pyrazinyl, 5-Methyl-2-furyl, 2-Thienyl und 2,4-Dimethyl-5-oxazolyl.

**6.** Verbindung nach einem der vorhergehenden Ansprüche, worin $R_6$ und $R_7$ unabhängig voneinander ausgewählt sind aus der Gruppe, bestehend aus Hexyl, Allyl, Hydroxyethyl, Cyclopropylmethyl, 1,3,4-Thiadiazol-2-yl, 1,3-Dimethyl-5-pyrazolyl, 1-Methyl-3-pyrazolyl, 2,5-Dimethyl-3-pyrrolinyl, 1,2,3,6-Tetrahydropiperidinyl, 2,5-Dimethyl-3-pyrazolyl, 2-Acetyl-3-thienyl, 2-Indanyl, 2-Methyl-3-pyrazolyl, 2-Methyl-5-indolyl, 2-Pyrazinyl, 2-Pyrimidinyl, 2-Chinolyl, 2-Thiazolyl, 3,5-Isoxazol-4-yl, 3-Isoxazolyl, 3-Methyl-2-thienylmethyl, 3-Methyl-5-isoxazolyl, 3-Methyl-6-pyridyl, 3-Methylisoxazol-5-yl, 3-Oxazolyl, 3-Pyrazolyl, 1,3,4-Thiadiazol-2-yl, 4-Methyl-2-thiazolyl, 5-Trifluormethyl-1,3,4-thiadiazol-2-yl, 5-Cyclopropyl-3-pyrazolyl, 5-Methyl-2-pyridyl, 5-Methyl-3-pyrazolyl, 5-Methyl-isoxazol-3-yl, 5-Nitro-2-thiazol-2-yl, 6-Methoxy-4-pyrimidinyl, 2-Furylmethyl, 2-Pyridylmethyl, 2-Thienylethyl, Phenyl, *p*-Tolyl, 4-Trifluormethylphenyl, 4-Dimethylaminophenyl, 4-Fluorphenyl und 4-Methoxyphenyl; oder $R_6$ und $R_7$ gemeinsam mit dem Stickstoffatom, an das sie gebunden sind, Pyrrol, 2,5-Dimethyl-3-pyrrolin, Piperidin, 1,2,3,6-Tetrahydropyridin, 4-Hydroxypiperidin, 3,5-Dimethylpiperidin, Morpholin, 2,6-Dimethylmorpholin, 4-Methylpiperazin und 4-Acetylpiperazin bilden.

**7.** Verbindung nach Anspruch 1, die ausgewählt ist aus der Gruppe, bestehend aus

5-Methyl-1-(2-morpholin-4-yl-2-oxo-ethyl)-2-phenyl-1H-chinolin-4-on;
N,N-Diethyl-2-(7-methyl-4-oxo-2-phenyl-4H-chinolin-1-yl)-acetamid;
7-Methyl-1-(2-morpholin-4-yl-2-oxo-ethyl)-2-phenyl-1H-chinolin-4-on;
N,N-Dibutyl-2-(7-methyl-4-oxo-2-phenyl-4H-chinolin-1-yl)-acetamid;
N,N-Diisopropyl-2-(5-methyl-4-oxo-2-phenyl-4H-chinolin-1-yl)-acetamid;
5-Methyl-1-(2-oxo-2-pyrrolidin-1-yl-ethyl)-2-phenyl-1H-chinolin-4-on;
2-(7-Methyl-4-oxo-2-phenyl-4H-chinolin-1-yl)-N,N-dipropyl-acetamid;
N,N-Dihexyl-2-(5-methyl-4-oxo-2-phenyl-4H-chinolin-1-yl)-acetamid;
5-Methyl-1-(2-oxo-2-piperidin-1-yl-ethyl)-2-phenyl-1H-chinolin-4-on;
5-Methyl-1-(2-oxo-2-pyrrolidin-1-yl-ethyl)-2-phenyl-1H-chinolin-4-on;
7-Methyl-1-(2-oxo-2-pyrrolidin-1-yl-ethyl)-2-phenyl-1H-chinolin-4-on;
N,N-Dibutyl-2-(7-methyl-4-oxo-3-phenyl-4H-chinolin-1-yl)-acetamid;
2-(7-Methyl-4-oxo-3-phenyl-4H-chinolin-1-yl)-N,N-dipropyl-acetamid;
7-Methyl-1-(2-oxo-2-piperidin-1-yl-ethyl)-3-phenyl-1H-chinolin-4-on;
N, N-Dihexyl-2-(7-methyl-4-oxo-3-phenyl-4H-chinolin-1-yl)-acetamid;
N,N-Diethyl-2-(7-methyl-4-oxo-3-phenyl-4H-chinolin-1-yl)-acetamid;
7-Methyl-1-(2-oxo-2-pyrrolidin-1-yl-ethyl)-3-phenyl-1H-chinolin-4-on;
7-Methyl-1-(2-morpholin-4-yl-2-oxo-ethyl)-3-phenyl-1H-chinolin-4-on;
N,N-Diisopropyl-2-(7-methyl-4-oxo-3-phenyl-4H-chinolin-1-yl)-acetamid;
N,N-Dihexyl-2-(5-methyl-4-oxo-3-phenyl-4H-chinolin-1-yl)-acetamid;
5-Methyl-1-(2-oxo-2-piperidin-1-yl-ethyl)-3-phenyl-1H-chinolin-4-on;
5-Methyl-1-(2-morpholin-4-yl-2-oxo-ethyl)-3-phenyl-1H-chinolin-4-on;
N,N-Diethyl-2-(5-methyl-4-oxo-3-phenyl-4H-chinolin-1-yl)-acetamid;
N,N-Dibutyl-2-(5-methyl-4-oxo-3-phenyl-4H-chinolin-1-yl)-acetamid;
7-Methyl-1-(2-oxo-2-pyridin-3-yl-ethyl)-3-phenyl-1H-chinolin-4-on;
7-Methyl-1-(2-oxo-2-thiophen-2-yl-ethyl)-3-phenyl-1H-chinolin-4-on;
7-Methyl-1-(2-oxo-2-pyridin-2-yl-ethyl)-3-phenyl-1H-chinolin-4-on;
7-Methyl-1-(2-oxo-2-pyridin-4-yl-ethyl)-3-phenyl-1H-chinolin-4-on;
1-[2-(4-Acetyl-piperazin-1-yl)-2-oxo-ethyl]-7-methyl-3-phenyl-1H-chinolin-4-on;
7-Methyl-1-[2-(5-methyl-furan-2-yl)-2-oxo-ethyl]-3-phenyl-1H-chinolin-4-on;
1-[2-(2,6-Dimethyl-morpholin-4-yl)-2-oxo-ethyl]-7-methyl-3-phenyl-1H-chinolin-4-on;
1-[2-(2,4-Dimethyl-oxazol-5-yl)-2-oxo-ethyl]-7-methyl-3-phenyl-1H-chinolin-4-on;
7-Methyl-1-(2-oxo-2-pyrazin-2-yl-ethyl)-3-phenyl-1H-chinolin-4-on;
7-Methyl-1-[2-(4-methyl-piperazin-1-yl)-2-oxo-ethyl]-3-phenyl-1H-chinolin-4-on;
2-[3-(4-Methoxy-phenyl)-7-methyl-4-oxo-4H-ch inolin-1-yl]-N, N-dipropyl-acetamid;
2-[3-(4-Chlor-phenyl)-7-methyl-4-oxo-4H-chinolin-1-yl]-N, N-dipropyl-acetamid;
2-(7-Methyl-4-oxo-3-p-tolyl-4H-chinolin-1-yl)-N,N-dipropyl-acetamid;
N-Cyclopropylmethyl-2-(7-methyl-4-oxo-3-phenyl-4H-chinolin-1-yl)-N-propyl-acetamid;
1-[2-(3,5-Dimethyl-piperidin-1-yl)-2-oxo-ethyl]-7-methyl-3-phenyl-1H-chinolin-4-on;
1-[2-(3,6-Dihydro-2H-pyridin-1-yl)-2-oxo-ethyl]-7-methyl-3-phenyl-H-chinolin-4-on;
1-[2-(2,5-Dimethyl-2,5-d ihydro-pyrrol-1-yl)-2-oxo-ethyl]-7-methyl-3-phenyl-1 H-chinolin-4-on;
N,N-Diallyl-2-(7-methyl-4-oxo-3-phenyl-4H-chinolin-1-yl)-acetamid;
2-(7-Brom-4-oxo-3-phenyl-4H-chinolin-1-yl)-N,N-dipropyl-acetamid;
2-(5-Brom-4-oxo-3-phenyl-4H-chinolin-1-yl)-N,N-dipropyl-acetamid;
2-(4-Oxo-3,7-diphenyl-4H-chinolin-1-yl)-N,N-dipropyl-acetamid;
2-(4-Oxo-3-phenyl-4H-chinolin-1-yl)-N,N-dipropyl-acetamid;
2-(4-Oxo-3,5-diphenyl-4H-chinolin-1-yl)-N,N-dipropyl-acetamid;
2-[3-(4-Chlor-phenyl)-7-methyl-4-oxo-4H-chinolin-1-yl]-N-phenyl-acetamid;
2-[3-(4-Chlor-phenyl)-7-methyl-4-oxo-4H-chinolin-1-yl]-N-pyrazin-2-yl-acetamid;
[3-(4-Chlor-phenyl)-7-methyl-4-oxo-4H-chinolin-1-yl]-essigsäurehydrazid;
2-(7-Methyl-4-oxo-3-p-tolyl-4H-chinolin-1-yl)-N-phenyl-acetamid;
2-[3-(4-Fluor-phenyl)-7-methyl-4-oxo-4H-chinolin-1-yl]-N-phenyl-acetamid;
2-(7-Methyl-4-oxo-3-p-tolyl-4H-chinolin-1-yl)-N-thiazol-2-yl-acetamid;
N-(5-Methyl-isoxazol-3-yl)-2-(7-methyl-4-oxo-3-p-tolyl-4H-chinolin-1-yl)-acetamid;
[3-(4-Fluor-phenyl)-7-methyl-4-oxo-4H-chinolin-1-yl]-essigsäurehydrazid;
(7-Methyl-4-oxo-3-p-tolyl-4H-chinolin-1-yl)-essigsäurehydrazid;
2-[3-(4-Chlor-phenyl)-7-methyl-4-oxo-4H-chinolin-1-yl]-N-(5-methyl-isoxazol-3-yl)-acetamid;
2-[3-(4-Fluor-phenyl)-7-methyl-4-oxo-4H-chinolin-1-yl]-N-(5-methyl-isoxazol-3-yl)-acetamid;

N-Ethyl-N-(2-hydroxy-ethyl)-2-(7-methyl-4-oxo-3-phenyl-4H-chinolin-1-yl)-acetamid;
N-(2-Hydroxy-ethyl)-2-(7-methyl-4-oxo-3-phenyl-4H-chinolin-1-yl)-N-propyl-acetamid;
N,N-Diallyl-2-(5-methyl-4-oxo-3-phenyl-4H-chinolin-1-yl)-acetamid;
N-(2-Hydroxy-ethyl)-2-(5-methyl-4-oxo-3-phenyl-4H-chinolin-1-yl)-N-propyl-acetamid;
[3-(4-Chlor-phenyl)-7-methyl-4-oxo-4H-chinolin-1-yl]-essigsäure-N-methyl-hydrazid;
(7-Methyl-4-oxo-3-p-tolyl-4H-chinolin-1-yl)-essigsäure-N-methyl-hydrazid;
[3-(4-Fluor-phenyl)-7-methyl-4-oxo-4H-chinolin-1-yl]-essigsäure-N-methyl-hydrazid;
2-[3-(4-Fluor-phenyl)-7-methyl-4-oxo-4H-chinolin-1-yl]-N-thiazol-2-yl-acetamid;
2-[3-(4-Chlor-phenyl)-7-methyl-4-oxo-4H-chinolin-1-yl]-N-thiazol-2-yl-acetamid;
2-[3-(4-Methoxy-phenyl)-7-methyl-4-oxo-4H-chinolin-1-yl]-N-(4-trifluormethyl-phenyl)-acetamid;
2-(7-Methyl-4-oxo-3-phenyl-4H-chinolin-1-yl)-N-(4-trifluormethyl-phenyl)-acetamid;
N-(4-Methoxy-phenyl)-2-(7-methyl-4-oxo-3-phenyl-4H-chinolin-1-yl)-acetamid;
N-(4-Methoxy-phenyl)-2-[3-(4-methoxy-phenyl)-7-methyl-4-oxo-4H-chinolin-1-yl]-acetam id;
2-[3-(4-Methoxy-phenyl)-7-methyl-4-oxo-4H-chinolin-1-yl]-N-(5-methyl-2H-pyrazol-3-yl)-acetamid;
2-(7-Methyl-4-oxo-3-phenyl-4H-chinolin-1-yl)-N-thiazol-2-yl-acetamid;
2-[3-(4-Methoxy-phenyl)-7-methyl-4-oxo-4H-chinolin-1-yl]-N-thiazol-2-yl-acetamid;
2-(7-Methyl-4-oxo-3-phenyl-4H-chinolin-1-yl)-N-(3-methyl-thiophen-2-ylmethyl)-acetamid;
2-[3-(4-Methoxy-phenyl)-7-methyl-4-oxo-4H-chinolin-1-yl]-N-(3-methyl-isoxazol-5-yl)-acetamid;
2-[3-(4-Methoxy-phenyl)-7-methyl-4-oxo-4H-chinolin-1-yl]-N-(5-methyl-pyridin-2-yl)-acetamid;
2-(7-Methyl-4-oxo-3-phenyl-4H-chinolin-1-yl)-N-pyrimidin-2-yl-acetamid;
N-Furan-2-ylmethyl-2-(7-methyl-4-oxo-3-phenyl-4H-chinolin-1-yl)-acetamid;
N-(2,5-Dimethyl-2H-pyrazol-3-yl)-2-[3-(4-methoxy-phenyl)-7-methyl-4-oxo-4H-chinolin-1-yl]-acetamid;
N,N-Diethyl-2-[3-(4-methoxy-phenyl)-7-methyl-4-oxo-4H-chinolin-1-yl]-acetamid;
3-(4-Methoxy-phenyl)-7-methyl-1-(2-oxo-2-pyrrolidin-1-yl-ethyl)-1H-chinolin-4-on;
3-(4-Methoxy-phenyl)-7-methyl-1-(2-oxo-2-piperidin-1-yl-ethyl)-1H-chinolin-4-on;
N-Ethyl-N-(2-hydroxy-ethyl)-2-[3-(4-methoxy-phenyl)-7-methyl-4-oxo-4H-chinolin-1-yl]-acetamid;
N-(2-Hydroxy-ethyl)-2-[3-(4-methoxy-phenyl)-7-methyl-4-oxo-4H-chinolin-1-yl]-N-propyl-acetamid;
N-Cyclopropylmethyl-2-[3-(4-methoxy-phenyl)-7-methyl-4-oxo-4H-chinolin-1-yl]-N-propyl-acetamid;
N, N-Bis-(2-hydroxy-ethyl)-2-[3-(4-methoxy-phenyl)-7-methyl-4-oxo-4H-chinolin-1-yl]-acetamid;
N,N-Diallyl-2-[3-(4-methoxy-phenyl)-7-methyl-4-oxo-4H-chinolin-1-yl]-acetamid;
N-(3,5-Dimethyl-isoxazol-4-yl)-2-(7-methyl-4-oxo-3-phenyl-4H-chinolin-1-yl)-acetamid;
2-(7-Methyl-4-oxo-3-phenyl-4H-chinolin-1-yl)-N-(1-methyl-1H-pyrazol-3-yl)-acetamid;
N-(2,5-Dimethyl-2H-pyrazol-3-yl)-2-(7-methyl-4-oxo-3-phenyl-4H-chinolin-1-yl)-acetamid;
2-(7-Methyl-4-oxo-3-phenyl-4H-chinolin-1-yl)-N-(5-methyl-pyridin-2-yl)-acetamid;
2-(7-Methyl-4-oxo-3-phenyl-4H-chinolin-1-yl)-N-[1,3,4]thiadiazol-2-yl-acetamid;
N-(4-Dimethylamino-phenyl)-2-(7-methyl-4-oxo-3-phenyl-4H-chinolin-1-yl)-acetamid;
N-(3-Methyl-isoxazol-5-yl)-2-(7-methyl-4-oxo-3-phenyl-4H-chinolin-1-yl)-acetamid;
2-(7-Methyl-4-oxo-3-phenyl-4H-chinolin-1-yl)-N-p-tolyl-acetamid;
2-[3-(4-Methoxy-phenyl)-7-methyl-4-oxo-4H-chinolin-1-yl]-N-(1-methyl-1H-pyrazol-3-yl)-acetamid;
1-[2-(2,5-Dimethyl-2,5-dihydro-pyrrol-1-yl)-2-oxo-ethyl]-3-(4-methoxy-phenyl)-7-methyl-1H-chinolin-4-on;
2-(7-Methyl-4-oxo-3-phenyl-4H-chinolin-1-yl)-N-(5-methyl-2H-pyrazol-3-yl)-acetamid;
2-(7-Methyl-4-oxo-3-phenyl-4H-chinolin-1-yl)-N-pyridin-2-ylmethyl-acetamid;
N-(2-Acetyl-thiophen-3-yl)-2-(7-methyl-4-oxo-3-phenyl-4H-chinolin-1-yl)-acetamid;
N-Ethyl-2-(7-methyl-4-oxo-3-phenyl-4H-chinolin-1-yl)-N-[1,3,4]thiadiazol-2-yl-acetamid;
N-(2-Methyl-1H-indol-5-yl)-2-(7-methyl-4-oxo-3-phenyl-4H-chinolin-1-yl)-acetamid;
1-[2-(3,6-Dihydro-2H-pyridin-1-yl)-2-oxo-ethyl]-7-methyl-3-phenyl-1H-chinolin-4-on;
2-(7-Methyl-4-oxo-3-phenyl-4H-chinolin-1-yl)-N-(1H-pyrazol-3-yl)-acetamid;
N-(4-Fluor-phenyl)-2-(7-methyl-4-oxo-3-phenyl-4H-chinolin-1-yl)-acetamid;
2-(7-Methyl-4-oxo-3-phenyl-4H-chinolin-1-yl)-N-(5-trifluormethyl-[1,3,4]thiadiazol-2-yl)-acetamid;
2-(7-Methyl-4-oxo-3-phenyl-4H-chinolin-1-yl)-N-chinolin-2-yl-acetamid;
2-(7-Methyl-4-oxo-3-phenyl-4H-chinolin-1-yl)-N-(2-methyl-2H-pyrazol-3-yl)-acetamid;
N-(5-Cyclopropyl-2H-pyrazol-3-yl)-2-(7-methyl-4-oxo-3-phenyl-4H-chinolin-1-yl)-acetamid;
N-Isochinolin-3-yl-2-(7-methyl-4-oxo-3-phenyl-4H-chinolin-1-yl)-acetamid;
N-Indan-2-yl-2-(7-methyl-4-oxo-3-phenyl-4H-chinolin-1-yl)-acetamid;
2-(7-Methyl-4-oxo-3-phenyl-4H-chinolin-1-yl)-N-(2-thiophen-2-yl-ethyl)-acetamid;
7-Brom-1-[2-(3,6-dihydro-2H-pyridin-1-yl)-2-oxo-ethyl]-3-phenyl-1H-chinolin-4-on;
(7-Brom-4-oxo-3-phenyl-4H-chinolin-1-yl)-essigsäurehydrazid;
2-(7-Brom-4-oxo-3-phenyl-4H-chinolin-1-yl)-N,N-dibutyl-acetamid;

7-Brom-1-(2-oxo-2-pyrrolidin-1-yl-ethyl)-3-phenyl-1H-chinolin-4-on;
7-Brom-1-(2-oxo-2-piperidin-1-yl-ethyl)-3-phenyl-1H-chinolin-4-on;
7-Brom-1-[2-(2,6-dimethyl-morpholin-4-yl)-2-oxo-ethyl]-3-phenyl-1H-chinolin-4-on;
N,N-Diallyl-2-(7-brom-4-oxo-3-phenyl-4H-chinolin-1-yl)-acetamid;
7-Brom-1-[2-(2,5-dimethyl-2,5-dihydro-pyrrol-1-yl)-2-oxo-ethyl]-3-phenyl-1H-chinolin-4-on;
2-(7-Brom-4-oxo-3-phenyl-4H-chinolin-1-yl)-N-cyclopropylmethyl-N-propyl-acetamid;
2-(7-Methyl-4-oxo-3-phenyl-4H-chinolin-1-yl)-N-(5-nitro-thiazol-2-yl)-acetamid;
N-Isoxazol-3-yl-2-(7-methyl-4-oxo-3-phenyl-4H-chinolin-1-yl)-acetamid;
2-(7-Methyl-4-oxo-3-phenyl-4H-chinolin-1-yl)-N-(4-methyl-thiazol-2-yl)-acetamid;
7-Brom-1-(2-oxo-2-piperidin-1-yl-ethyl)-3-phenyl-1H-chinolin-4-on;
1-[2-(2,6-Dimethyl-morpholin-4-yl)-2-oxo-ethyl]-3-(4-methoxy-phenyl)-7-methyl-1H-chinolin-4-on;
1-[2-(3, 5-Dimethyl-piperidin-1-yl)-2-oxo-ethyl]-3-(4-methoxy-phenyl)-7-methyl-1H-chinolin-4-on;
N-(6-Methoxy-pyrimidin-4-yl)-2-(7-methyl-4-oxo-3-phenyl-4H-chinolin-1-yl)-acetamid;
1-[2-(4-Hydroxy-piperidin-1-yl)-2-oxo-ethyl]-7-methyl-3-phenyl-1H-chinolin-4-on;
N-Isoxazol-3-yl-2-[3-(4-methoxy-phenyl)-7-methyl-4-oxo-4H-chinolin-1-yl]-acetamid; und
1-[2-(4-Hydroxy-piperidin-1-yl)-2-oxo-ethyl]-3-(4-methoxy-phenyl)-7-methyl-1H-chinolin-4-on; und
die pharmazeutisch akzeptablen Salze und Hydrate davon.

**8.** Verbindung, ausgewählt unter
(7-Methyl-4-oxo-2-phenyl-4H-chinolin-1-yl)-essigsäureethylester;
(7-Methyl-4-oxo-3-p-tolyl-4H-chinolin-1-yl)-essigsäuremethylester;
(7-Methyl-4-oxo-3-p-tolyl-4H-chinolin-1-yl)-essigsäure;
[3-(4-Chlor-phenyl)-7-methyl-4-oxo-4H-chinolin-1-yl]-essigsäuremethylester;
[3-(4-Fluor-phenyl)-7-methyl-4-oxo-4H-chinolin-1-yl]-essigsäuremethylester;
[3-(4-Fluor-phenyl)-7-methyl-4-oxo-4H-chinolin-1-yl]-essigsäure;
[3-(4-Chlor-phenyl)-7-methyl-4-oxo-4H-chinolin-1-yl]-essigsäure;
(7-Methyl-4-oxo-3-phenyl-4H-chinolin-1-yl)-essigsäure;
(5-Methyl-4-oxo-3-phenyl-4H-chinolin-1-yl)-essigsäure;
(7-Methyl-4-oxo-3-phenyl-4H-chinolin-1-yl)-essigsäuremethylester;
(7-Methyl-4-oxo-3-phenyl-4H-chinolin-1-yl)-essigsäure;
[3-(4-Methoxy-phenyl)-7-methyl-4-oxo-4H-chinolin-1-yl]-essigsäuremethylester;
[3-(4-Methoxy-phenyl)-7-methyl-4-oxo-4H-chinolin-1-yl]-essigsäure;
(7-Brom-4-oxo-3-phenyl-4H-chinolin-1-yl)-essigsäure; und
die pharmazeutisch akzeptablen Salze und Hydrate davon.

**9.** Verfahren zur Herstellung einer Verbindung der Formel **(I),** wie in Anspruch 1 definiert, umfassend das Reagieren eines Zwischenprodukts der Formel **(VII):**

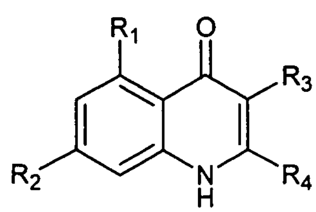

**(VII)**

worin $R_1$, $R_2$, $R_3$ und $R_4$ wie für **(I)** definiert sind, mit einem geeigneten 2-Bromethanon **(XII):**

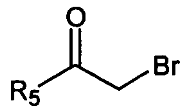

**(XII)**

worin $R_5$ wie in Anspruch 1 definiert ist.

**10.** Verfahren nach Anspruch 9 zur Herstellung einer Amid-Verbindung der Formel **(I),** worin $R_5$ für $NR_6R_7$ steht, umfassend das Reagieren einer sauren Verbindung der Formel (I), wie in Anspruch 1 definiert, worin $R_5$ für OH steht, mit einem geeigneten Amin $R_6R_7NH$ **(IX),** worin $R_6$ und $R_7$ wie in Anspruch 1 definiert sind.

**11.** Verfahren nach Anspruch 9 zur Herstellung einer Hydrazid-Verbindung der Formel (I), worin $R_5$ für $N(R_8)NH_2$ steht, umfassend das Reagieren einer Ester-Verbindung der Formel (I), wie in Anspruch 1 definiert, worin $R_5$ für $OR_9$ steht, mit einem geeigneten Hydrazin $NH_2NHR_8$ **(X),** worin $R_8$ wie in Anspruch 1 definiert ist.

**12.** Verwendung einer Verbindung nach einem der Ansprüche 1 bis 8 bei der Herstellung eines Medikaments zur Behandlung oder Vermeidung von Krankheiten, die mit der $GABA_A$-Rezeptormodulation zusammenhängen, ausgewählt unter Angst, Epilepsie, Schlafstörungen oder Schlaflosigkeit, zur Induktion von Sedations-Hypnose, Anästhesie oder Muskelrelaxation, oder zur Modulierung der erforderlichen Zeit zur Induktion von Schlaf und seiner Dauer bei einem menschlichen oder nicht-menschlichen Säuger, der dessen bedarf.

**13.** Verwendung nach Anspruch 12, worin der $GABA_A$-Rezeptor der $\alpha_1$-$GABA_A$-Rezeptor oder der $\alpha_2$-$GABA_A$-Rezeptor ist.

**14.** Pharmazeutische Zusammensetzung, umfassend einen therapeutisch wirksamen Anteil einer Verbindung, wie in den Ansprüchen 1 bis 8 definiert, gemeinsam mit geeigneten Anteilen pharmazeutischer Exzipienten oder Trägern.

**15.** Verwendung einer 1H-Chinolin-on-Verbindung der Formel **(I):**

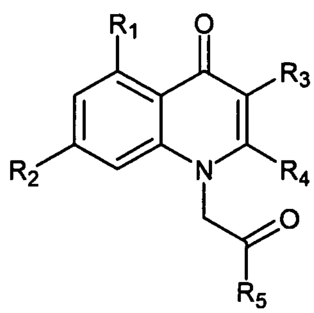

**(I)**

worin
$R_5$ ausgewählt ist aus der Gruppe, bestehend aus $NR_6R_7$, $N(R_8)NH_2$, OH, $OR_9$ und einem Heteroaryl-Ring, der ausgewählt ist aus der Gruppe, bestehend aus Pyridyl, Furyl, Thienyl, Oxazolyl, Isoxazolyl, Imidazolyl, Pyrrolyl, Pyrazolyl, Pyrimidinyl und Pyrazinyl, worin jeder Heteroaryl-Ring gegebenenfalls einen oder zwei Alkyl($C_1$-$C_6$)-Substituenten enthält;
$R_9$ für Alkyl($C_1$-$C_6$) steht; und
$R_1$, $R_2$, $R_3$, $R_4$, $R_6$, $R_7$, und $R_8$ wie in einem der Ansprüche 1 bis 6 definiert sind;
oder ein pharmazeutisches Salz oder Hydrat davon,

bei der Herstellung eines Medikaments zur Behandlung oder Vermeidung von Krankheiten, die mit der $GABA_A$-Rezeptormodulation zusammenhängen, ausgewählt unter Angst, Epilepsie, Schlafstörungen oder Schlaflosigkeit, zur Induktion von Sedations-Hypnose, Anästhesie oder Muskelrelaxation, oder zur Modulierung der erforderlichen Zeit zur Induktion von Schlaf und seiner Dauer bei einem menschlichen oder nicht-menschlichen Säuger, der dessen bedarf.

**16.** 1H-Chinolin-on-Verbindung wie in einem der Ansprüche 1 bis 8 oder 15 definiert, zur Verwendung bei der Behandlung oder Vermeidung von Krankheiten, die mit der $GABA_A$-Rezeptormodulation zusammenhängen, ausgewählt unter Angst, Epilepsie, Schlafstörungen oder Schlaflosigkeit, zur Induktion von Sedations-Hypnose, Anästhesie oder Muskelrelaxation, oder zur Modulierung der erforderlichen Zeit zur Induktion von Schlaf und seiner Dauer bei einem menschlichen oder nicht-menschlichen Säuger, der dessen bedarf.

**17.** 1H-Chinolin-on Verbindung nach Anspruch 16, worin der $GABA_A$-Rezeptor der $\alpha_1$-$GABA_A$-Rezeptor oder der $\alpha_2$-$GABA_A$-Rezeptor ist.

**Revendications**

**1.** 1H-quinoléinone répondant à la formule (I) :

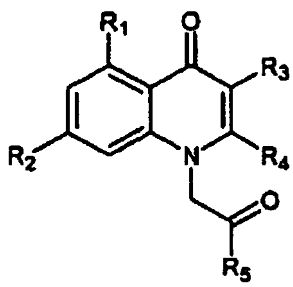

(I)

dans laquelle

$R_1$ et $R_2$ sont indépendamment choisis dans le groupe constitué par un atome d'hydrogène, un groupe alkyle en $C_1$ à $C_6$, un groupe O-alkyle en $C_1$ à $C_6$, un atome d'halogène et un groupe phényle ;

$R_3$ et $R_4$ sont indépendamment choisis dans le groupe constitué par un atome d'hydrogène et un groupe phényle facultativement substitué par un groupe alkyle en $C_1$ à $C_6$, un atome d'halogène et un groupe O-alkyle en $C_1$ à $C_6$ ;

$R_5$ est choisi dans le groupe constitué par $NR_6R_7$, N ($R_8$) $NH_2$ et un cycle hétéroaryle choisi dans le groupe constitué par un groupe pyridyle, un groupe furyle, un groupe thiényle, un groupe oxazolyle, un groupe isoxazolyle, un groupe imidazolyle, un groupe pyrrolyle, un groupe pyrazolyle, un groupe pyrimidinyle et un groupe pyrazinyle, chaque cycle hétéroaryle contenant un ou deux substituants alkyle en $C_1$-$C_6$ facultatifs ;

$R_6$ et $R_7$ sont indépendamment choisis dans le groupe constitué par un atome d'hydrogène, un groupe alkyle en $C_1$ à $C_6$, un groupe alcényle en $C_2$ à $C_6$, un groupe (cycloalkyle en $C_3$ à $C_6$) alkyle en $C_1$ à $C_6$, un groupe hydroxyalkyle en $C_1$ à $C_6$, un groupe hétéroaryle choisi dans le groupe constitué par les groupes pyridyle, pyrimidinyle, pyrazinyle, thiazolyle, benzothiazolyle, oxazolyle, benzoxazolyle, isoxazolyle, benzisoxazolyle, pyrazolyle, furyle, benzofuryle, thiényle, benzothiényle, thiadiazolyle, pyrrolyle, imidazolyle, benzimidazolyle, indolyle, quinoléyle et isoquinoléyle, chaque groupe hétéroaryle contenant un ou deux substituants facultatifs indépendamment choisis dans le groupe constitué par un groupe alkyle en $C_1$ à $C_6$, un groupe O-alkyle en $C_1$ à $C_6$, un groupe halogénoalkyle en $C_1$ à $C_6$, un groupe cycloalkyle en $C_3$-$C_6$, $NO_2$ et un groupe CO-alkyle en $C_1$ à $C_6$ ; un groupe thiénylalkyle en $C_1$ à $C_6$, un groupe furylalkyle en $C_1$ à $C_6$, un groupe pyridylalkyle en $C_1$ à $C_6$, un groupe 3-méthyl-2-thiénylméthyle, et un groupe aryle choisi dans le groupe constitué par un groupe phényle et un groupe indanyle, chaque groupe aryle contenant un ou deux substituants facultatifs indépendamment choisis dans le groupe constitué par un groupe alkyle en $C_1$ à $C_6$, un groupe halogénoalkyle en $C_1$ à $C_6$, un atome d'halogène, un groupe N-dialkyle en $C_1$ à $C_6$, un groupe NH-alkyle en $C_1$ à $C_6$, un groupe O-alkyle en $C_1$ à $C_6$, $NO_2$, CN, OH, $NH_2$, COOH, un groupe CO-alkyle en $C_1$ à $C_6$, un groupe COO-alkyle en $C_1$ à $C_6$, un groupe CONH-alkyle en $C_1$ à $C_6$, un groupe CON-dialkyle en $C_1$ à $C_6$ et un groupe CO-phényle ; à condition que $NR_6R_7$ ne soit pas $NH_2$, NH(alkyle en $C_1$-$C_4$) ou N(alkyle en $C_1$-C4$)_2$.

ou $R_6$ et $R_7$ peuvent former ensemble avec l'atome d'azote auxquels ils sont attachés un hétérocycle choisi parmi

un groupe pyrrolidine, un groupe 3-pyrroline, un groupe 1,2,3,6-tétrahydropyridine, un groupe pipéridine, un groupe morpholine, un groupe thiomorpholine et un groupe pipérazine, chaque hétérocycle contenant un, deux, trois ou quatre substituants facultatifs choisis dans le groupe constitué par OH, un groupe alkyle en $C_1$ à $C_6$, et un groupe CO-alkyle en $C_1$ à $C_6$ ;
$R_8$ est choisi dans le groupe constitué par un atome d'hydrogène et un groupe alkyle en $C_1$ à $C_6$ ;
à condition que $R_3$ et $R_4$ ne soient pas simultanément des atomes d'hydrogène ;
et ses sels et hydrates pharmaceutiquement acceptables.

**2.** Composé selon la revendication 1, dans lequel $R^1$ est choisi dans le groupe constitué par un atome d'hydrogène, un groupe alkyle en $C_1$ à $C_6$, un groupe O-alkyle en $C_1$ à $C_6$, un atome d'halogène et un groupe phényle ; $R^2$ est choisi dans le groupe constitué par un atome d'hydrogène, un groupe alkyle en $C_1$ à $C_6$, un atome d'halogène et un groupe phényle ; et $R_3$, $R_4$, $R_5$, $R_6$, $R_7$ et $R_8$ sont tels que définis selon la revendication 1.

**3.** Composé selon l'une quelconque des revendications précédentes, dans lequel $R_1$ et $R_2$ sont indépendamment choisis dans le groupe constitué par un atome d'hydrogène, un groupe méthyle, un atome de brome et un groupe phényle.

**4.** Composé selon l'une quelconque des revendications précédentes, dans lequel $R_3$ et $R_4$ sont indépendamment choisis dans le groupe constitué par un atome d'hydrogène, un groupe phényle, un groupe 4-fluorophényle, un groupe 4-chlorophényle, un groupe p-tolyle et un groupe 4-méthoxyphényle.

**5.** Composé selon l'une quelconque des revendications précédentes, dans lequel $R_5$ est choisi dans le groupe constitué par un groupe $NHNH_2$, un groupe N(méthyle)$NH_2$, un groupe 2-pyridyle, un groupe 3-pyridyle, un groupe 4-pyridyle, un groupe 2-pyrazinyle, un groupe 5-méthyl-2-furyle, un groupe 2-thiényle et un groupe 2,4-diméthyl-5-oxazolyle.

**6.** Composé selon l'une quelconque des revendications précédentes, dans lequel $R_6$ et $R_7$ sont indépendamment choisis dans le groupe constitué par les groupes hexyle, allyle, hydroxyéthyle, cyclopropylméthyle, 1,3,4-thiadiazol-2-yle, 1,3-diméthyl-5-pyrazolyle, 1-méthyl-3-pyrazolyle, 2,5-diméthyl-3-pyrrolinyle, 1,2,3,6-tétrahydropipéridinyle, 2,5-diméthyl-3-pyrazolyle, 2-acétyl-3-thiényle, 2-indanyle, 2-méthyl-3-pyrazolyle, 2-méthyl-5-indolyle, 2-pyrazinyle, 2-pyrimidinyle, 2-quinoléyle, 2-thiazolyle, 3,5-isoxazol-4-yle, 3-isoxazolyle, 3-méthyl-2-thiénylméthyle, 3-méthyl-5-isoxazolyle, 3-méthyl-6-pyridyle, 3-méthylisoxazol-5-yle, 3-oxazolyle, 3-pyrazolyle, 1,3,4-thiadiazol-2-yle, 4-méthyl-2-thiazolyle, 5-trifluorométhyl-1,3,4-thiadiazol-2-yle, 5-cyclopropyl-3-pyrazolyle, 5-méthyl-2-pyridyle, 5-méthyl-3-pyrazolyle, 5-méthyl-isoxazol-3-yle, 5-nitro-2-thiazol-2-yle, 6-méthoxy-4-pyrimidinyle, 2-furylméthyle, 2-pyridylméthyle, 2-thiényléthyle, phényle, *p*-tolyle, 4-trifluorométhylphényle, 4-diméthylaminophényle, 4-fluorophényle et 4-méthoxyphényle ; ou $R_6$ et $R_7$ forment ensemble avec l'atome d'azote auxquels ils sont attachés les groupes pyrrole, 2,5-diméthyl-3-pyrroline, pipéridine, 1,2,3,6-tétrahydropyridine, 4-hydroxypipéridine, 3,5-diméthylpipéridine, morpholine, 2,6-diméthylmorpholine, 4-méthyl-pipérazine et 4-acétylpipérazine.

**7.** Composé selon la revendication 1, choisi dans le groupe constitué par
5-méthyl-1-(2-morpholin-4-yl-2-oxo-éthyl)-2-phényl-1H-quinoléin-4-one ;
N,N-diéthyl-2-(7-méthyl-4-oxo-2-phényl-4H-quinoléin-1-yl)-acétamide ;
7-méthyl-1-(2-morpholin-4-yl-2-oxo-éthyl)-2-phényl-1H-quinoléin-4-one ;
N,N-dibutyl-2-(7-méthyl-4-oxo-2-phényl-4H-quinoléin-1-yl)-acétamide ;
N,N-diisopropyl-2-(5-méthyl-4-oxo-2-phényl-4H-quinoléin-1-yl)-acétamide ;
5-méthyl-1-(2-oxo-2-pyrrolidin-1-yl-éthyl)-2-phényl-1H-quinoléin-4-one ;
2-(7-méthyl-4-oxo-2-phényl-4H-quinoléin-1-yl)-N,N-dipropylacétamide ;
N,N-dihexyl-2-(5-méthyl-4-oxo-2-phényl-4H-quinoléin-1-yl)-acétamide ;
5-méthyl-1-(2-oxo-2-pipéridin-1-yl-éthyl)-2-phényl-1H-quinoléin-4-one ;
5-méthyl-1-(2-oxo-2-pyrrolidin-1-yl-éthyl)-2-phényl-1H-quinoléin-4-one ;
7-méthyl-1-(2-oxo-2-pyrrolidin-1-yl-éthyl)-2-phényl-1H-quinoléin-4-one ;
N,N-dibutyl-2-(7-méthyl-4-oxo-3-phényl-4H-quinoléin-1-yl)-acétamide ;
2-(7-méthyl-4-oxo-3-phényl-4H-quinoléin-1-yl)-N,N-dipropylacétamide ;
7-méthyl-1-(2-oxo-2-pipéridin-1-yl-éthyl)-3-phényl-1H-quinoléin-4-one ;
N,N-dihexyl-2-(7-méthyl-4-oxo-3-phényl-4H-quinoléin-1-yl)-acétamide ;
N,N-diéthyl-2-(7-méthyl-4-oxo-3-phényl-4H-quinoléin-1-yl)-acétamide ;
7-méthyl-1-(2-oxo-2-pyrrolidin-1-yl-éthyl)-3-phényl-1H-quinoléin-4-one ;
7-méthyl-1-(2-morpholin-4-yl-2-oxo-éthyl)-3-phényl-1H-quinoléin-4-one ;
N,N-diisopropyl-2-(7-méthyl-4-oxo-3-phényl-4H-quinoléin-1-yl)-acétamide ;

N,N-dihexyl-2-(5-méthyl-4-oxo-3-phényl-4H-quinoléin-1-yl)-acétamide ;
5-méthyl-1-(2-oxo-2-pipéridin-1-yl-éthyl)-3-phényl-1H-quinoléin-4-one ;
5-méthyl-1-(2-morpholin-4-yl-2-oxo-éthyl)-3-phényl-1H-quinoléin-4-one ;
N,N-diéthyl-2-(5-méthyl-4-oxo-3-phényl-4H-quinoléin-1-yl)-acétamide ;
N,N-dibutyl-2-(5-méthyl-4-oxo-3-phényl-4H-quinoléin-1-yl)-acétamide ;
7-méthyl-1-(2-oxo-2-pyridin-3-yl-éthyl)-3-phényl-1H-quinoléin-4-one ;
7-méthyl-1-(2-oxo-2-thiophén-2-yl-éthyl)-3-phényl-1H-quinoléin-4-one ;
7-méthyl-1-(2-oxo-2-pyridin-2-yl-éthyl)-3-phényl-1H-quinoléin-4-one ;
7-méthyl-1-(2-oxo-2-pyridin-4-yl-éthyl)-3-phényl-1H-quinoléin-4-one ;
1-[2-(4-acétyl-pipérazin-1-yl)-2-oxo-éthyl]-7-méthyl-3-phényl-1H-quinoléin-4-one ;
7-méthyl-1-[2-(5-méthyl-furan-2-yl)-2-oxo-éthyl]-3-phényl-1H-quinoléin-4-one ;
1-[2-(2,6-diméthyl-morpholin-4-yl)-2-oxo-éthyl]-7-méthyl-3-phényl-1H-quinoléin-4-one ;
1-[2-(2,4-diméthyl-oxazol-5-yl)-2-oxo-éthyl]-7-méthyl-3-phényl-1H-quinoléin-4-one ;
7-méthyl-1-(2-oxo-2-pyrazin-2-yl-éthyl)-3-phényl-1H-quinoléin-4-one ;
7-méthyl-1-[2-(4-méthyl-pipérazin-1-yl)-2-oxo-éthyl]-3-phényl-1H-quinoléin-4-one ;
2-[3-(4-méthoxy-phényl)-7-méthyl-4-oxo-4H-quinoléin-1-yl]-N,N-dipropyl-acétamide ;
2-[3-(4-chloro-phényl)-7-méthyl-4-oxo-4H-quinoléin-1-yl]-N,N-dipropyl-acétamide ;
2-(7-méthyl-4-oxo-3-p-tolyl-4H-quinoléin-1-yl)-N,N-dipropyl-acétamide ;
N-cyclopropylméthyl-2-(7-méthyl-4-oxo-3-phényl-4H-quinoléin-1-yl)-N-propyl-acétamide ;
1-[2-(3,5-diméthyl-pipéridin-1-yl)-2-oxo-éthyl]-7-méthyl-3-phényl-1H-quinoléin-4-one ;
1-[2-(3,6-dihydro-2H-pyridin-1-yl)-2-oxo-éthyl]-7-méthyl-3-phényl-1H-quinoléin-4-one ;
1-[2-(2,5-diméthyl-2,5-dihydro-pyrrol-1-yl)-2-oxo-éthyl]-7-méthyl-3-phényl-1H-quinoléin-4-one ;
N,N-diallyl-2-(7-méthyl-4-oxo-3-phényl-4H-quinoléin-1-yl)-acétamide ;
2-(7-bromo-4-oxo-3-phényl-4H-quinoléin-1-yl)-N,N-dipropyl-acétamide ;
2-(5-bromo-4-oxo-3-phényl-4H-quinoléin-1-yl)-N,N-dipropyl-acétamide ;
2-(4-oxo-3,7-diphényl-4H-quinoléin-1-yl)-N,N-dipropyl-acétamide ;
2-(4-oxo-3-phényl-4H-quinoléin-1-yl)-N,N-dipropyl-acétamide ;
2-(4-oxo-3,5-diphényl-4H-quinoléin-1-yl)-N,N-dipropyl-acétamide ;
2-[3-(4-chloro-phényl)-7-méthyl-4-oxo-4H-quinoléin-1-yl]-N-phényl-acétamide ;
2-[3-(4-chloro-phényl)-7-méthyl-4-oxo-4H-quinoléin-1-yl]-N-pyrazin-2-yl-acétamide ;
hydrazide d'acide [3-(4-Chloro-phényl)-7-méthyl-4-oxo-4H-quinoléin-1-yl]-acétique ;
2-(7-méthyl-4-oxo-3-p-tolyl-4H-quinoléin-1-yl)-N-phényl-acétamide ;
2-[3-(4-fluoro-phényl)-7-méthyl-4-oxo-4H-quinoléin-1-yl]-N-phényl-acétamide ;
2-(7-méthyl-4-oxo-3-p-tolyl-4H-quinoléin-1-yl)-N-thiazol-2-yl-acétamide ;
N-(5-méthyl-isoxazol-3-yl)-2-(7-méthyl-4-oxo-3-p-tolyl-4H-quinoléin-1-yl)-acétamide ;
hydrazide d'acide [3-(4-fluoro-phényl)-7-méthyl-4-oxo-4H-quinoléin-1-yl]-acétique ;
hydrazide d'acide (7-méthyl-4-oxo-3-p-tolyl-4H-quinoléin-1-yl)-acétique ;
2-[3-(4-chloro-phényl)-7-méthyl-4-oxo-4H-quinoléin-1-yl]-N-(5-méthyl-isoxazol-3-yl)-acétamide ;
2-[3-(4-fluoro-phényl)-7-méthyl-4-oxo-4H-quinoléin-1-yl]-N-(5-méthyl-isoxazol-3-yl)-acétamide ;
N-éthyl-N-(2-hydroxy-éthyl)-2-(7-méthyl-4-oxo-3-phényl-4H-quinoléin-1-yl)-acétamide ;
N-(2-hydroxy-éthyl)-2-(7-méthyl-4-oxo-3-phényl-4H-quinoléin-1-yl)-N-propyl-acétamide ;
N,N-diallyl-2-(5-méthyl-4-oxo-3-phényl-4H-quinoléin-1-yl)-acétamide ;
N-(2-hydroxy-éthyl)-2-(5-méthyl-4-oxo-3-phényl-4H-quinoléin-1-yl)-N-propyl-acétamide ;
hydrazide d'acide [3-(4-chloro-phényl)-7-méthyl-4-oxo-4H-quinoléin-1-yl]-acétique ;
N-méthylhydrazide d'acide (7-méthyl-4-oxo-3-p-tolyl-4H-quinoléin-1-yl)-acétique ;
N-méthylhydrazide d'acide [3-(4-fluoro-phényl)-7-méthyl-4-oxo-4H-quinoléin-1-yl]-acétique ;
2-[3-(4-fluoro-phényl)-7-méthyl-4-oxo-4H-quinoléin-1-yl]-N-thiazol-2-yl-acétamide ;
2-[3-(4-chloro-phényl)-7-méthyl-4-oxo-4H-quinoléin-1-yl]-N-thiazol-2-yl-acétamide ;
2-[3-(4-méthoxy-phényl)-7-méthyl-4-oxo-4H-quinoléin-1-yl]-N-(4-trifluorométhyl-phényl)-acétamide ;
2-(7-méthyl-4-oxo-3-phényl-4H-quinoléin-1-yl)-N-(4-trifluorométhyl-phényl)-acétamide ;
N-(4-méthoxy-phényl)-2-(7-méthyl-4-oxo-3-phényl-4H-quinoléin-1-yl)-acétamide ;
N-(4-méthoxy-phényl)-2-[3-(4-méthoxy-phényl)-7-méthyl-4-oxo-4H-quinoléin-1-yl]-acétamide ;
2-[3-(4-méthoxy-phényl)-7-méthyl-4-oxo-4H-quinoléin-1-yl]-N-(5-méthyl-2H-pyrazol-3-yl)-acétamide ;
2-(7-méthyl-4-oxo-3-phényl-4H-quinoléin-1-yl)-N-thiazol-2-yl-acétamide ;
2-[3-(4-méthoxy-phényl)-7-méthyl-4-oxo-4H-quinoléin-1-yl]-N-thiazol-2-yl-acétamide ;
2-(7-méthyl-4-oxo-3-phényl-4H-quinoléin-1-yl)-N-(3-méthyl-thiophén-2-ylméthyl)-acétamide ;
2-[3-(4-méthoxy-phényl)-7-méthyl-4-oxo-4H-quinoléin-1-yl]-N-(3-méthyl-isoxazol-5-yl)-acétamide ;
2-[3-(4-méthoxy-phényl)-7-méthyl-4-oxo-4H-quinoléin-1-yl]-N-(5-méthyl-pyridin-2-yl)-acétamide ;

2-(7-méthyl-4-oxo-3-phényl-4H-quinoléin-1-yl)-N-pyrimidin-2-yl-acétamide ;
N-furan-2-ylméthyl-2-(7-méthyl-4-oxo-3-phényl-4H-quinoléin-1-yl)-acétamide ;
N-(2,5-diméthyl-2H-pyrazol-3-yl)-2-[3-(4-méthoxy-phényl)-7-méthyl-4-oxo-4H-quinoléin-1-yl]-acétamide ;
N,N-diéthyl-2-[3-(4-méthoxy-phényl)-7-méthyl-4-oxo-4H-quinoléin-1-yl]-acétamide ;
3-(4-méthoxy-phényl)-7-méthyl-1-(2-oxo-2-pyrrolidin-1-yl-éthyl)-1H-quinoléin-4-one ;
3-(4-méthoxy-phényl)-7-méthyl-1-(2-oxo-2-pipéridin-1-yl-éthyl)-1H-quinoléin-4-one ;
N-éthyl-N-(2-hydroxy-éthyl)-2-[3-(4-méthoxy-phényl)-7-méthyl-4-oxo-4H-quinoléin-1-yl]-acétamide ;
N-(2-hydroxy-éthyl)-2-[3-(4-méthoxy-phényl)-7-méthyl-4-oxo-4H-quinoléin-1-yl]-N-propyl-acétamide ;
N-cyclopropylméthyl-2-[3-(4-méthoxy-phényl)-7-méthyl-4-oxo-4H-quinoléin-1-yl]-N-propyl-acétamide ;
N,N-bis-(2-hydroxy-éthyl)-2-[3-(4-méthoxy-phényl)-7-méthyl-4-oxo-4H-quinoléin-1-yl]-acétamide ;
N,N-diallyl-2-[3-(4-méthoxy-phényl)-7-méthyl-4-oxo-4H-quinoléin-1-yl]-acétamide ;
N-(3,5-diméthyl-isoxazol-4-yl)-2-(7-méthyl-4-oxo-3-phényl-4H-quinoléin-1-yl)-acétamide ;
2-(7-méthyl-4-oxo-3-phényl-4H-quinoléin-1-yl)-N-(1-méthyl-1H-pyrazol-3-yl)-acétamide ;
N-(2,5-diméthyl-2H-pyrazol-3-yl)-2-(7-méthyl-4-oxo-3-phényl-4H-quinoléin-1-yl)-acétamide ;
2-(7-méthyl-4-oxo-3-phényl-4H-quinoléin-1-yl)-N-(5-méthyl-pyridin-2-yl)-acétamide ;
2-(7-méthyl-4-oxo-3-phényl-4H-quinoléin-1-yl)-N-[1,3,4]thiadiazol-2-yl-acétamide ;
N-(4-diméthylamino-phényl)-2-(7-méthyl-4-oxo-3-phényl-4H-quinoléin-1-yl)-acétamide ;
N-(3-méthyl-isoxazol-5-yl)-2-(7-méthyl-4-oxo-3-phényl-4H-quinoléin-1-yl)-acétamide ;
2-(7-méthyl-4-oxo-3-phényl-4H-quinoléin-1-yl)-N-*p*-tolyl-acétamide ;
2-[3-(4-méthoxy-phényl)-7-méthyl-4-oxo-4H-quinoléin-1-yl]-N-(1-méthyl-1H-pyrazol-3-yl)-acétamide ;
1-[2-(2,5-diméthyl-2,5-dihydro-pyrrol-1-yl)-2-oxo-éthyl]-3-(4-méthoxy-phényl)-7-méthyl-1H-quinoléin-4-one ;
2-(7-méthyl-4-oxo-3-phényl-4H-quinoléin-1-yl)-N-(5-méthyl-2H-pyrazol-3-yl)-acétamide ;
2-(7-méthyl-4-oxo-3-phényl-4H-quinoléin-1-yl)-N-pyridin-2-ylméthyl-acétamide ;
N-(2-acétyl-thiophén-3-yl)-2-(7-méthyl-4-oxo-3-phényl-4H-quinoléin-1-yl)-acétamide ;
N-éthyl-2-(7-méthyl-4-oxo-3-phényl-4H-quinoléin-1-yl)-N-[1,3,4]thiadiazol-2-yl-acétamide ;
N-(2-méthyl-1H-indol-5-yl)-2-(7-méthyl-4-oxo-3-phényl-4H-quinoléin-1-yl)-acétamide ;
1-[2-(3,6-dihydro-2H-pyridin-1-yl)-2-oxo-éthyl]-7-méthyl-3-phényl-1H-quinoléin-4-one ;
2-(7-méthyl-4-oxo-3-phényl-4H-quinoléin-1-yl)-N-(1H-pyrazol-3-yl)-acétamide ;
N-(4-fluoro-phényl)-2-(7-méthyl-4-oxo-3-phényl-4H-quinoléin-1-yl)-acétamide ;
2-(7-méthyl-4-oxo-3-phényl-4H-quinoléin-1-yl)-N-(5-trifluorométhyl-[1,3,4]thiadiazol-2-yl)-acétamide ;
2-(7-méthyl-4-oxo-3-phényl-4H-quinoléin-1-yl)-N-quinoléin-2-yl-acétamide ;
2-(7-méthyl-4-oxo-3-phényl-4H-quinoléin-1-yl)-N-(2-méthyl-2H-pyrazol-3-yl)-acétamide ;
N-(5-cyclopropyl-2H-pyrazol-3-yl)-2-(7-méthyl-4-oxo-3-phényl-4H-quinoléin-1-yl)-acétamide ;
N-isoquinoléin-3-yl-2-(7-méthyl-4-oxo-3-phényl-4H-quinoléin-1-yl)-acétamide ;
N-indan-2-yl-2-(7-méthyl-4-oxo-3-phényl-4H-quinoléin-1-yl)-acétamide ;
2-(7-méthyl-4-oxo-3-phényl-4H-quinoléin-1-yl)-N-(2-thiophén-2-yl-éthyl)-acétamide ;
7-bromo-1-[2-(3,6-dihydro-2H-pyridin-1-yl)-2-oxo-éthyl]-3-phényl-1H-quinoléin-4-one ;
hydrazide d'acide (7-bromo-4-oxo-3-phényl-4H-quinoléin-1-yl)-acétique ;
2-(7-bromo-4-oxo-3-phényl-4H-quinoléin-1-yl)-N,N-dibutyl-acétamide ;
7-bromo-1-(2-oxo-2-pyrrolidin-1-yl-éthyl)-3-phényl-1H-quinoléin-4-one ;
7-bromo-1-(2-oxo-2-pipéridin-1-yl-éthyl)-3-phényl-1H-quinoléin-4-one ;
7-bromo-1-[2-(2,6-diméthyl-morpholin-4-yl)-2-oxo-éthyl]-3-phényl-1H-quinoléin-4-one ;
N,N-diallyl-2-(7-bromo-4-oxo-3-phényl-4H-quinoléin-1-yl)-acétamide ;
7-bromo-1-[2-(2,5-diméthyl-2,5-dihydro-pyrrol-1-yl)-2-oxo-éthyl]-3-phényl-1H-quinoléin-4-one ;
2-(7-bromo-4-oxo-3-phényl-4H-quinoléin-1-yl)-N-cyclopropylméthyl-N-propyl-acétamide ;
2-(7-méthyl-4-oxo-3-phényl-4H-quinoléin-1-yl)-N-(5-nitro-thiazol-2-yl)-acétamide ;
N-isoxazol-3-yl-2-(7-méthyl-4-oxo-3-phényl-4H-quinoléin-1-yl)-acétamide ;
2-(7-méthyl-4-oxo-3-phényl-4H-quinoléin-1-yl)-N-(4-méthyl-thiazol-2-yl)-acétamide ;
7-bromo-1-(2-oxo-2-pipéridin-1-yl-éthyl)-3-phényl-1H-quinoléin-4-one ;
1-[2-(2,6-diméthyl-morpholin-4-yl)-2-oxo-éthyl]-3-(4-méthoxy-phényl)-7-méthyl-1H-quinoléin-4-one ;
1-[2-(3,5-diméthyl-pipéridin-1-yl)-2-oxo-éthyl]-3-(4-méthoxy-phényl)-7-méthyl-1H-quinoléin-4-one ;
N-(6-méthoxy-pyrimidin-4-yl)-2-(7-méthyl-4-oxo-3-phényl-4H-quinoléin-1-yl)-acétamide ;
1-[2-(4-hydroxy-pipéridin-1-yl)-2-oxo-éthyl]-7-méthyl-3-phényl-1H-quinoléin-4-one ;
N-isoxazol-3-yl-2-[3-(4-méthoxy-phényl)-7-méthyl-4-oxo-4H-quinoléin-1-yl]-acétamide ; et
1-[2-(4-hydroxy-pipéridin-1-yl)-2-oxo-éthyl]-3-(4-méthoxy-phényl)-7-méthyl-1H-quinoléin-4-one ; et
leurs sels et hydrates pharmaceutiquement acceptables.

**8.** Composé choisi parmi

ester éthylique d'acide (7-méthyl-4-oxo-2-phényl-4H-quinoléin-1-yl)-acétique ;
ester méthylique d'acide (7-méthyl-4-oxo-3-p-tolyl-4H-quinoléin-1-yl)-acétique ;
acide (7-méthyl-4-oxo-3-p-tolyl-4H-quinoléin-1-yl)-acétique ;
ester méthylique d'acide [3-(4-chloro-phényl)-7-méthyl-4-oxo-4H-quinoléin-1-yl]-acétique ;
ester méthylique d'acide [3-(4-fluoro-phényl)-7-méthyl-4-oxo-4H-quinoléin-1-yl]-acétique ;
acide [3-(4-fluoro-phényl)-7-méthyl-4-oxo-4H-quinoléin-1-yl]-acétique ;
acide [3-(4-chloro-phényl)-7-méthyl-4-oxo-4H-quinoléin-1-yl]-acétique ;
acide (7-méthyl-4-oxo-3-phényl-4H-quinoléin-1-yl)-acétique ;
acide (5-méthyl-4-oxo-3-phényl-4H-quinoléin-1-yl)-acétique ;
ester méthylique de l'acide (7-méthyl-4-oxo-3-phényl-4H-quinoléin-1-yl)-acétique ;
acide (7-méthyl-4-oxo-3-phényl-4H-quinoléin-1-yl)-acétique ;
ester méthylique d'acide [3-(4-méthoxy-phényl)-7-méthyl-4-oxo-4H-quinoléin-1-yl]-acétique ;
acide [3-(4-méthoxy-phényl)-7-méthyl-4-oxo-4H-quinoléin-1-yl]-acétique ;
acide (7-bromo-4-oxo-3-phényl-4H-quinoléin-1-yl)-acétique ; et
leurs sels et hydrates pharmaceutiquement acceptables.

**9.** Procédé de préparation d'un composé répondant à la formule (I) selon la revendication 1, comprenant la réaction d'un composé intermédiaire répondant à la formule (VII) :

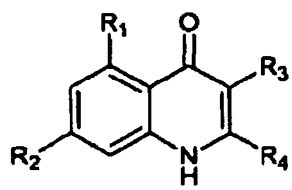

(VII)

dans laquelle $R_1$, $R_2$, $R_3$ et $R_4$ sont tels que définis pour (I), avec la 2-bromoéthanone appropriée (XII) :

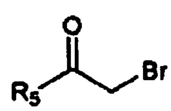

(XII)

dans laquelle $R_5$ est tel que défini selon la revendication 1.

**10.** Procédé selon la revendication 9, pour la préparation d'un composé amide répondant à la formule (I), dans laquelle $R_5$ est $NR_6R_7$, comprenant la réaction d'un composé acide répondant à la formule (I) telle que définie selon la revendication 1, dans laquelle $R_5$ est OH, avec l'amine $R_6R_7NH$ appropriée (IX), dans laquelle $R_6$ et $R_7$ sont tels que définis selon la revendication 1.

**11.** Procédé selon la revendication 9, pour la préparation d'un composé hydrazide répondant à la formule (I), dans laquelle $R_5$ est $N(R_8)NH_2$, comprenant la réaction d'un composé ester répondant à la formule (I), dans laquelle $R_5$ est $OR_9$, avec l'hydrazine appropriée de formule $NH_2NHR_8$ (X), dans laquelle $R_8$ est tel que défini selon la revendication 1.

**12.** Utilisation d'un composé selon l'une quelconque des revendications 1 à 8, pour la préparation d'un médicament pour traiter ou prévenir des maladies associées à la modulation du récepteur $GABA_A$, choisies parmi l'anxiété, l'épilepsie, les troubles du sommeil ou l'insomnie, induire une hypnose par sédation, une anesthésie ou une relaxation musculaire ou moduler le temps nécessaire à l'induction du sommeil et sa durée chez un être humain ou un mammifère non humain en ayant besoin.

**13.** Utilisation selon la revendication 12, dans laquelle le récepteur $GABA_A$ est le récepteur $\alpha_1$-$GABA_A$ ou le récepteur $\alpha_2$-$GABA_A$.

**14.** Composition pharmaceutique comprenant une quantité thérapeutiquement efficace d'un composé tel que défini selon l'une quelconque des revendications 1 à 8, avec des quantités appropriées d'excipients ou de véhicules pharmaceutiques.

**15.** Utilisation d'une 1H-quinoléinone répondant à la formule (I) :

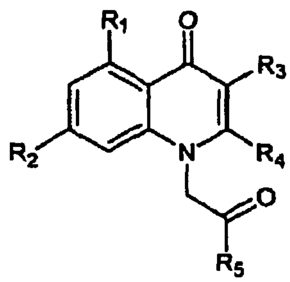

(I)

dans laquelle
$R_5$ est choisi dans le groupe constitué par $NR_6R_7$, $N(R_8)NH_2$, OH, $OR_9$ et un cycle hétéroaryle choisi dans le groupe constitué par un groupe pyridyle, un groupe furyle, un groupe thiényle, un groupe oxazolyle, un groupe isoxazolyle, un groupe imidazolyle, un groupe pyrrolyle, un groupe pyrazolyle, un groupe pyrimidinyle et un groupe pyrazinyle, chaque cycle hétéroaryle contenant un ou deux substituants alkyle en $C_1$-$C_6$ facultatifs ;
$R_9$ est un groupe alkyle en $C_1$-$C_6$ ; et
$R_1$, $R_2$, $R_3$, $R_4$, $R_6$, $R_7$ et $R_8$ sont tels que définis selon l'une quelconque des revendications 1 à 6 ;
ou ses sels ou hydrates pharmaceutiquement acceptables, pour la préparation d'un médicament pour traiter ou prévenir des maladies associées à la modulation du récepteur $GABA_A$, choisies parmi l'anxiété, l'épilepsie, les troubles du sommeil ou l'insomnie, induire une hypnose par sédation, une anesthésie ou une relaxation musculaire ou moduler le temps nécessaire à l'induction du sommeil et sa durée chez un être humain ou un mammifère non humain en ayant besoin.

**16.** 1H-quinoléinone selon l'une quelconque des revendications 1 à 8 ou 15, pour une utilisation dans le traitement ou la prévention de maladies associées à la modulation du récepteur $GABA_A$, choisies parmi l'anxiété, l'épilepsie, les troubles du sommeil ou l'insomnie, l'induction d'une hypnose par sédation, d'une anesthésie ou d'une relaxation musculaire ou la modulation du temps nécessaire à l'induction du sommeil et de sa durée chez un être humain ou un mammifère non humain en ayant besoin.

**17.** 1H-quinoléinone selon la revendication 16, dans laquelle le récepteur $GABA_A$ est le récepteur $\alpha_1$-$GABA_A$ ou le récepteur $\alpha_2$-$GABA_A$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 856255 A
- WO 2001012607 A
- WO 2002022074 A
- WO 9817662 A
- EP 719764 A
- WO 03097564 A

### Non-patent literature cited in the description


- **H. Möhler et al.** *J. Pharmacol. Exp. Ther.,* 2002, vol. 300, 2-8
- **H. Möhler et al.** *Curr. Opin. Pharmacol.,* 2001, vol. 1, 22-25
- **U. Rudolph et al.** *Nature,* 1999, vol. 401, 796-800
- **D.J. Nutt et al.** *Br. J. Psychiatry,* 2001, vol. 179, 390-396
- **C. F. P. George.** *The Lancet,* 2001, vol. 358, 1623-1626
- **Traxler et al.** *J. Med. Chem.,* 1999, vol. 42, 1018-1026
- **J. Lameh et al.** *Prog. Neuro-Psychopharmacol. Biol. Psychiatry,* 2000, vol. 24, 979-991
- **H. Noguchi et al.** *Eur J Pharm,* 2002, vol. 434, 21-28
- **S. Arbilla et al.** *Eur.. J. Pharmacol.,* 1986, vol. 130, 257-263
- **Y. Wu et al.** *Eur. J. Pharmacol.,* 1995, vol. 278, 125-132
- **D. J. Sanger et al.** *Eur. J. Pharmacol.,* 1996, vol. 313, 35-42
- **G. Griebel et al.** *Psychopharmacology,* 1999, vol. 146, 205-213